# EUROPEAN PATENT SPECIFICATION

(11) **EP 1 571 211 B1**
(45) Date of publication and mention of the grant of the patent: **20.01.2010**
(21) Application number: 03775858.8
(22) Date of filing: 21.11.2003
(51) Int. Cl.: C12N 15/12, C12N 15/09, C07K 16/32, C07K 16/18, G01N 33/53, C07K 16/00, C12N 15/13, C12N 15/70, C12N 1/21, A61K 39/395, A61P 35/00, C07K 16/30, G01N 33/547, G01N 33/577

(54) **ANTIBODIES AGAINST LESIONAL TISSUES**
ANTIKÖRPER GEGEN GESCHÄDIGTES GEWEBE
ANTICORPS DIRIGES CONTRE TISSUES LESES

(30) Priority: 22.11.2002 JP 2002339241
(43) Date of publication of application: 07.09.2005
(73) Proprietor: CHUGAI SEIYAKU KABUSHIKI KAISHA, Tokyo, 115-8543 (JP); Pharmalogicals Research Pte. Ltd., Singapore 258500 (SG)
(72) Inventor: TSUCHIYA, Masayuki, c/o CHUGAI SEIYAKU KABUSHIKI K, Gotenba-shi, Shizuoka 412-8513 (JP); SUZUKI, Masami, c/o CHUGAI SEIYAKU KABUSHIKI K., Gotenba-shi, Shizuoka 412-8513 (JP); YOSHIDA, Kenji, c/o CHUGAI SEIYAKU KABUSHIKI K., Gotenba-shi, Shizuoka 412-8513 (JP); FUJII, Etsuko, c/o CHUGAI SEIYAKU KABUSHIKI K., Gotenba-shi, Shizuoka 412-8513 (JP); MATSUBARA, Kouichi, c/o PHARM. RESEARCH PTE. LTD., Singapore 258500 (SG); TSUNODA, Hiroyuki, c/o CHUGAI SEIYAKU KABUSHIKI K., Gotenba-shi, Shizuoka 412-8513 (JP)
(74) Representative: Vossius & Partner
(86) International application number: PCT/JP2003/014919
(87) International publication number: WO 2004/048571

(56) References cited:
- WO-A-01/68682
- JP-A- 06 141 884
- ZHANG H ET AL: "A human monoclonal antimelanoma single-chain Fv antibody derived from tumor-infiltrating lymphocytes" CANCER RESEARCH, AMERICAN ASSOCIATION FOR CANCER RESEARCH, BALTIMORE, MD, US, vol. 55, no. 16, 15 August 1995 (1995-08-15), pages 3584-3591, XP002971556 ISSN: 0008-5472
- CORONELLA J A ET AL: "Antigen-driven oligoclonal expansion of tumor-infiltrating B cells in infiltrating ductal carcinoma of the breast" JOURNAL OF IMMUNOLOGY, THE WILLIAMS AND WILKINS CO. BALTIMORE, US, vol. 169, no. 4, 15 August 2002 (2002-08-15), pages 1829-1836, XP002325246 ISSN: 0022-1767
- NZULA S ET AL: "Antigen-driven clonal proliferation, somatic hypermutation, and selection of B lymphocytes infiltrating human ductal breast carcinomas" CANCER RESEARCH, AMERICAN ASSOCIATION FOR CANCER RESEARCH, BALTIMORE, MD, US, vol. 63, no. 12, 15 June 2003 (2003-06-15), pages 3275-3280, XP002325249 ISSN: 0008-5472
- WU H ET AL: "Cloning, isolation and characterization of human tumor in situ monoclonal antibodies" CANCER IMMUNOLOGY AND IMMUNOTHERAPY, BERLIN, DE, vol. 51, no. 2, April 2002 (2002-04), pages 79-90, XP002260156 ISSN: 0340-7004
- BONNER R F ET AL: "LASER CAPTURE MICRODISSECTION: MOLECULAR ANALYSIS OF TISSUE" SCIENCE, AMERICAN ASSOCIATION FOR THE ADVANCEMENT OF SCIENCE,, US, vol. 278, 21 November 1997 (1997-11-21), page 1481,1483, XP000941813 ISSN: 0036-8075
- DATABASE EMBASE [Online] ELSEVIER SCIENCE PUBLISHERS, AMSTERDAM, NL; 1976, IOACHIM H L: "The stromal reaction of tumors: an expression of immune surveillance" XP002363208 Database accession no. EMB-1977199257 & JOURNAL OF THE NATIONAL CANCER INSTITUTE 1976, vol. 57, no. 3, 1976, pages 465-470,
- ABE AKIHIRO: 'DNA o mochiita shuyo tokuiteki kogen ni taisuru men'eki yudo B saibosei akusei shuyo o model ni' SANKYO SEIMEI KAGAKU KENKYU SHINKO ZAIDAN KENKYU HOKOKUSHU vol. 11, 1998, pages 213 - 219, XP002986497
- VARSHA PATKI ET AL.: 'Evidence for B cell oligo-clonality in the blood and joints of patients with rheumatoid arthritis' ANN. N.Y. ACAD. SCI. vol. 815, 1997, pages 472 - 474, XP002986498
- RATECH HOWARD: 'Rapid cloning of rearranged immunoglobulin heavy chain genes from human B-cell lines using anchored polymerase chain reaction' BIOCHEMICAL AND BIOPHYSICAL RESEARCH COMMUNICATIONS vol. 182, no. 3, 1992, pages 1260 - 1263, XP002986499
- ICHINOMIYA SHINGO ET AL.: 'VII. Men'eki saibo 2. Laser microdissection o riyo shita men'eki soshiki no atarashii kaisekiho' ANNUAL REVIEW MEN'EKI 2002 2001, pages 147 - 179, XP002986500
- LUO LIN ET AL.: 'Gene expression profiles of laser-captured adjacent neuroal subtypes' NATURE MEDICINE vol. 5, no. 1, 1999, pages 117 - 122, XP002187082
- TACHIKAWA TETSUHIKO ET AL.: 'Laser microdissection-ho no gan chiryo eno oyo' HEMATOLOGY & ONCOLOGY vol. 42, no. 6, 2001, pages 565 - 571, XP002986701
- PUNT C.J. ET AL: 'Anti-tumor antibody produced by human tumor-infiltrating and peripheral blood B lymphocytes.' CANCER IMMUNOLOGY IMMUNOTHERAPY April 1994, GERMANY, pages 225 - 232

### SEQUENCE LISTING

<110> CHUGAI SEIYAKU KABUSHIKI KAISHA
   PharmaLogicals Research Pte., Ltd.
<120> Antibody against focus tissue
<130> C1-A0230P
<150> JP 2002-339241
   <151> 2002-11-22
<160> 188
<170> PatentIn version 3.1
<210> 1
   <211> 360
   <212> DNA
   <213> Homo sapiens
<220>
   <221> CDS
   <222> (1).. (360)
   <223>
<400> 1
   cag gtg cag ctg gtg caa tct ggg gct gag gtg aag aag cct ggg gcc        48
   Gln Val Gln Leu Val Gln Ser Gly Ala Glu Val Lys Lys Pro Gly Ala
   1               5                   10                  15
   tca gtg aag gtc tcc tgt cag gct tct gga tac atg ttc acc ggc ttc        96
   Ser Val Lys Val Ser Cys Gln Ala Ser Gly Tyr Met Phe Thr Gly Phe
               20                  25                  30
   tat atg cac tgg gtg cga cag gcc cct gga caa ggg ctt gag tgg atg       144
   Tyr Met His Trp Val Arg Gln Ala Pro Gly Gln Gly Leu Glu Trp Met
   35 40 45
   gga tgg atg aac act aac agt ggt gcc aca ggc tat gca cac aag ttt 192
   Gly Trp Met Asn Thr Asn Ser Gly Ala Thr Gly Tyr Ala His Lys Phe
   50 55 60
   cag gac agg gtc acc ctg acc agg gac acg tcc atc agc aca ggc tac 240
   Gln Asp Arg Val Thr Leu Thr Arg Asp Thr Ser Ile Ser Thr Gly Tyr
   65 70 75 80
   atg gag ctg ggc ggc ctg aca tct gac gac acg gcc gtg tat tat tgt 288
   Met Glu Leu Gly Gly Leu Thr Ser Asp Asp Thr Ala Val Tyr Tyr Cys
   85 90 95
   gcg aga acc cag gag gtt tac tac tac gct atg gac gtc tgg ggc caa 336
   Ala Arg Thr Gln Glu Val Tyr Tyr Tyr Ala Met Asp Val Trp Gly Gln
   100 105 110
   ggg cca atg gtc acc gtc tct tca 360
   Gly Pro Met Val Thr Val Ser Ser
   115 120
<210> 2
   <211> 120
   <212> PRT
   <213> Homo sapiens
<400> 2
   Gln Val Gln Leu Val Gln Ser Gly Ala Glu Val Lys Lys Pro Gly Ala
   1 5 10 15
   Ser Val Lys Val Ser Cys Gln Ala Ser Gly Tyr Met Phe Thr Gly Phe
   20 25 30
   Tyr Met His Trp Val Arg Gln Ala Pro Gly Gln Gly Leu Glu Trp Met
   35 40 45
   Gly Trp Met Asn Thr Asn Ser Gly Ala Thr Gly Tyr Ala His Lys Phe
   50 55 60
   Gln Asp Arg Val Thr Leu Thr Arg Asp Thr Ser Ile Ser Thr Gly Tyr
   65 70 75 80
   Met Glu Leu Gly Gly Leu Thr Ser Asp Asp Thr Ala Val Tyr Tyr Cys
   85 90 95
   Ala Arg Thr Gln Glu Val Tyr Tyr Tyr Ala Met Asp Val Trp Gly Gln
   100 105 110
   Gly Pro Met Val Thr Val Ser Ser
   115 120
<210> 3
   <211> 366
   <212> DNA
   <213> Homo sapiens
<220>
   <221> CDS
   <222> (1).. (366)
   <223>
<400> 3
   cag gtc cag ctg gtg caa tct gga gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc acc ttc agt agc aat 96
   Ser Leu Arg Leu Ser Cys Ala Ala Ser Gly Phe Thr Phe Ser Ser Asn
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt aat aaa tac tat gca gac tcc gtg 192
   Ala Val Ile Trp Tyr Asp Gly Ser Asn Lys Tyr Tyr Ala Asp Ser Val
   50 55 60
   aag ggc cga ttc acc atc tcc aga gac aat tcc aag aac aca ctg tat 240
   Lys Gly Arg Phe Thr Ile Ser Arg Asp Asn Ser Lys Asn Thr Leu Tyr
   65 70 75 80
   ctg caa atg aac agc ctg aga gcc gag gac acg gct gtg tat tac tgt 288
   Leu Gln Met Asn Ser Leu Arg Ala Glu Asp Thr Ala Val Tyr Tyr Cys
   85 90 95
   gcg aga gat cac ggc ctt ggt gat caa gcc tcc tgg ttc gac ccc tgg 336
   Ala Arg Asp His Gly Leu Gly Asp Gln Ala Ser Trp Phe Asp Pro Trp
   100 105 110
   ggc cag gga acc ctg gtc acc gtc tcc tca 366
   Gly Gln Gly Thr Leu Val Thr Val Ser Ser
   115 120
<210> 4
   <211> 122
   <212> PRT
   <213> Homo sapiens
<400> 4
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Thr Phe Ser Ser Asn
   20 25 30
   Gly Met His Trp Val Arg Gln Ala Pro Gly Lys Gly Leu Glu Trp Val
   35 40 45
   Ala Val Ile Trp Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp His Gly Leu Gly Asp Gln Ala Ser Trp Phe Asp Pro Trp
   100 105 110
   Gly Gln Gly Thr Leu Val Thr Val Ser Ser
   115 120
<210> 5
   <211> 366
   <212> DNA
   <213> Homo sapiens
<220>
   <221> CDS
   <222> (1).. (366)
   <223>
<400> 5
   cag gtc cag ctg gtg caa tct ggg gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc acc ttc agt agc aat 96
   Ser Leu Arg Leu Ser Cys Ala Ala Ser Gly Phe Thr Phe Ser Ser Asn
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt aat aaa tac tat gca gac tcc gtg 192
   Ala Val Ile Trp Tyr Asp Gly Ser Asn Lys Tyr Tyr Ala Asp Ser Val
   50 55 60
   aag ggc cga ttc acc atc tcc aga gac aat tcc aag aac aca ctg tat 240
   Lys Gly Arg Phe Thr Ile Ser Arg Asp Asn Ser Lys Asn Thr Leu Tyr
   65 70 75 80
   ctg caa atg aac agc ctg aga gcc gag gac acg gct gtg tat tac tgt 288
   Leu Gln Met Asn Ser Leu Arg Ala Glu Asp Thr Ala Val Tyr Tyr Cys
   85 90 95
   gcg aga gat cac ggc ctt ggt gat caa gcc tcc tgg ttc gac ccc tgg 336
   Ala Arg Asp His Gly Leu Gly Asp Gln Ala Ser Trp Phe Asp Pro Trp
   100 105 110
   ggc cag gga acc ctg gtc acc gtc tcc tca 366
   Gly Gln Gly Thr Leu Val Thr Val Ser Ser
   115 120
<210> 6
   <211> 122
   <212> PRT
   <213> Homo sapiens
<400> 6
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Thr Phe Ser Ser Asn
   20 25 30
   Gly Met His Trp Val Arg Gln Ala Pro Gly Lys Gly Leu Glu Trp Val
   35 40 45
   Ala Val Ile Trp Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp His Gly Leu Gly Asp Gln Ala Ser Trp Phe Asp Pro Trp
   100 105 110
   Gly Gln Gly Thr Leu Val Thr Val Ser Ser
   115 120
<210> 7
   <211> 340
   <212> DNA
   <213> Homo sapiens
<220>
   <221> CDS
   <222> (1).. (339)
   <223>
<400> 7
   cag gtg cag ctg gtg caa tct ggg gct gag gtg agg aag cct ggg acg 48
   Gln Val Gln Leu Val Gln Ser Gly Ala Glu Val Arg Lys Pro Gly Thr
   1 5 10 15
   aca gtg aca atc tcc tgc aag gtt tct gga cac aac ttc atc gac cac 96
   Thr Val Thr Ile Ser Cys Lys Val Ser Gly His Asn Phe Ile Asp His
   20 25 30
   tac atg cat tgg gta caa cag gcc cct gga aaa ggg ctt gac tgg atg 144
   Tyr Met His Trp Val Gln Gln Ala Pro Gly Lys Gly Leu Asp Trp Met
   35 40 45
   gga cta att gac cct gaa gat ggt cag acg aaa tat tca gag agg ttt 192
   Gly Leu Ile Asp Pro Glu Asp Gly Gln Thr Lys Tyr Ser Glu Arg Phe
   50 55 60
   gag ggc aga gtc aca att acc gcg gac aag tca aca gac aca acc tac 240
   Glu Gly Arg Val Thr Ile Thr Ala Asp Lys Ser Thr Asp Thr Thr Tyr
   65 70 75 80
   ttg gag gtg agc ggc ctg aga tcg gaa gac acg gcc gtt tat ttc tgt 288
   Leu Glu Val Ser Gly Leu Arg Ser Glu Asp Thr Ala Val Tyr Phe Cys
   85 90 95
   aca acg gac ttg ggt gac ttg aat tat tgg aac cct ggt cac cgt ctc 336
   Thr Thr Asp Leu Gly Asp Leu Asn Tyr Trp Asn Pro Gly His Arg Leu
   100 105 110
   ctc a 340
   Leu
<210> 8
   <211> 113
   <212> PRT
   <213> Homo sapiens
<400> 8
   Gln Val Gln Leu Val Gln Ser Gly Ala Glu Val Arg Lys Pro Gly Thr
   1 5 10 15
   Thr Val Thr Ile Ser Cys Lys Val Ser Gly His Asn Phe Ile Asp His
   20 25 30
   Tyr Met His Trp Val Gln Gln Ala Pro Gly Lys Gly Leu Asp Trp Met
   35 40 45
   Gly Leu Ile Asp Pro Glu Asp Gly Gln Thr Lys Tyr Ser Glu Arg Phe
   50 55 60
   Glu Gly Arg Val Thr Ile Thr Ala Asp Lys Ser Thr Asp Thr Thr Tyr
   65 70 75 80
   Leu Glu Val Ser Gly Leu Arg Ser Glu Asp Thr Ala Val Tyr Phe Cys
   85 90 95
   Thr Thr Asp Leu Gly Asp Leu Asn Tyr Trp Asn Pro Gly His Arg Leu
   100 105 110
   Leu
<210> 9
   <211> 366
   <212> DNA
   <213> Homo sapiens
<220>
   <221> CDS
   <222> (1).. (366)
   <223>
<400> 9
   cag gtc cag ctg gtg caa tct ggg gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc acc ttc agt agc aat 96
   Ser Leu Arg Leu Ser Cys Ala Ala Ser Gly Phe Thr Phe Ser Ser Asn
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt aat aaa tac tat gca gac tcc gtg 192
   Ala Val Ile Trp Tyr Asp Gly Ser Asn Lys Tyr Tyr Ala Asp Ser Val
   50 55 60
   aag ggc cga ttc acc atc tcc aga gac aat tcc aag aac aca ctg tat 240
   Lys Gly Arg Phe Thr Ile Ser Arg Asp Asn Ser Lys Asn Thr Leu Tyr
   65 70 75 80
   ctg caa atg aac agc ctg aga gcc gag gac acg gct gtg tat tac tgt 288
   Leu Gln Met Asn Ser Leu Arg Ala Glu Asp Thr Ala Val Tyr Tyr Cys
   85 90 95
   gcg aga gat cac ggc ctt ggt gat caa gcc tcc tgg ttc gac ccc tgg 336
   Ala Arg Asp His Gly Leu Gly Asp Gln Ala Ser Trp Phe Asp Pro Trp
   100 105 110
   ggc cag gga acc ctg gtc acc gtc tcc tca 366
   Gly Gln Gly Thr Leu Val Thr Val Ser Ser
   115 120
<210> 10
   <211> 122
   <212> PRT
   <213> Homo sapiens
<400> 10
   Gln Val Gln Leu Val Gln Ser Gly Gly Gly Val Val Gln Pro Gly Arg
   1               5                   10                  15
   Ser Leu Arg Leu Ser Cys Ala Ala Ser Gly Phe Thr Phe Ser Ser Asn
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp His Gly Leu Gly Asp Gln Ala Ser Trp Phe Asp Pro Trp
               100                 105                 110
   Gly Gln Gly Thr Leu Val Thr Val Ser Ser
           115                 120
<210> 11
   <211> 381
   <212> DNA
   <213> Homo sapiens
<220>
   <221> CDS
   <222> (1)..(381)
   <223>
<400> 11
   cag gtc cag ctg gtg caa tct gga gct gag ggg aaa aag ccg gga gag 48
   Gln Val Gln Leu Val Gln Ser Gly Ala Glu Gly Lys Lys Pro Gly Glu
   1 5 10 15
   tct ctg aag atc tcc tgt cag ggt tct gga tac aca ttt agc aat tac 96
   Ser Leu Lys Ile Ser Cys Gln Gly Ser Gly Tyr Thr Phe Ser Asn Tyr
   20 25 30
   tgg atc gcc tgg gtg cgc cag agg ccc ggg aaa ggc ctg gag tgg atg 144
   Trp Ile Ala Trp Val Arg Gln Arg Pro Gly Lys Gly Leu Glu Trp Met
   35 40 45
   ggg atc atc tat cct ggt gac tct gat atc aaa tac agt ccg tcc ttc 192
   Gly Ile Ile Tyr Pro Gly Asp Ser Asp Ile Lys Tyr Ser Pro Ser Phe
   50 55 60
   caa ggc cat gtc acc atc tca gcc gac acg tcc atg aac acc gcc tac 240
   Gln Gly His Val Thr Ile Ser Ala Asp Thr Ser Met Asn Thr Ala Tyr
   65 70 75 80
   ctg cag tgg aac acc ctg aag gcc tcg gac acc gcc atg tac tac tgt 288
   Leu Gln Trp Asn Thr Leu Lys Ala Ser Asp Thr Ala Met Tyr Tyr Cys
   85 90 95
   gcg aga cat aaa ggg acc agg ttc ggg gag gtt ttg gcg gtt ggc aac 336
   Ala Arg His Lys Gly Thr Arg Phe Gly Glu Val Leu Ala Val Gly Asn
   100 105 110
   tgg ttc gac ccc tgg ggc cag gga acc ctg gtc acc gtc tcc tca 381
   Trp Phe Asp Pro Trp Gly Gln Gly Thr Leu Val Thr Val Ser Ser
   115 120 125
<210> 12
   <211> 127
   <212> PRT
   <213> Homo sapiens
<400> 12
   Gln Val Gln Leu Val Gln Ser Gly Ala Glu Gly Lys Lys Pro Gly Glu
   1 5 10 15
   Ser Leu Lys Ile Ser Cys Gln Gly Ser Gly Tyr Thr Phe Ser Asn Tyr
   20 25 30
   Trp Ile Ala Trp Val Arg Gln Arg Pro Gly Lys Gly Leu Glu Trp Met
   35 40 45
   Gly Ile Ile Tyr Pro Gly Asp Ser Asp Ile Lys Tyr Ser Pro Ser Phe
   50 55 60
   Gln Gly His Val Thr Ile Ser Ala Asp Thr Ser Met Asn Thr Ala Tyr
   65 70 75 80
   Leu Gln Trp Asn Thr Leu Lys Ala Ser Asp Thr Ala Met Tyr Tyr Cys
   85 90 95
   Ala Arg His Lys Gly Thr Arg Phe Gly Glu Val Leu Ala Val Gly Asn
   100 105 110
   Trp Phe Asp Pro Trp Gly Gln Gly Thr Leu Val Thr Val Ser Ser
   115 120 125
<210> 13
   <211> 368
   <212> DNA
   <213> Homo sapiens
<220>
   <221> CDS
   <222> (1)..(366)
   <223>
<400> 13
   cag gtc cag ctg gtg caa tct ggg gct gag ttg aag acg cct ggg tcc 48
   Gln Val Gln Leu Val Gln Ser Gly Ala Glu Leu Lys Thr Pro Gly Ser
   1 5 10 15
   tcg gtg aaa ttc tcc tgc aag gct tcc gga ggc agc ttc agc aac tat 96
   Ser Val Lys Phe Ser Cys Lys Ala Ser Gly Gly Ser Phe Ser Asn Tyr
   20 25 30
   gct atc acc tgg gtg cga cag gcc cct gga caa ggt ctt gag tgg atg 144
   Ala Ile Thr Trp Val Arg Gln Ala Pro Gly Gln Gly Leu Glu Trp Met
   35 40 45
   gga agg atc atc cct atc ttt ggt ata cca aac tac gca cag gaa ttc 192
   Gly Arg Ile Ile Pro Ile Phe Gly Ile Pro Asn Tyr Ala Gln Glu Phe
   50 55 60
   cag ggc aga gtc acg att acc gcc gac gat tcc acg acc aca gtc tac 240
   Gln Gly Arg Val Thr Ile Thr Ala Asp Asp Ser Thr Thr Thr Val Tyr
   65 70 75 80
   atg gaa ctg agc agc ctg aga tct gag gac acg gcc gtg tat tac tgt 288
   Met Glu Leu Ser Ser Leu Arg Ser Glu Asp Thr Ala Val Tyr Tyr Cys
   85 90 95
   gcg aga gat aat tca ata gga gca cct gat act tgg tgg ttc gac ccc 336
   Ala Arg Asp Asn Ser Ile Gly Ala Pro Asp Thr Trp Trp Phe Asp Pro
   100 105 110
   tgg ggc cag gga cca cgg tca ccg tct cct ca 368
   Trp Gly Gln Gly Pro Arg Ser Pro Ser Pro
   115 120
<210> 14
   <211> 122
   <212> PRT
   <213> Homo sapiens
<400> 14
   Gln Val Gln Leu Val Gln Ser Gly Ala Glu Leu Lys Thr Pro Gly Ser
   1 5 10 15
   Ser Val Lys Phe Ser Cys Lys Ala Ser Gly Gly Ser Phe Ser Asn Tyr
   20 25 30
   Ala Ile Thr Trp Val Arg Gln Ala Pro Gly Gln Gly Leu Glu Trp Met
   35 40 45
   Gly Arg Ile Ile Pro Ile Phe Gly Ile Pro Asn Tyr Ala Gln Glu Phe
   50 55 60
   Gln Gly Arg Val Thr Ile Thr Ala Asp Asp Ser Thr Thr Thr Val Tyr
   65 70 75 80
   Met Glu Leu Ser Ser Leu Arg Ser Glu Asp Thr Ala Val Tyr Tyr Cys
   85 90 95
   Ala Arg Asp Asn Ser Ile Gly Ala Pro Asp Thr Trp Trp Phe Asp Pro
   100 105 110
   Trp Gly Gln Gly Pro Arg Ser Pro Ser Pro
   115 120
<210> 15
   <211> 360
   <212> DNA
   <213> Homo sapiens
<220>
   <221> CDS
   <222> (1).. (360)
   <223>
<400> 15
   cag gtc cag ctg gtg caa tct ggg gct gag gtg aag aag cct ggg gcc      48
   Gln Val Gln Leu Val Gln Ser Gly Ala Glu Val Lys Lys Pro Gly Ala
   1               5                   10                  15
   tca gtg aag gtc tcc tgt cag gct tct gga tac atg ttc acc ggc ttc      96
   Ser Val Lys Val Ser Cys Gln Ala Ser Gly Tyr Met Phe Thr Gly Phe
               20                  25                  30
   tat atg cac tgg gtg cga cag gcc cct gga caa ggg ctt gag tgg atg      144
   Tyr Met His Trp Val Arg Gln Ala Pro Gly Gln Gly Leu Glu Trp Met
           35                  40                  45
   gga tgg atg aac act aac agt ggt gcc aca ggc tat gca cac aag ttt      192
   Gly Trp Met Asn Thr Asn Ser Gly Ala Thr Gly Tyr Ala His Lys Phe
       50                  55                  60
   cag gac agg gtc acc ctg acc agg gac acg tcc atc agc aca ggc tac      240
   Gln Asp Arg Val Thr Leu Thr Arg Asp Thr Ser Ile Ser Thr Gly Tyr
   65                  70                  75                  80
   atg gag ctg ggc ggc ctg aca tct gac gac acg gcc gtg tat tat tgt      288
   Met Glu Leu Gly Gly Leu Thr Ser Asp Asp Thr Ala Val Tyr Tyr Cys
                   85                  90                  95
   gcg aga acc cag gag gtt tac tac tac gct atg gac gtc tgg ggc caa      336
   Ala Arg Thr Gln Glu Val Tyr Tyr Tyr Ala Met Asp Val Trp Gly Gln
               100                 105                 110
   ggg aca atg gtc acc gtc tct tca                                      360
   Gly Thr Met Val Thr Val Ser Ser
           115                 120
<210> 16
   <211> 120
   <212> PRT
   <213> Homo sapiens
<400> 16
   Gln Val Gln Leu Val Gln Ser Gly Ala Glu Val Lys Lys Pro Gly Ala
   1               5                   10                  15
   Ser Val Lys Val Ser Cys Gln Ala Ser Gly Tyr Met Phe Thr Gly Phe
               20                  25                  30
   Tyr Met His Trp Val Arg Gln Ala Pro Gly Gln Gly Leu Glu Trp Met
           35                  40                  45
   Gly Trp Met Asn Thr Asn Ser Gly Ala Thr Gly Tyr Ala His Lys Phe
       50                  55                  60
   Gln Asp Arg Val Thr Leu Thr Arg Asp Thr Ser Ile Ser Thr Gly Tyr
   65                  70                  75                  80
   Met Glu Leu Gly Gly Leu Thr Ser Asp Asp Thr Ala Val Tyr Tyr Cys
                   85                  90                  95
   Ala Arg Thr Gln Glu Val Tyr Tyr Tyr Ala Met Asp Val Trp Gly Gln
               100                 105                 110
   Gly Thr Met Val Thr Val Ser Ser
           115                 120
<210> 17
   <211> 365
   <212> DNA
   <213> Homo sapiens
<220>
   <221> CDS
   <222> (1).. (363)
   <223>
<400> 17
   cag gtc cag ctg gtg caa tct ggg gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc acc ttc agt agc aat 96
   Ser Leu Arg Leu Ser Cys Ala Ala Ser Gly Phe Thr Phe Ser Ser Asn
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt aat aaa tac tat gca gac tcc gtg 192
   Ala Val Ile Trp Tyr Asp Gly Ser Asn Lys Tyr Tyr Ala Asp Ser Val
   50 55 60
   aag ggc cga ttc acc atc tcc aga gac aat tcc aag aac aca ctg tat 240
   Lys Gly Arg Phe Thr Ile Ser Arg Asp Asn Ser Lys Asn Thr Leu Tyr
   65 70 75 80
   ctg caa atg aac agc ctg agg gcc gag gac acg gct gtg tat tac tgt 288
   Leu Gln Met Asn Ser Leu Arg Ala Glu Asp Thr Ala Val Tyr Tyr Cys
   85 90 95
   gcg aga gat cac ggc ctt ggt gat caa gcc tcc tgg ttc gac ccc tgg 336
   Ala Arg Asp His Gly Leu Gly Asp Gln Ala Ser Trp Phe Asp Pro Trp
   100 105 110
   ggc cag gga acc ctg gtc acc gtc tcc tc 365
   Gly Gln Gly Thr Leu Val Thr Val Ser
   115 120
<210> 18
   <211> 121
   <212> PRT
   <213> Homo sapiens
<400> 18
   Gln Val Gln Leu Val Gln Ser Gly Gly Gly Val Val Gln Pro Gly Arg
   1               5                   10                  15
   Ser Leu Arg Leu Ser Cys Ala Ala Ser Gly Phe Thr Phe Ser Ser Asn
               20                  25                  30
   Gly Met His Trp Val Arg Gln Ala Pro Gly Lys Gly Leu Glu Trp Val
           35                  40                  45
   Ala Val Ile Trp Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp His Gly Leu Gly Asp Gln Ala Ser Trp Phe Asp Pro Trp
               100                 105                 110
   Gly Gln Gly Thr Leu Val Thr Val Ser
           115                 120
<210> 19
   <211> 366
   <212> DNA
   <213> Homo sapiens
<220>
   <221> CDS
   <222> (1).. (366)
   <223>
<400> 19
   cag gtc cag ctg gcg caa tct gga gga ggc gtg gtc cag cct ggg agg      48
   Gln Val Gln Leu Ala Gln Ser Gly Gly Gly Val Val Gln Pro Gly Arg
   1               5                   10                  15
   tcc ctg aga ctc tcc tgt gca gcc tct gga ttc agc ttc agt agc tat      96
   Ser Leu Arg Leu Ser Cys Ala Ala Ser Gly Phe Ser Phe Ser Ser Tyr
               20                  25                  30
   ggc atg cac tgg gtc cgc cag gct cca ggc aag ggg ctg gag tgg gtg     144
   Gly Met His Trp Val Arg Gln Ala Pro Gly Lys Gly Leu Glu Trp Val
           35                  40                  45
   gca gtt ata tgg tat gat gga agc tat aaa tac tat gca gaa tcc gtg     192
   Ala Val Ile Trp Tyr Asp Gly Ser Tyr Lys Tyr Tyr Ala Glu Ser Val
       50                  55                  60
   aag ggc cga ttc atc atc tcc aga gac aat tcc aag aac acc ctg tat     240
   Lys Gly Arg Phe Ile Ile Ser Arg Asp Asn Ser Lys Asn Thr Leu Tyr
   65                  70                  75                  80
   ctg caa atg aac agc ctg aga gcc gag gac acg gct gtc tat tac tgt     288
   Leu Gln Met Asn Ser Leu Arg Ala Glu Asp Thr Ala Val Tyr Tyr Cys
                   85                  90                  95
   gcg aga gat cgg ggg tcg gtg gag atg gct aca atc gcg gac tac tgg     336
   Ala Arg Asp Arg Gly Ser Val Glu Met Ala Thr Ile Ala Asp Tyr Trp
               100                 105                 110
   ggc cag gga acc ctg gtc acc gtc tcc tca                             366
   Gly Gln Gly Thr Leu Val Thr Val Ser Ser
           115                 120

<210> 20
   <211> 122
   <212> PRT
   <213> Homo sapiens
   <400> 20
   Gln Val Gln Leu Ala Gln Ser Gly Gly Gly Val Val Gln Pro Gly Arg
   1 5 10 15
   Ser Leu Arg Leu Ser Cys Ala Ala Ser Gly Phe Ser Phe Ser Ser Tyr
   20 25 30
   Gly Met His Trp Val Arg Gln Ala Pro Gly Lys Gly Leu Glu Trp Val
   35 40 45
   Ala Val Ile Trp Tyr Asp Gly Ser Tyr Lys Tyr Tyr Ala Glu Ser Val
   50 55 60
   Lys Gly Arg Phe Ile Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Arg Gly Ser Val Glu Met Ala Thr Ile Ala Asp Tyr Trp
   100 105 110
   Gly Gln Gly Thr Leu Val Thr Val Ser Ser
   115 120
<210> 21
   <211> 366
   <212> DNA
   <213> Homo sapiens
<220>
   <221> CDS
   <222> (1).. (366)
   <223>
<400> 21
   cag gtc cag ctg gtg caa tct ggg gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc agc ttc agt agc tat 96
   Ser Leu Arg Leu Ser Cys Ala Ala Ser Gly Phe Ser Phe Ser Ser Tyr
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt tat aaa tac tat gca gaa tcc gtg 192
   Ala Val Ile Trp Tyr Asp Gly Ser Tyr Lys Tyr Tyr Ala Glu Ser Val
   50 55 60
   aag ggc cga ttc atc atc tcc aga gac aat tcc aag aac acc ctg tat 240
   Lys Gly Arg Phe Ile Ile Ser Arg Asp Asn Ser Lys Asn Thr Leu Tyr
   65 70 75 80
   ctg caa atg aac agc ctg aga gcc gag gac acg gct gtg tat tac tgt 288
   Leu Gln Met Asn Ser Leu Arg Ala Glu Asp Thr Ala Val Tyr Tyr Cys
   85 90 95
   gcg aga gat cgg ggg tcg gta gag atg gct aca atc gcg gac tac tgg 336
   Ala Arg Asp Arg Gly Ser Val Glu Met Ala Thr Ile Ala Asp Tyr Trp
   100 105 110
   ggc cag gga acc ctg gtc acc gtc tcc tca 366
   Gly Gln Gly Thr Leu Val Thr Val Ser Ser
   115 120
<210> 22
   <211> 122
   <212> PRT
   <213> Homo sapiens
<400> 22
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Ser Phe Ser Ser Tyr
   20 25 30
   Gly Met His Trp Val Arg Gln Ala Pro Gly Lys Gly Leu Glu Trp Val
   35 40 45
   Ala Val Ile Trp Tyr Asp Gly Ser Tyr Lys Tyr Tyr Ala Glu Ser Val
   50 55 60
   Lys Gly Arg Phe Ile Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Arg Gly Ser Val Glu Met Ala Thr Ile Ala Asp Tyr Trp
   100 105 110
   Gly Gln Gly Thr Leu Val Thr Val Ser Ser
   115 120
<210> 23
   <211> 366
   <212> DNA
   <213> Homo sapiens
<220>
   <221> CDS
   <222> (1).. (366)
   <223>
<400> 23
   cag gtc cag ctg gtg caa tct ggg gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc agc ttc agt agc tat 96
   Ser Leu Arg Leu Ser Cys Ala Ala Ser Gly Phe Ser Phe Ser Ser Tyr
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt tat aaa tac tat gca gaa tcc gtg 192
   Ala Val Ile Trp Tyr Asp Gly Ser Tyr Lys Tyr Tyr Ala Glu Ser Val
   50 55 60
   aag ggc cga ttc atc atc tcc aga gac aat tcc aag aac acc ctg tat 240
   Lys Gly Arg Phe Ile Ile Ser Arg Asp Asn Ser Lys Asn Thr Leu Tyr
   65 70 75 80
   ctg caa atg aac agc ctg aga gcc gag gac acg gct gtc tat tac tgt 288
   Leu Gln Met Asn Ser Leu Arg Ala Glu Asp Thr Ala Val Tyr Tyr Cys
   85 90 95
   gcg aga gat cgg ggg tcg gta gag atg gct aca atc gcg gac tac tgg 336
   Ala Arg Asp Arg Gly Ser Val Glu Met Ala Thr Ile Ala Asp Tyr Trp
   100 105 110
   ggc cag gga acc ctg gtc acc gtc tcc tca 366
   Gly Gln Gly Thr Leu Val Thr Val Ser Ser
   115 120
<210> 24
   <211> 122
   <212> PRT
   <213> Homo sapiens
<400> 24
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Ser Phe Ser Ser Tyr
   20 25 30
   Gly Met His Trp Val Arg Gln Ala Pro Gly Lys Gly Leu Glu Trp Val
   35 40 45
   Ala Val Ile Trp Tyr Asp Gly Ser Tyr Lys Tyr Tyr Ala Glu Ser Val
   50 55 60
   Lys Gly Arg Phe Ile Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Arg Gly Ser Val Glu Met Ala Thr Ile Ala Asp Tyr Trp
   100 105 110
   Gly Gln Gly Thr Leu Val Thr Val Ser Ser
   115 120
<210> 25
   <211> 370
   <212> DNA
   <213> Homo sapiens
<220>
   <221> CDS
   <222> (1).. (369)
   <223>
<400> 25
   cag gta cag ctg cag cag tca ggt cca gga ctg gtg aag ccc tcg cag 48
   Gln Val Gln Leu Gln Gln Ser Gly Pro Gly Leu Val Lys Pro Ser Gln
   1 5 10 15
   acc ctc tca ctc acc tgt gcc atc tcc ggg gac agt gtc tct agc aac 96
   Thr Leu Ser Leu Thr Cys Ala Ile Ser Gly Asp Ser Val Ser Ser Asn
   20 25 30
   agt gct gct tgg cac tgg atc agg cag tcc cca tcg aga ggc ctt gag 144
   Ser Ala Ala Trp His Trp Ile Arg Gln Ser Pro Ser Arg Gly Leu Glu
   35 40 45
   tgg ctg gga agg aca tac tac agg tcc aag tgg tat aat gat tat aca 192
   Trp Leu Gly Arg Thr Tyr Tyr Arg Ser Lys Trp Tyr Asn Asp Tyr Thr
   50 55 60
   gtg tct gtg aaa agt cga ata acc atc aag cca gac aca tcc aag aac 240
   Val Ser Val Lys Ser Arg Ile Thr Ile Lys Pro Asp Thr Ser Lys Asn
   65 70 75 80
   cag ttc tcc ctg cag ctg aac tct gtg act ccc gag gac acg gct gtg 288
   Gln Phe Ser Leu Gln Leu Asn Ser Val Thr Pro Glu Asp Thr Ala Val
   85 90 95
   tat tac tgt gca aga tca cag gaa gag cac cgg tcg ttg gat gat gct 336
   Tyr Tyr Cys Ala Arg Ser Gln Glu Glu His Arg Ser Leu Asp Asp Ala
   100 105 110
   ttt gat atc tgg gac cac ggt cac cgt ctc ctc a 370
   Phe Asp Ile Trp Asp His Gly His Arg Leu Leu
   115 120
<210> 26
   <211> 123
   <212> PRT
   <213> Homo sapiens
<400> 26
   Gln Val Gln Leu Gln Gln Ser Gly Pro Gly Leu Val Lys Pro Ser Gln
   1               5                   10                  15
   Thr Leu Ser Leu Thr Cys Ala Ile Ser Gly Asp Ser Val Ser Ser Asn
               20                  25                  30
   Ser Ala Ala Trp His Trp Ile Arg Gln Ser Pro Ser Arg Gly Leu Glu
           35                  40                  45
   Trp Leu Gly Arg Thr Tyr Tyr Arg Ser Lys Trp Tyr Asn Asp Tyr Thr
       50                  55                  60
   Val Ser Val Lys Ser Arg Ile Thr Ile Lys Pro Asp Thr Ser Lys Asn
   65                  70                  75                  80
   Gln Phe Ser Leu Gln Leu Asn Ser Val Thr Pro Glu Asp Thr Ala Val
                   85                  90                  95
   Tyr Tyr Cys Ala Arg Ser Gln Glu Glu His Arg Ser Leu Asp Asp Ala
               100                 105                 110
   Phe Asp Ile Trp Asp His Gly His Arg Leu Leu
           115                 120
<210> 27
   <211> 360
   <212> DNA
   <213> Homo sapiens
<220>
   <221> CDS
   <222> (1).. (360)
   <223>
<400> 27
   cag gtc cag ctg gtg caa tct ggg gct gag gtg aag aag cct ggg gcc 48
   Gln Val Gln Leu Val Gln Ser Gly Ala Glu Val Lys Lys Pro Gly Ala
   1 5 10 15
   tca gtg aag gtc tcc tgt cag gct tct gga tac atg ttc acc ggc ttc 96
   Ser Val Lys Val Ser Cys Gln Ala Ser Gly Tyr Met Phe Thr Gly Phe
   20 25 30
   tat atg cac tgg gtg cga cag gcc cct gga caa ggg ctt gag tgg atg 144
   Tyr Met His Trp Val Arg Gln Ala Pro Gly Gln Gly Leu Glu Trp Met
   35 40 45
   gga tgg atg aac act aac agt ggt gcc aca ggc tat gca cac aag ttt 192
   Gly Trp Met Asn Thr Asn Ser Gly Ala Thr Gly Tyr Ala His Lys Phe
   50 55 60
   cag gac agg gtc acc ctg acc agg gac acg tcc atc agc aca ggc tac 240
   Gln Asp Arg Val Thr Leu Thr Arg Asp Thr Ser Ile Ser Thr Gly Tyr
   65 70 75 80
   atg gag ctg ggc ggc ctg aca tct gac gac acg gcc gtg tat tat tgt 288
   Met Glu Leu Gly Gly Leu Thr Ser Asp Asp Thr Ala Val Tyr Tyr Cys
   85 90 95
   gcg aga acc cag gag gtt tac tac tac gct atg gac gtc tgg ggc caa 336
   Ala Arg Thr Gln Glu Val Tyr Tyr Tyr Ala Met Asp Val Trp Gly Gln
   100 105 110
   ggg aca atg gtc acc gtc tct tca 360
   Gly Thr Met Val Thr Val Ser Ser
   115 120
<210> 28
   <211> 120
   <212> PRT
   <213> Homo sapiens
<400> 28
   Gln Val Gln Leu Val Gln Ser Gly Ala Glu Val Lys Lys Pro Gly Ala
   1 5 10 15
   Ser Val Lys Val Ser Cys Gln Ala Ser Gly Tyr Met Phe Thr Gly Phe
   20 25 30
   Tyr Met His Trp Val Arg Gln Ala Pro Gly Gln Gly Leu Glu Trp Met
   35 40 45
   Gly Trp Met Asn Thr Asn Ser Gly Ala Thr Gly Tyr Ala His Lys Phe
   50 55 60
   Gln Asp Arg Val Thr Leu Thr Arg Asp Thr Ser Ile Ser Thr Gly Tyr
   65 70 75 80
   Met Glu Leu Gly Gly Leu Thr Ser Asp Asp Thr Ala Val Tyr Tyr Cys
   85 90 95
   Ala Arg Thr Gln Glu Val Tyr Tyr Tyr Ala Met Asp Val Trp Gly Gln
   100 105 110
   Gly Thr Met Val Thr Val Ser Ser
   115 120
<210> 29
   <211> 348
   <212> DNA
   <213> Homo sapiens
<220>
   <221> CDS
   <222> (1).. (348)
   <223>
<400> 29
   cag gtc cag ctg gtg caa tct ggg gct gag gtg agg aag ccc ggg acg 48
   Gln Val Gln Leu Val Gln Ser Gly Ala Glu Val Arg Lys Pro Gly Thr
   1 5 10 15
   aca gtg aca atc tcc tgc aag gtt tct gga cac aac ttc atc gac cac 96
   Thr Val Thr Ile Ser Cys Lys Val Ser Gly His Asn Phe Ile Asp His
   20 25 30
   tac atg cat tgg gta caa cag gcc cct gga aaa ggg ctt gac tgg atg 144
   Tyr Met His Trp Val Gln Gln Ala Pro Gly Lys Gly Leu Asp Trp Met
   35 40 45
   gga cta att gac cct gaa gat ggt cag acg aaa tat tca gag agg ttt 192
   Gly Leu Ile Asp Pro Glu Asp Gly Gln Thr Lys Tyr Ser Glu Arg Phe
   50 55 60
   gag ggc aga gtc aca att acc gcg gac aag tca aca gac aca acc tac 240
   Glu Gly Arg Val Thr Ile Thr Ala Asp Lys Ser Thr Asp Thr Thr Tyr
   65 70 75 80
   ttg gag gtg agc ggc ctg aga tcg gaa gac acg gcc gtt tat ttc tgt 288
   Leu Glu Val Ser Gly Leu Arg Ser Glu Asp Thr Ala Val Tyr Phe Cys
   85 90 95
   aca acg gac ttg ggt gac ttg aat tat tgg ggc cag gga acc ctg gtc 336
   Thr Thr Asp Leu Gly Asp Leu Asn Tyr Trp Gly Gln Gly Thr Leu Val
   100 105 110
   acc gtc tcc tca 348
   Thr Val Ser Ser
   115
<210> 30
   <211> 116
   <212> PRT
   <213> Homo sapiens
<400> 30
   Gln Val Gln Leu Val Gln Ser Gly Ala Glu Val Arg Lys Pro Gly Thr
   1 5 10 15
   Thr Val Thr Ile Ser Cys Lys Val Ser Gly His Asn Phe Ile Asp His
   20 25 30
   Tyr Met His Trp Val Gln Gln Ala Pro Gly Lys Gly Leu Asp Trp Met
   35 40 45
   Gly Leu Ile Asp Pro Glu Asp Gly Gln Thr Lys Tyr Ser Glu Arg Phe
   50 55 60
   Glu Gly Arg Val Thr Ile Thr Ala Asp Lys Ser Thr Asp Thr Thr Tyr
   65 70 75 80
   Leu Glu Val Ser Gly Leu Arg Ser Glu Asp Thr Ala Val Tyr Phe Cys
   85 90 95
   Thr Thr Asp Leu Gly Asp Leu Asn Tyr Trp Gly Gln Gly Thr Leu Val
   100 105 110
   Thr Val Ser Ser
   115
<210> 31
   <211> 366
   <212> DNA
   <213> Homo sapiens
<220>
   <221> CDS
   <222> (1).. (366)
   <223>
<400> 31
   cag gtc cag ctg gtg caa tct ggg gga ggc gtg gtc cag cct ggg agg 48
   Gln Val Gln Leu Val Gln Ser Gly Gly Gly Val Val Gln Pro Gly Arg
   1 5 10 15
   tcc ctg aga ctc tcc tgt gca gcc tct gga ttc acc ttc agt agc aat 96
   Ser Leu Arg Leu Ser Cys Ala Ala Ser Gly Phe Thr Phe Ser Ser Asn
   20 25 30
   ggc atg cac tgg gtc cgc cag gct cca ggc aag ggg ctg gag tgg gtg 144
   Gly Met His Trp Val Arg Gln Ala Pro Gly Lys Gly Leu Glu Trp Val
   35 40 45
   gca gtt ata tgg tat gat gga agt aat aaa tac tat gca gac tcc gtg 192
   Ala Val Ile Trp Tyr Asp Gly Ser Asn Lys Tyr Tyr Ala Asp Ser Val
   50 55 60
   aag ggc cga ttc acc atc tcc aga gac aat tcc aag aac aca ctg tat 240
   Lys Gly Arg Phe Thr Ile Ser Arg Asp Asn Ser Lys Asn Thr Leu Tyr
   65 70 75 80
   ctg caa atg aac agc ctg aga gcc gag gac acg gct gtg tat tac tgt 288
   Leu Gln Met Asn Ser Leu Arg Ala Glu Asp Thr Ala Val Tyr Tyr Cys
   85 90 95
   gcg aga gat cac ggc ctt ggt gat caa gcc tcc tgg ttc gac ccc tgg 336
   Ala Arg Asp His Gly Leu Gly Asp Gln Ala Ser Trp Phe Asp Pro Trp
   100 105 110
   ggc cag ggc acc ctg gtc acc gtc tcc tca 366
   Gly Gln Gly Thr Leu Val Thr Val Ser Ser
   115 120
<210> 32
   <211> 122
   <212> PRT
   <213> PRT
<400> 32
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Thr Phe Ser Ser Asn
   20 25 30
   Gly Met His Trp Val Arg Gln Ala Pro Gly Lys Gly Leu Glu Trp Val
   35 40 45
   Ala Val Ile Trp Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp His Gly Leu Gly Asp Gln Ala Ser Trp Phe Asp Pro Trp
   100 105 110
   Gly Gln Gly Thr Leu Val Thr Val Ser Ser
   115 120
<210> 33
   <211> 368
   <212> DNA
   <213> Homo sapiens
<220>
   <221> CDS
   <222> (1).. (366)
   <223>
<400> 33
   cag gtc cag ctg gtg caa tct ggg gct gag gtg aag aag tct ggg gcc     48
   Gln Val Gln Leu Val Gln Ser Gly Ala Glu Val Lys Lys Ser Gly Ala
   1               5                   10                  15
   tca gtg aag gtc tcc tgc aag gct tct gga tac acc ttc acc ggc cac     96
   Ser Val Lys Val Ser Cys Lys Ala Ser Gly Tyr Thr Phe Thr Gly His
               20                  25                  30
   ttt atc cac tgg gtg cgg cag gcc cct gga caa ggg ctt gag tgg atg    144
   Phe Ile His Trp Val Arg Gln Ala Pro Gly Gln Gly Leu Glu Trp Met
           35                  40                  45
   gga tgg atc aac cct aac gtt ggt gtc aca aat tat gca cag aag ttt    192
   Gly Trp Ile Asn Pro Asn Val Gly Val Thr Asn Tyr Ala Gln Lys Phe
       50                  55                  60
   cag ggc agg gtc acc atg acc agg gac acg tcc ata agc aca gcc tac    240
   Gln Gly Arg Val Thr Met Thr Arg Asp Thr Ser Ile Ser Thr Ala Tyr
   65                  70                  75                  80
   ata gaa ctg agg agg ctg aga tct gac gac acg gcc gtg tat tac tgt    288
   Ile Glu Leu Arg Arg Leu Arg Ser Asp Asp Thr Ala Val Tyr Tyr Cys
                   85                  90                  95
   gtg aga gaa tcc gac aca gct gcg gtg gcc tac tac tac cac ggt atg    336
   Val Arg Glu Ser Asp Thr Ala Ala Val Ala Tyr Tyr Tyr His Gly Met
               100                 105                 110
   gac gtc tgg gga caa tgg tca ccg tct ctt ca                         368
   Asp Val Trp Gly Gln Trp Ser Pro Ser Leu
           115                 120

<210> 34
   <211> 122
   <212> PRT
   <213> Homo sapiens
<400> 34
   Gln Val Gln Leu Val Gln Ser Gly Ala Glu Val Lys Lys Ser Gly Ala
   1 5 10 15
   Ser Val Lys Val Ser Cys Lys Ala Ser Gly Tyr Thr Phe Thr Gly His
   20 25 30
   Phe Ile His Trp Val Arg Gln Ala Pro Gly Gln Gly Leu Glu Trp Met
   35 40 45
   Gly Trp Ile Asn Pro Asn Val Gly Val Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Ile Glu Leu Arg Arg Leu Arg Ser Asp Asp Thr Ala Val Tyr Tyr Cys
   85 90 95
   Val Arg Glu Ser Asp Thr Ala Ala Val Ala Tyr Tyr Tyr His Gly Met
   100 105 110
   Asp Val Trp Gly Gln Trp Ser Pro Ser Leu
   115 120
<210> 35
   <211> 375
   <212> DNA
   <213> Homo sapiens
<220>
   <221> CDS
   <222> (1)..(375)
   <223>
<400> 35
   cag gtc cag ctg gtg caa tct ggg gga gac tgg gta aag cct ggg ggg 48
   Gln Val Gln Leu Val Gln Ser Gly Gly Asp Trp Val Lys Pro Gly Gly
   1 5 10 15
   tcc ctt aga ctc tcc tgt gca gcg tct gga ttc cct ttc gct aat gcc 96
   Ser Leu Arg Leu Ser Cys Ala Ala Ser Gly Phe Pro Phe Ala Asn Ala
   20 25 30
   tgg atg tat tgg ttc cgc cag gct cca ggg aag ggg ctg gag tgg gtt 144
   Trp Met Tyr Trp Phe Arg Gln Ala Pro Gly Lys Gly Leu Glu Trp Val
   35 40 45
   ggc cgt att aaa agc aaa cca agt ggt ggg gct aca gag ttc gct gca 192
   Gly Arg Ile Lys Ser Lys Pro Ser Gly Gly Ala Thr Glu Phe Ala Ala
   50 55 60
   ccc gtg gaa ggt aga ttc agc atc tcc aga gac gat tcg aaa aac acg 240
   Pro Val Glu Gly Arg Phe Ser Ile Ser Arg Asp Asp Ser Lys Asn Thr
   65 70 75 80
   atg gat ctg caa atg aat agc ctg aga acc gac gac aca gcc gta tat 288
   Met Asp Leu Gln Met Asn Ser Leu Arg Thr Asp Asp Thr Ala Val Tyr
   85 90 95
   tat tgt acc aca gat tgg ggt tcg ggg acc tat cat aag ttt gct tta 336
   Tyr Cys Thr Thr Asp Trp Gly Ser Gly Thr Tyr His Lys Phe Ala Leu
   100 105 110
   gat gtc tgg ggc caa ggg aca atg gtc acc gtc tct tca 375
   Asp Val Trp Gly Gln Gly Thr Met Val Thr Val Ser Ser
   115 120 125
<210> 36
   <211> 125
   <212> PRT
   <213> Homo sapiens
<400> 36
   Gln Val Gln Leu Val Gln Ser Gly Gly Asp Trp Val Lys Pro Gly Gly
   1 5 10 15
   Ser Leu Arg Leu Ser Cys Ala Ala Ser Gly Phe Pro Phe Ala Asn Ala
   20 25 30
   Trp Met Tyr Trp Phe Arg Gln Ala Pro Gly Lys Gly Leu Glu Trp Val
   35 40 45
   Gly Arg Ile Lys Ser Lys Pro Ser Gly Gly Ala Thr Glu Phe Ala Ala
   50 55 60
   Pro Val Glu Gly Arg Phe Ser Ile Ser Arg Asp Asp Ser Lys Asn Thr
   65 70 75 80
   Met Asp Leu Gln Met Asn Ser Leu Arg Thr Asp Asp Thr Ala Val Tyr
   85 90 95
   Tyr Cys Thr Thr Asp Trp Gly Ser Gly Thr Tyr His Lys Phe Ala Leu
   100 105 110
   Asp Val Trp Gly Gln Gly Thr Met Val Thr Val Ser Ser
   115 120 125
<210> 37
   <211> 327
   <212> DNA
   <213> Homo sapiens
<220>
   <221> CDS
   <222> (1).. (357)
   <223>
<400> 37
<210> 38
   <211> 119
   <212> PRT
   <213> Homo sapiens
<400> 38
   Val Gln Leu Val Gln Ser Gly Ala Glu Val Lys Lys Pro Gly Ala Ser
   1 5 10 15
   Val Lys Val Ser Cys Gln Ala Ser Gly Tyr Met Phe Thr Gly Phe Tyr
   20 25 30
   Met His Trp Val Arg Gln Ala Pro Gly Gln Gly Leu Glu Trp Met Gly
   35 40 45
   Trp Met Asn Thr Asn Ser Gly Ala Thr Gly Tyr Ala His Lys Phe Gln
   50 55 60
   Asp Arg Val Thr Leu Thr Arg Asp Thr Ser Ile Ser Thr Gly Tyr Met
   65 70 75 80
   Glu Leu Gly Gly Leu Thr Ser Asp Asp Thr Ala Val Tyr Tyr Cys Ala
   85 90 95
   Arg Thr Gln Glu Val Tyr Tyr Tyr Ala Met Asp Val Trp Gly Gln Gly
   100 105 110
   Thr Met Val Thr Val Ser Ser
   115

<210> 39
   <211> 360
   <212> DNA
   <213> Homo sapiens
<220>
   <221> CDS
   <222> (1)..(360)
   <223>
<400> 39
   cag gtg cag ctg gtg caa tct ggg gct gag gtg aag aag cct ggg gcc 48
   Gln Val Gln Leu Val Gln Ser Gly Ala Glu Val Lys Lys Pro Gly Ala
   1 5 10 15
   tca gtg aag gtc tcc tgt cag gct tct gga tac atg ttc acc ggc ttc 96
   Ser Val Lys Val Ser Cys Gln Ala Ser Gly Tyr Met Phe Thr Gly Phe
   20 25 30
   tat atg cac tgg gtg cga cag gcc cct gga caa ggg ctt gag tgg atg 144
   Tyr Met His Trp Val Arg Gln Ala Pro Gly Gln Gly Leu Glu Trp Met
   35 40 45
   gga tgg atg aac act aac agt ggt gcc aca ggc tat gca cac aag ttt 192
   Gly Trp Met Asn Thr Asn Ser Gly Ala Thr Gly Tyr Ala His Lys Phe
   50 55 60
   cag gac agg gtc acc ctg acc agg gac acg tcc atc agc aca ggc tac 240
   Gln Asp Arg Val Thr Leu Thr Arg Asp Thr Ser Ile Ser Thr Gly Tyr
   65 70 75 80
   atg gag ctg ggc ggc ctg aca tct gac gac acg gcc gtg tat tat tgt 288
   Met Glu Leu Gly Gly Leu Thr Ser Asp Asp Thr Ala Val Tyr Tyr Cys
   85 90 95
   gcg aga acc cag gag gtt tac tac tac gct atg gac gtc tgg ggc caa 336
   Ala Arg Thr Gln Glu Val Tyr Tyr Tyr Ala Met Asp Val Trp Gly Gln
   100 105 110
   ggg aca atg gtc acc gtc tct tca 360
   Gly Thr Met Val Thr Val Ser Ser
   115 120
<210> 40
   <211> 120
   <212> PRT
   <213> Homo sapiens
<400> 40
   Gln Val Gln Leu Val Gln Ser Gly Ala Glu Val Lys Lys Pro Gly Ala
   1 5 10 15
   Ser Val Lys Val Ser Cys Gln Ala Ser Gly Tyr Met Phe Thr Gly Phe
   20 25 30
   Tyr Met His Trp Val Arg Gln Ala Pro Gly Gln Gly Leu Glu Trp Met
   35 40 45
   Gly Trp Met Asn Thr Asn Ser Gly Ala Thr Gly Tyr Ala His Lys Phe
   50 55 60
   Gln Asp Arg Val Thr Leu Thr Arg Asp Thr Ser Ile Ser Thr Gly Tyr
   65 70 75 80
   Met Glu Leu Gly Gly Leu Thr Ser Asp Asp Thr Ala Val Tyr Tyr Cys
   85 90 95
   Ala Arg Thr Gln Glu Val Tyr Tyr Tyr Ala Met Asp Val Trp Gly Gln
   100 105 110
   Gly Thr Met Val Thr Val Ser Ser
   115 120
<210> 41
   <211> 360
   <212> DNA
   <213> Homo sapiens
<220>
   <221> CDS
   <222> (1)..(360)
   <223>
<400> 41
   cag gtc cag ctg gtg caa tct ggg gct gag gcg aag aag cct ggg gcc 48
   Gln Val Gln Leu Val Gln Ser Gly Ala Glu Ala Lys Lys Pro Gly Ala
   1 5 10 15
   tca gtg aag gtc tcc tgt cag gct tct gga tac atg ttc acc ggc ttc 96
   Ser Val Lys Val Ser Cys Gln Ala Ser Gly Tyr Met Phe Thr Gly Phe
   20 25 30
   tat atg cac tgg gtg cga cag gcc cct gga caa ggg ctt gag tgg atg 144
   Tyr Met His Trp Val Arg Gln Ala Pro Gly Gln Gly Leu Glu Trp Met
   35 40 45
   gga tgg atg aac act aac agt ggt gcc aca ggc tat gca cac aag ttt 192
   Gly Trp Met Asn Thr Asn Ser Gly Ala Thr Gly Tyr Ala His Lys Phe
   50 55 60
   cag gac agg gtc acc ctg acc agg gac acg tcc atc agc aca ggc tac 240
   Gln Asp Arg Val Thr Leu Thr Arg Asp Thr Ser Ile Ser Thr Gly Tyr
   65 70 75 80
   atg gag ctg ggc ggc ctg aca tct gac gac acg gcc gtg tat tat tgt 288
   Met Glu Leu Gly Gly Leu Thr Ser Asp Asp Thr Ala Val Tyr Tyr Cys
   85 90 95
   gcg aga acc cag gag gtt tac tac tac gct atg gac gtc tgg ggc caa 336
   Ala Arg Thr Gln Glu Val Tyr Tyr Tyr Ala Met Asp Val Trp Gly Gln
   100 105 110
   ggg acc acg gtc acc gtc tcc tca 360
   Gly Thr Thr Val Thr Val Ser Ser
   115 120
<210> 42
   <211> 120
   <212> PRT
   <213> Homo sapiens
<400> 42
   Gln Val Gln Leu Val Gln Ser Gly Ala Glu Ala Lys Lys Pro Gly Ala
   1               5                   10                  15
   Ser Val Lys Val Ser Cys Gln Ala Ser Gly Tyr Met Phe Thr Gly Phe
               20                  25                  30
   Tyr Met His Trp Val Arg Gln Ala Pro Gly Gln Gly Leu Glu Trp Met
           35                  40                  45
   Gly Trp Met Asn Thr Asn Ser Gly Ala Thr Gly Tyr Ala His Lys Phe
       50                  55                  60
   Gln Asp Arg Val Thr Leu Thr Arg Asp Thr Ser Ile Ser Thr Gly Tyr
   65                  70                  75                  80
   Met Glu Leu Gly Gly Leu Thr Ser Asp Asp Thr Ala Val Tyr Tyr Cys
                   85                  90                  95
   Ala Arg Thr Gln Glu Val Tyr Tyr Tyr Ala Met Asp Val Trp Gly Gln
               100                 105                 110
   Gly Thr Thr Val Thr Val Ser Ser
           115                 120
<210> 43
   <211> 369
   <212> DNA
   <213> Homo sapiens
<220>
   <221> CDS
   <222> (1).. (369)
   <223>
<400> 43
   cag gtc cag ctg gtg caa tct ggg gga ggc ttg gta cag cca ggg cgg      48
   Gln Val Gln Leu Val Gln Ser Gly Gly Gly Leu Val Gln Pro Gly Arg
   1               5                   10                  15
   tcc ctg aga ctc tcc tgt aca act tct gga ttc acc ttt agt gat tat      96
   Ser Leu Arg Leu Ser Cys Thr Thr Ser Gly Phe Thr Phe Ser Asp Tyr
               20                  25                  30
   gct ttg agc tgg gtc cgc cag gct cca ggg agg ggg ctg gag tgg gta     144
   Ala Leu Ser Trp Val Arg Gln Ala Pro Gly Arg Gly Leu Glu Trp Val
           35                  40                  45
   ggt ttc att aga aat aaa att tat ggt ggg aca aca gat tac gcc gca     192
   Gly Phe Ile Arg Asn Lys Ile Tyr Gly Gly Thr Thr Asp Tyr Ala Ala
       50                  55                  60
   tct gtg aaa ggc aga ttc acc atc tca aga gat gat tcc aaa agt atc     240
   Ser Val Lys Gly Arg Phe Thr Ile Ser Arg Asp Asp Ser Lys Ser Ile
   65                  70                  75                  80
   gcc tat ctg caa atg aac agc ctg aaa acc gag gac tca gcc gtc tat     288
   Ala Tyr Leu Gln Met Asn Ser Leu Lys Thr Glu Asp Ser Ala Val Tyr
                   85                  90                  95
   tac tgt act aga gat tcg ggt gtg gtg act gct gcc tac ttt gac tac     336
   Tyr Cys Thr Arg Asp Ser Gly Val Val Thr Ala Ala Tyr Phe Asp Tyr
               100                 105                 110
   tgg ggc cag ggc acc ctg gtc acc gtc tcc tca                         369
   Trp Gly Gln Gly Thr Leu Val Thr Val Ser Ser
           115                 120
<210> 44
   <211> 123
   <212> PRT
   <213> Homo sapiens
<400> 44
   Gln Val Gln Leu Val Gln Ser Gly Gly Gly Leu Val Gln Pro Gly Arg
   1 5 10 15
   Ser Leu Arg Leu Ser Cys Thr Thr Ser Gly Phe Thr Phe Ser Asp Tyr
   20 25 30
   Ala Leu Ser Trp Val Arg Gln Ala Pro Gly Arg Gly Leu Glu Trp Val
   35 40 45
   Gly Phe Ile Arg Asn Lys Ile Tyr Gly Gly Thr Thr Asp Tyr Ala Ala
   50 55 60
   Ser Val Lys Gly Arg Phe Thr Ile Ser Arg Asp Asp Ser Lys Ser Ile
   65 70 75 80
   Ala Tyr Leu Gln Met Asn Ser Leu Lys Thr Glu Asp Ser Ala Val Tyr
   85 90 95
   Tyr Cys Thr Arg Asp Ser Gly Val Val Thr Ala Ala Tyr Phe Asp Tyr
   100 105 110
   Trp Gly Gln Gly Thr Leu Val Thr Val Ser Ser
   115 120
<210> 45
   <211> 366
   <212> DNA
   <213> Homo sapiens
<220>
   <221> CDS
   <222> (1).. (366)
   <223>
<400> 45
   cag gtc cag ctg gtg caa tct ggg gga ggc gtg gtc cag cct ggg agg      48
   Gln Val Gln Leu Val Gln Ser Gly Gly Gly Val Val Gln Pro Gly Arg
   1               5                   10                  15
   tcc ctg aga ctc tcc tgt gca gcc tct gga ttc acc ttc agt agc aat      96
   Ser Leu Arg Leu Ser Cys Ala Ala Ser Gly Phe Thr Phe Ser Ser Asn
               20                  25                  30
   ggc atg cac tgg gtc cgc cag gct cca ggc aag ggg ctg gag tgg gtg      144
   Gly Met His Trp Val Arg Gln Ala Pro Gly Lys Gly Leu Glu Trp Val
           35                  40                  45
   gca gtt ata tgg tat gat gga agt aat aaa tac tat gca gac tcc gtg      192
   Ala Val Ile Trp Tyr Asp Gly Ser Asn Lys Tyr Tyr Ala Asp Ser Val
       50                  55                  60
   aag ggc cga ttc acc atc tcc aga gac aat tcc aag aac aca ctg tat      240
   Lys Gly Arg Phe Thr Ile Ser Arg Asp Asn Ser Lys Asn Thr Leu Tyr
   65                  70                  75                  80
   ctg caa atg aac agc ctg aga gcc gag gac acg gct gtg tat tac tgt      288
   Leu Gln Met Asn Ser Leu Arg Ala Glu Asp Thr Ala Val Tyr Tyr Cys
                   85                  90                  95
   gcg aga gat cac ggc ctt ggt gat caa gcc tcc tgg ttc gac ccc tgg      336
   Ala Arg Asp His Gly Leu Gly Asp Gln Ala Ser Trp Phe Asp Pro Trp
               100                 105                 110
   ggc cag ggg acc acg gtc acc gtc tcc tca                              366
   Gly Gln Gly Thr Thr Val Thr Val Ser Ser
           115                 120
<210> 46
   <211> 122
   <212> PRT
   <213> Homo sapiens
<400> 46
   Gln Val Gln Leu Val Gln Ser Gly Gly Gly Val Val Gln Pro Gly Arg
   1 5 10 15
   Ser Leu Arg Leu Ser Cys Ala Ala Ser Gly Phe Thr Phe Ser Ser Asn
   20 25 30
   Gly Met His Trp Val Arg Gln Ala Pro Gly Lys Gly Leu Glu Trp Val
   35 40 45
   Ala Val Ile Trp Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp His Gly Leu Gly Asp Gln Ala Ser Trp Phe Asp Pro Trp
   100 105 110
   Gly Gln Gly Thr Thr Val Thr Val Ser Ser
   115 120
<210> 47
   <211> 360
   <212> DNA
   <213> Homo sapiens
<220>
   <221> CDS
   <222> (1).. (360)
   <223>
<400> 47
   cag gtc cag ctg gtg caa tct ggg gct gag gtg aag aag cct ggg gcc 48
   Gln Val Gln Leu Val Gln Ser Gly Ala Glu Val Lys Lys Pro Gly Ala
   1 5 10 15
   tca gtg aag gtc tcc tgt cag gct tct gga tac atg ttc acc ggc ttc 96
   Ser Val Lys Val Ser Cys Gln Ala Ser Gly Tyr Met Phe Thr Gly Phe
   20 25 30
   tat atg cac tgg gtg cga cag gcc cct gga caa ggg ctt gag tgg atg 144
   Tyr Met His Trp Val Arg Gln Ala Pro Gly Gln Gly Leu Glu Trp Met
   35 40 45
   gga tgg atg aac act aac agt ggt gcc aca ggc tat gca cac aag ttt 192
   Gly Trp Met Asn Thr Asn Ser Gly Ala Thr Gly Tyr Ala His Lys Phe
   50 55 60
   cag gac agg gtc acc ctg acc agg gac acg tcc atc agc aca ggc tac 240
   Gln Asp Arg Val Thr Leu Thr Arg Asp Thr Ser Ile Ser Thr Gly Tyr
   65 70 75 80
   atg gag ctg ggc ggc ctg aca tct gac gac acg gcc gtg tat tat tgt 288
   Met Glu Leu Gly Gly Leu Thr Ser Asp Asp Thr Ala Val Tyr Tyr Cys
   85 90 95
   gcg aga acc cag gag gtt tac tac tac gct atg gac gtc tgg ggc caa 336
   Ala Arg Thr Gln Glu Val Tyr Tyr Tyr Ala Met Asp Val Trp Gly Gln
   100 105 110
   ggg aca atg gtc acc gtc tct tca 360
   Gly Thr Met Val Thr Val Ser Ser
   115 120
<210> 48
   <211> 120
   <212> PRT
   <213> Homo sapiens
<400> 48
   Gln Val Gln Leu Val Gln Ser Gly Ala Glu Val Lys Lys Pro Gly Ala
   1 5 10 15
   Ser Val Lys Val Ser Cys Gln Ala Ser Gly Tyr Met Phe Thr Gly Phe
   20 25 30
   Tyr Met His Trp Val Arg Gln Ala Pro Gly Gln Gly Leu Glu Trp Met
   35 40 45
   Gly Trp Met Asn Thr Asn Ser Gly Ala Thr Gly Tyr Ala His Lys Phe
   50 55 60
   Gln Asp Arg Val Thr Leu Thr Arg Asp Thr Ser Ile Ser Thr Gly Tyr
   65 70 75 80
   Met Glu Leu Gly Gly Leu Thr Ser Asp Asp Thr Ala Val Tyr Tyr Cys
   85 90 95
   Ala Arg Thr Gln Glu Val Tyr Tyr Tyr Ala Met Asp Val Trp Gly Gln
   100 105 110
   Gly Thr Met Val Thr Val Ser Ser
   115 120
<210> 49
   <211> 353
   <212> DNA
   <213> Homo sapiens
<220>
   <221> CDS
   <222> (1).. (351)
   <223>
<400> 49
   cag ctg gtg caa tct ggg gct gag gtg aag aag cct ggg gcc tca gtg 48
   Gln Leu Val Gln Ser Gly Ala Glu Val Lys Lys Pro Gly Ala Ser Val
   1 5 10 15
   aag gtc tcc tgt cag gct tct gga tac atg ttc acc ggc ttc tat atg 96
   Lys Val Ser Cys Gln Ala Ser Gly Tyr Met Phe Thr Gly Phe Tyr Met
   20 25 30
   cac tgg gtg cga cag gcc cct gga caa ggg ctt gag tgg atg gga tgg 144
   His Trp Val Arg Gln Ala Pro Gly Gln Gly Leu Glu Trp Met Gly Trp
   35 40 45
   atg aac act aac agt ggt gcc aca ggc tat gca cac aag ttt cag gac 192
   Met Asn Thr Asn Ser Gly Ala Thr Gly Tyr Ala His Lys Phe Gln Asp
   50 55 60
   agg gtc acc ctg acc agg gac acg tcc atc agc aca ggc tac atg gag 240
   Arg Val Thr Leu Thr Arg Asp Thr Ser Ile Ser Thr Gly Tyr Met Glu
   65 70 75 80
   ctg ggc ggc ctg aca tct gac gac acg gcc gtg tat tat tgt gcg aga 288
   Leu Gly Gly Leu Thr Ser Asp Asp Thr Ala Val Tyr Tyr Cys Ala Arg
   85 90 95
   acc cag gag gtt tac tac tac gct atg gac gtc tgg ggc caa ggg aca 336
   Thr Gln Glu Val Tyr Tyr Tyr Ala Met Asp Val Trp Gly Gln Gly Thr
   100 105 110
   atg gtc acc gtc tct tc 353
   Met Val Thr Val Ser
   115
<210> 50
   <211> 117
   <212> PRT
   <213> Homo sapiens
<400> 50
   Gln Leu Val Gln Ser Gly Ala Glu Val Lys Lys Pro Gly Ala Ser Val
   1               5                   10                  15
   Lys Val Ser Cys Gln Ala Ser Gly Tyr Met Phe Thr Gly Phe Tyr Met
               20                  25                  30
   His Trp Val Arg Gln Ala Pro Gly Gln Gly Leu Glu Trp Met Gly Trp
           35                  40                  45
   Met Asn Thr Asn Ser Gly Ala Thr Gly Tyr Ala His Lys Phe Gln Asp
       50                  55                  60
   Arg Val Thr Leu Thr Arg Asp Thr Ser Ile Ser Thr Gly Tyr Met Glu
   65                  70                  75                  80
   Leu Gly Gly Leu Thr Ser Asp Asp Thr Ala Val Tyr Tyr Cys Ala Arg
                   85                  90                  95
   Thr Gln Glu Val Tyr Tyr Tyr Ala Met Asp Val Trp Gly Gln Gly Thr
               100                 105                 110
   Met Val Thr Val Ser
           115
<210> 51
   <211> 360
   <212> DNA
   <213> Homo sapiens
<220>
   <221> CDS
   <222> (1).. (360)
   <223>
<400> 51
   cag gtc cag ctg gtg caa tct ggg gct gag gtg aag aag cct ggg gcc 48
   Gln Val Gln Leu Val Gln Ser Gly Ala Glu Val Lys Lys Pro Gly Ala
   1 5 10 15
   tca gtg aag gtc tcc tgt cag gct tct gga tac atg ttc acc ggc ttc 96
   Ser Val Lys Val Ser Cys Gln Ala Ser Gly Tyr Met Phe Thr Gly Phe
   20 25 30
   tat atg cac tgg gtg cga cag gcc cct gga caa ggg ctt gag tgg atg 144
   Tyr Met His Trp Val Arg Gln Ala Pro Gly Gln Gly Leu Glu Trp Met
   35 40 45
   gga tgg atg aac act aac agt ggt gcc aca ggc tat gca cac aag ttt 192
   Gly Trp Met Asn Thr Asn Ser Gly Ala Thr Gly Tyr Ala His Lys Phe
   50 55 60
   cag gac agg gtc acc ctg acc agg gac acg tcc atc agc aca ggc tac 240
   Gln Asp Arg Val Thr Leu Thr Arg Asp Thr Ser Ile Ser Thr Gly Tyr
   65 70 75 80
   atg gag ctg ggc ggc ctg aca tct gac gac acg gcc gtg tat tat tgt 288
   Met Glu Leu Gly Gly Leu Thr Ser Asp Asp Thr Ala Val Tyr Tyr Cys
   85 90 95
   gcg aga acc cag gag gtt tac tac tac gct atg gac gtc tgg ggc caa 336
   Ala Arg Thr Gln Glu Val Tyr Tyr Tyr Ala Met Asp Val Trp Gly Gln
   100 105 110
   gga acc ctg gtc acc gtc tct tca 360
   Gly Thr Leu Val Thr Val Ser Ser
   115 120
<210> 52
   <211> 120
   <212> PRT
   <213> Homo sapiens
<400> 52
   Gln Val Gln Leu Val Gln Ser Gly Ala Glu Val Lys Lys Pro Gly Ala
   1 5 10 15
   Ser Val Lys Val Ser Cys Gln Ala Ser Gly Tyr Met Phe Thr Gly Phe
   20 25 30
   Tyr Met His Trp Val Arg Gln Ala Pro Gly Gln Gly Leu Glu Trp Met
   35 40 45
   Gly Trp Met Asn Thr Asn Ser Gly Ala Thr Gly Tyr Ala His Lys Phe
   50 55 60
   Gln Asp Arg Val Thr Leu Thr Arg Asp Thr Ser Ile Ser Thr Gly Tyr
   65 70 75 80
   Met Glu Leu Gly Gly Leu Thr Ser Asp Asp Thr Ala Val Tyr Tyr Cys
   85 90 95
   Ala Arg Thr Gln Glu Val Tyr Tyr Tyr Ala Met Asp Val Trp Gly Gln
   100 105 110
   Gly Thr Leu Val Thr Val Ser Ser
   115 120
<210> 53
   <211> 357
   <212> DNA
   <213> Homo sapiens
<220>
   <221> CDS
   <222> (1)..(357)
   <223>
<400> 53
   gtc cag ctg gtg caa tct ggg gct gag gtg aag aag cct ggg gcc tca 48
   Val Gln Leu Val Gln Ser Gly Ala Glu Val Lys Lys Pro Gly Ala Ser
   1 5 10 15
   gtg aag gtc tcc tgt cag gct tct gga tac atg ttc acc ggc ttc tat       96
   Val Lys Val Ser Cys Gln Ala Ser Gly Tyr Met Phe Thr Gly Phe Tyr
               20                  25                  30
   atg cac tgg gtg cga cag gcc cct gga caa ggg ctt gag tgg atg gga      144
   Met His Trp Val Arg Gln Ala Pro Gly Gln Gly Leu Glu Trp Met Gly
           35                  40                  45
   tgg atg aac act aac agt ggt gcc aca ggc tat gca cac aag ttt cag      192
   Trp Met Asn Thr Asn Ser Gly Ala Thr Gly Tyr Ala His Lys Phe Gln
       50                  55                  60
   gac agg gtc acc ctg acc agg gac acg tcc atc agc aca ggc tac atg      240
   Asp Arg Val Thr Leu Thr Arg Asp Thr Ser Ile Ser Thr Gly Tyr Met
   65                  70                  75                  80
   gag ctg ggc ggc ctg aca tct gac gac acg gcc gtg tat tat tgt gcg      288
   Glu Leu Gly Gly Leu Thr Ser Asp Asp Thr Ala Val Tyr Tyr Cys Ala
                   85                  90                  95
   aga acc cag gag gtt tac tac tac gct atg gac gta ctg ggg cca agg      336
   Arg Thr Gln Glu Val Tyr Tyr Tyr Ala Met Asp Val Leu Gly Pro Arg
               100                 105                 110
   gac aat ggt cac cgt ctc ttc                                          357
   Asp Asn Gly His Arg Leu Phe
           115
<210> 54
   <211> 119
   <212> PRT
   <213> Homo sapiens
<400> 54
   Val Gln Leu Val Gln Ser Gly Ala Glu Val Lys Lys Pro Gly Ala Ser
   1 5 10 15
   Val Lys Val Ser Cys Gln Ala Ser Gly Tyr Met Phe Thr Gly Phe Tyr
   20 25 30
   Met His Trp Val Arg Gln Ala Pro Gly Gln Gly Leu Glu Trp Met Gly
   35 40 45
   Trp Met Asn Thr Asn Ser Gly Ala Thr Gly Tyr Ala His Lys Phe Gln
   50 55 60
   Asp Arg Val Thr Leu Thr Arg Asp Thr Ser Ile Ser Thr Gly Tyr Met
   65 70 75 80
   Glu Leu Gly Gly Leu Thr Ser Asp Asp Thr Ala Val Tyr Tyr Cys Ala
   85 90 95
   Arg Thr Gln Glu Val Tyr Tyr Tyr Ala Met Asp Val Leu Gly Pro Arg
   100 105 110
   Asp Asn Gly His Arg Leu Phe
   115
<210> 55
   <211> 342
   <212> DNA
   <213> Homo sapiens
<220>
   <221> CDS
   <222> (1)..(342)
   <223>
<400> 55
   gat att gtg atg acc cag act cca gac tcc ctg gct gtg tct ctg ggc 48
   Asp Ile Val Met Thr Gln Thr Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ctg ctc att tac tgg gca tct acc cgg gaa tcc ggg gtc 192
   Pro Pro Lys Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc acc ctg cag gct gaa gat gtg gca gtt tat tac tgt cag caa 288
   Ile Ser Thr Leu Gln Ala Glu Asp Val Ala Val Tyr Tyr Cys Gln Gln
   85 90 95
   tat tat agt act cct ccg acg ttc ggc caa ggg acc aag gtg gaa atc 336
   Tyr Tyr Ser Thr Pro Pro Thr Phe Gly Gln Gly Thr Lys Val Glu Ile
   100 105 110
   aaa cgt 342
   Lys Arg
<210> 56
   <211> 114
   <212> PRT
   <213> Homo sapiens
<400> 56
   Asp Ile Val Met Thr Gln Thr Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Thr Leu Gln Ala Glu Asp Val Ala Val Tyr Tyr Cys Gln Gln
   85 90 95
   Tyr Tyr Ser Thr Pro Pro Thr Phe Gly Gln Gly Thr Lys Val Glu Ile
   100 105 110
   Lys Arg
<210> 57
   <211> 337
   <212> DNA
   <213> Homo sapiens
<220>
   <221> CDS
   <222> (1).. (336)
   <223>
<400> 57
   gat ctt gtg atg act cag tct cca gac tcc ctg gct gtg tct ctg ggc      48
   Asp Leu Val Met Thr Gln Ser Pro Asp Ser Leu Ala Val Ser Leu Gly
   1               5                   10                  15
   gag agg gcc acc atc aac tgc aag tcc agc cag agt gtt tta tac agc      96
   Glu Arg Ala Thr Ile Asn Cys Lys Ser Ser Gln Ser Val Leu Tyr Ser
               20                  25                  30
   tcc aac aat aag aac tac tta gct tgg cac cag cag aaa cca gga cag      144
   Ser Asn Asn Lys Asn Tyr Leu Ala Trp His Gln Gln Lys Pro Gly Gln
           35                  40                  45
   cct cct aaa ctg ctc att tac tgg gca tct acc cgg gaa tcc ggg gtc      192
   Pro Pro Lys Leu Leu Ile Tyr Trp Ala Ser Thr Arg Glu Ser Gly Val
       50                  55                  60
   cct gac cga ttc agt ggc agc ggg tct ggg aca gat ttc act ctc acc      240
   Pro Asp Arg Phe Ser Gly Ser Gly Ser Gly Thr Asp Phe Thr Leu Thr
   65                  70                  75                  80
   atc agc acc ctg cag gct gaa gat gtg gca gtt tat tac tgt cag caa      288
   Ile Ser Thr Leu Gln Ala Glu Asp Val Ala Val Tyr Tyr Cys Gln Gln
                   85                  90                  95
   tat tat agt act cct ccg acg ttc ggc caa ggg acc aaa gtg gat atc a    337
   Tyr Tyr Ser Thr Pro Pro Thr Phe Gly Gln Gly Thr Lys Val Asp Ile
               100                 105                 110
<210> 58
   <211> 112
   <212> PRT
   <213> Homo sapiens

<400> 58
   Asp Leu Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp His Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Thr Leu Gln Ala Glu Asp Val Ala Val Tyr Tyr Cys Gln Gln
                   85                  90                  95
   Tyr Tyr Ser Thr Pro Pro Thr Phe Gly Gln Gly Thr Lys Val Asp Ile
               100                 105                 110
<210> 59
   <211> 342
   <212> DNA
   <213> Homo sapiens
<220>
   <221> CDS
   <222> (1).. (342)
   <223>
<400> 59
   gat att gtg atg act cag tct cca gac tcc ctg gct gtg tct ctg ggc 48
   Asp Ile Val Met Thr Gln Ser Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ctg ctc att tac tgg gca tct acc cgg gaa tcc ggg gtc 192
   Pro Pro Lys Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc acc ctg cag gct gaa gat gtg gca gtt tat tac tgt cag caa 288
   Ile Ser Thr Leu Gln Ala Glu Asp Val Ala Val Tyr Tyr Cys Gln Gln
   85 90 95
   tat tat agt act cct ccg acg ttc ggc caa ggg acc aag gtg gaa atc 336
   Tyr Tyr Ser Thr Pro Pro Thr Phe Gly Gln Gly Thr Lys Val Glu Ile
   100 105 110
   aaa cgt 342
   Lys Arg
<210> 60
   <211> 114
   <212> PRT
   <213> Homo sapiens
<400> 60
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Thr Leu Gln Ala Glu Asp Val Ala Val Tyr Tyr Cys Gln Gln
                   85                  90                  95
   Tyr Tyr Ser Thr Pro Pro Thr Phe Gly Gln Gly Thr Lys Val Glu Ile
               100                 105                 110
   Lys Arg
<210> 61
   <211> 342
   <212> DNA
   <213> Homo sapiens
<220>
   <221> CDS
   <222> (1).. (342)
   <223>
<400> 61
   gat att gtg atg act cag tct cca ctc tcc ctg ccc gtc acc cct gga 48
   Asp Ile Val Met Thr Gln Ser Pro Leu Ser Leu Pro Val Thr Pro Gly
   1 5 10 15
   gag ccg gcc tcc atc tcc tgc agg tct agt cag agc ctc ttg gat agt 96
   Glu Pro Ala Ser Ile Ser Cys Arg Ser Ser Gln Ser Leu Leu Asp Ser
   20 25 30
   gat gat gga aac acc tat ttg gac tgg tac ctg cag aag cca ggg cag 144
   Asp Asp Gly Asn Thr Tyr Leu Asp Trp Tyr Leu Gln Lys Pro Gly Gln
   35 40 45
   tct cca cag ctc cta atc tat acg ctt tcc tat cgg gcc tct gga gtc 192
   Ser Pro Gln Leu Leu Ile Tyr Thr Leu Ser Tyr Arg Ala Ser Gly Val
   50 55 60
   cca gac agg ttc agt ggc agt ggg tca ggc act gat ttc aca ctg aaa 240
   Pro Asp Arg Phe Ser Gly Ser Gly Ser Gly Thr Asp Phe Thr Leu Lys
   65 70 75 80
   atc agc agg gtg gag gct gag gat gtt gga gtt tat tac tgc atg caa 288
   Ile Ser Arg Val Glu Ala Glu Asp Val Gly Val Tyr Tyr Cys Met Gln
   85 90 95
   cgt ata gag ttt cct tac act ttt ggc cag ggg acc aaa gtg gat atc 336
   Arg Ile Glu Phe Pro Tyr Thr Phe Gly Gln Gly Thr Lys Val Asp Ile
   100 105 110
   aaa cgt 342
   Lys Arg
<210> 62
   <211> 114
   <212> PRT
   <213> Homo sapiens
<400> 62
   Asp Ile Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu Asp Ser
   20 25 30
   Asp Asp Gly Asn Thr Tyr Leu Asp Trp Tyr Leu Gln Lys Pro Gly Gln
   35 40 45
   Ser Pro Gln Leu Leu Ile Tyr Thr Leu Ser Tyr Arg Ala Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Tyr Cys Met Gln
   85 90 95
   Arg Ile Glu Phe Pro Tyr Thr Phe Gly Gln Gly Thr Lys Val Asp Ile
   100 105 110
   Lys Arg
<210> 63
   <211> 342
   <212> DNA
   <213> Homo sapiens
<220>
   <221> CDS
   <222> (1).. (342)
   <223>
<400> 63
   gat gtt gtg atg act cag tct cca ctc tcc ctg ccc gtc acc cct gga 48
   Asp Val Val Met Thr Gln Ser Pro Leu Ser Leu Pro Val Thr Pro Gly
   1 5 10 15
   gag ccg gcc tcc atc tcc tgc agg tct agt cag agc ctc ttg gat agt 96
   Glu Pro Ala Ser Ile Ser Cys Arg Ser Ser Gln Ser Leu Leu Asp Ser
   20 25 30
   gat gat gga aac acc tat ttg gac tgg tac ctg cag aag cca ggg cag 144
   Asp Asp Gly Asn Thr Tyr Leu Asp Trp Tyr Leu Gln Lys Pro Gly Gln
   35 40 45
   tct cca cag ctc cta atc tat acg ctt tcc tat cgg gcc tct gga gtc 192
   Ser Pro Gln Leu Leu Ile Tyr Thr Leu Ser Tyr Arg Ala Ser Gly Val
   50 55 60
   cca gac agg ttc agt ggc agt ggg tca ggc act gat ttc aca ctg aaa 240
   Pro Asp Arg Phe Ser Gly Ser Gly Ser Gly Thr Asp Phe Thr Leu Lys
   65 70 75 80
   atc agc agg gtg gag gct gag gat gtt gga gtt tat tac tgc atg caa 288
   Ile Ser Arg Val Glu Ala Glu Asp Val Gly Val Tyr Tyr Cys Met Gln
   85 90 95
   cgt ata gag ttt cct tac act ttt ggc cag ggg acc aag gtg gaa atc 336
   Arg Ile Glu Phe Pro Tyr Thr Phe Gly Gln Gly Thr Lys Val Glu Ile
   100 105 110
   aaa cgt 342
   Lys Arg
<210> 64
   <211> 114
   <212> PRT
   <213> Homo sapiens
<400> 64
   Asp Val Val Met Thr Gln Ser Pro Leu Ser Leu Pro Val Thr Pro Gly
   1 5 10 15
   Glu Pro Ala Ser Ile Ser Cys Arg Ser Ser Gln Ser Leu Leu Asp Ser
   20 25 30
   Asp Asp Gly Asn Thr Tyr Leu Asp Trp Tyr Leu Gln Lys Pro Gly Gln
   35 40 45
   Ser Pro Gln Leu Leu Ile Tyr Thr Leu Ser Tyr Arg Ala Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Tyr Cys Met Gln
   85 90 95
   Arg Ile Glu Phe Pro Tyr Thr Phe Gly Gln Gly Thr Lys Val Glu Ile
   100 105 110
   Lys Arg
<210> 65
   <211> 339
   <212> DNA
   <213> Homo sapiens
<220>
   <221> CDS
   <222> (1).. (339)
   <223>
<400> 65
   gat att gtg atg acc cag act cca ctc tcc ctg ccc gtc acc cct gga 48
   Asp Ile Val Met Thr Gln Thr Pro Leu Ser Leu Pro Val Thr Pro Gly
   1 5 10 15
   gag ccg gcc tcc atc tcc tgc agg tct agt cag agc ctc ttg gat agt 96
   Glu Pro Ala Ser Ile Ser Cys Arg Ser Ser Gln Ser Leu Leu Asp Ser
   20 25 30
   gat gat gga aac acc tat ttg gac tgg tac ctg cag aag cca ggg cag 144
   Asp Asp Gly Asn Thr Tyr Leu Asp Trp Tyr Leu Gln Lys Pro Gly Gln
   35 40 45
   tct cca cag ctc cta atc tat acg ctt tcc tat cgg gcc tct gga gtc 192
   Ser Pro Gln Leu Leu Ile Tyr Thr Leu Ser Tyr Arg Ala Ser Gly Val
   50 55 60
   cca gac agg ttc agt ggc agt ggg tca ggc act gat ttc aca ctg aaa 240
   Pro Asp Arg Phe Ser Gly Ser Gly Ser Gly Thr Asp Phe Thr Leu Lys
   65 70 75 80
   atc agc agg gtg gag gct gag gat gtt gga gtt tat tac tgc atg caa 288
   Ile Ser Arg Val Glu Ala Glu Asp Val Gly Val Tyr Tyr Cys Met Gln
   85 90 95
   gct aca caa ttg tac act ttt ggc cag ggg acc aag gtg gag atc aaa 336
   Ala Thr Gln Leu Tyr Thr Phe Gly Gln Gly Thr Lys Val Glu Ile Lys
   100 105 110
   cgt 339
   Arg
<210> 66
   <211> 113
   <212> PRT
   <213> Homo sapiens
<400> 66
   Asp Ile Val Met Thr Gln Thr Pro Leu Ser Leu Pro Val Thr Pro Gly
   1 5 10 15
   Glu Pro Ala Ser Ile Ser Cys Arg Ser Ser Gln Ser Leu Leu Asp Ser
   20 25 30
   Asp Asp Gly Asn Thr Tyr Leu Asp Trp Tyr Leu Gln Lys Pro Gly Gln
   35 40 45
   Ser Pro Gln Leu Leu Ile Tyr Thr Leu Ser Tyr Arg Ala Ser Gly Val
   50 55 60
   Pro Asp Arg Phe Ser Gly Ser Gly Ser Gly Thr Asp Phe Thr Leu Lys
   65 70 75 80
   Ile Ser Arg Val Glu Ala Glu Asp Val Gly Val Tyr Tyr Cys Met Gln
   85 90 95
   Ala Thr Gln Leu Tyr Thr Phe Gly Gln Gly Thr Lys Val Glu Ile Lys
   100 105 110
   Arg
<210> 67
   <211> 342
   <212> DNA
   <213> Homo sapiens
<220>
   <221> CDS
   <222> (1).. (342)
   <223>
<400> 67
   gat att gtg atg act cag tct cca gac tcc ctg gct gtg tct ctg ggc 48
   Asp Ile Val Met Thr Gln Ser Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ctg ctc att tac tgg gca tct acc cgg gaa tcc ggg gtc 192
   Pro Pro Lys Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc acc ctg cag gct gaa gat gtg gca gtt tat tac tgt cag caa 288
   Ile Ser Thr Leu Gln Ala Glu Asp Val Ala Val Tyr Tyr Cys Gln Gln
   85 90 95
   tat tat agt act cct ccg acg ttc ggc caa ggg acc aag ctg gag atc 336
   Tyr Tyr Ser Thr Pro Pro Thr Phe Gly Gln Gly Thr Lys Leu Glu Ile
   100 105 110
   aaa cgt 342
   Lys Arg
<210> 68
   <211> 114
   <212> PRT
   <213> Homo sapiens
<400> 68
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Thr Leu Gln Ala Glu Asp Val Ala Val Tyr Tyr Cys Gln Gln
   85 90 95
   Tyr Tyr Ser Thr Pro Pro Thr Phe Gly Gln Gly Thr Lys Leu Glu Ile
   100 105 110
   Lys Arg
<210> 69
   <211> 342
   <212> DNA
   <213> Homo sapiens
<220>
   <221> CDS
   <222> (1).. (342)
   <223>
<400> 69
   gat gtt gtg atg act cag act cca gac tcc ctg gct gtg tct ctg ggc 48
   Asp Val Val Met Thr Gln Thr Pro Asp Ser Leu Ala Val Ser Leu Gly
   1 5 10 15
   gag agg gcc acc atc aac tgc aag tcc agc cag agt gtt tta cac aag 96
   Glu Arg Ala Thr Ile Asn Cys Lys Ser Ser Gln Ser Val Leu His Lys
   20 25 30
   tcc aac aat aag aac tat tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ttg ctc att cac tgg gct tct acc cgg gaa ttc ggg gtc 192
   Pro Pro Lys Leu Leu Ile His Trp Ala Ser Thr Arg Glu Phe Gly Val
   50 55 60
   cct gac cga ctc agt ggc agc ggg tct gcg aca gat ttc act ctc acc 240
   Pro Asp Arg Leu Ser Gly Ser Gly Ser Ala Thr Asp Phe Thr Leu Thr
   65 70 75 80
   atc agc agc ctg cag gct gaa gac gtg gca gtc tat tac tgt cag caa 288
   Ile Ser Ser Leu Gln Ala Glu Asp Val Ala Val Tyr Tyr Cys Gln Gln
   85 90 95
   tat tat gct gtt cct ctc acc ttc ggc caa ggg aca cga ctg gag att 336
   Tyr Tyr Ala Val Pro Leu Thr Phe Gly Gln Gly Thr Arg Leu Glu Ile
   100 105 110
   aaa cgt 342
   Lys Arg
<210> 70
   <211> 114
   <212> PRT
   <213> Homo sapiens
<400> 70
   Asp Val Val Met Thr Gln Thr Pro Asp Ser Leu Ala Val Ser Leu Gly
   1 5 10 15
   Glu Arg Ala Thr Ile Asn Cys Lys Ser Ser Gln Ser Val Leu His Lys
   20 25 30
   Ser Asn Asn Lys Asn Tyr Leu Ala Trp Tyr Gln Gln Lys Pro Gly Gln
   35 40 45
   Pro Pro Lys Leu Leu Ile His Trp Ala Ser Thr Arg Glu Phe Gly Val
   50 55 60
   Pro Asp Arg Leu Ser Gly Ser Gly Ser Ala Thr Asp Phe Thr Leu Thr
   65 70 75 80
   Ile Ser Ser Leu Gln Ala Glu Asp Val Ala Val Tyr Tyr Cys Gln Gln
   85 90 95
   Tyr Tyr Ala Val Pro Leu Thr Phe Gly Gln Gly Thr Arg Leu Glu Ile
   100 105 110
   Lys Arg
<210> 71
   <211> 342
   <212> DNA
   <213> Homo sapiens
<220>
   <221> CDS
   <222> (1).. (342)
   <223>
<400> 71
   gat att gtg atg acc cag acg cca gac tcc ctg gct gtg tct ctg ggc 48
   Asp Ile Val Met Thr Gln Thr Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ctg ctc att tac tgg gca tct acc cgg gaa tcc ggg gtc 192
   Pro Pro Lys Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc acc ctg cag gct gaa gat gtg gca gtt tat tac tgt cag caa 288
   Ile Ser Thr Leu Gln Ala Glu Asp Val Ala Val Tyr Tyr Cys Gln Gln
   85 90 95
   tat tat agt act cct ccg acg ttc agc caa ggg acc aag gtg gaa atc 336
   Tyr Tyr Ser Thr Pro Pro Thr Phe Ser Gln Gly Thr Lys Val Glu Ile
   100 105 110
   aaa cgt 342
   Lys Arg
<210> 72
   <211> 114
   <212> PRT
   <213> Homo sapiens
<400> 72
   Asp Ile Val Met Thr Gln Thr Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Thr Leu Gln Ala Glu Asp Val Ala Val Tyr Tyr Cys Gln Gln
   85 90 95
   Tyr Tyr Ser Thr Pro Pro Thr Phe Ser Gln Gly Thr Lys Val Glu Ile
   100 105 110
   Lys Arg
<210> 73
   <211> 342
   <212> DNA
   <213> Homo sapiens
<220>
   <221> CDS
   <222> (1).. (342)
   <223>
<400> 73
   gat gtt gtg atg act cag tct cca gac tcc ctg act gtg tct ctg ggc 48
   Asp Val Val Met Thr Gln Ser Pro Asp Ser Leu Thr Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aag ctg ctc att tac tgg gca cct acc cgg gaa tcc ggg gtc 192
   Pro Pro Lys Leu Leu Ile Tyr Trp Ala Pro Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc agc ctg cag gct gaa gat gtg gca gtt tat tac tgt cag caa 288
   Ile Ser Ser Leu Gln Ala Glu Asp Val Ala Val Tyr Tyr Cys Gln Gln
   85 90 95
   tat tat agt act cct ccg acg ttc ggc cag ggg acc aag gtg gaa atc 336
   Tyr Tyr Ser Thr Pro Pro Thr Phe Gly Gln Gly Thr Lys Val Glu Ile
   100 105 110
   aaa cgt 342
   Lys Arg
<210> 74
   <211> 114
   <212> PRT
   <213> Homo sapiens
<400> 74
   Asp Val Val Met Thr Gln Ser Pro Asp Ser Leu Thr Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Pro Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Ser Leu Gln Ala Glu Asp Val Ala Val Tyr Tyr Cys Gln Gln
   85 90 95
   Tyr Tyr Ser Thr Pro Pro Thr Phe Gly Gln Gly Thr Lys Val Glu Ile
   100 105 110
   Lys Arg
<210> 75
   <211> 342
   <212> DNA
   <213> Homo sapiens
<220>
   <221> CDS
   <222> (1).. (342)
   <223>
<400> 75
   gat gtt gtg atg act cag tct cca gac tcc ctg gct gtg tct ctg ggc 48
   Asp Val Val Met Thr Gln Ser Pro Asp Ser Leu Ala Val Ser Leu Gly
   1 5 10 15
   gag agg gcc acc atc aac tgc aag tcc agc cag ggt gtt tta cac aag 96
   Glu Arg Ala Thr Ile Asn Cys Lys Ser Ser Gln Gly Val Leu His Lys
   20 25 30
   tcc aac aat aag aac tat tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ttg ctc att cac tgg gct tct acc cgg gaa ttc ggg gtc 192
   Pro Pro Lys Leu Leu Ile His Trp Ala Ser Thr Arg Glu Phe Gly Val
   50 55 60
   cct gac cga ctc agt ggc agc ggg tct gcg aca gat ttc act ctc acc 240
   Pro Asp Arg Leu Ser Gly Ser Gly Ser Ala Thr Asp Phe Thr Leu Thr
   65 70 75 80
   atc agc agc ctg cag gct gaa gac gtg gca gtc tat tac tgt cag caa 288
   Ile Ser Ser Leu Gln Ala Glu Asp Val Ala Val Tyr Tyr Cys Gln Gln
   85 90 95
   tat tat gct gtt cct ctc acc ttc ggc caa ggg aca cga ctg gag att 336
   Tyr Tyr Ala Val Pro Leu Thr Phe Gly Gln Gly Thr Arg Leu Glu Ile
   100 105 110
   aaa cgt 342
   Lys Arg
<210> 76
   <211> 114
   <212> PRT
   <213> Homo sapiens
<400> 76
   Asp Val Val Met Thr Gln Ser Pro Asp Ser Leu Ala Val Ser Leu Gly
   1               5                   10                  15
   Glu Arg Ala Thr Ile Asn Cys Lys Ser Ser Gln Gly Val Leu His Lys
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile His Trp Ala Ser Thr Arg Glu Phe Gly Val
       50                  55                  60
   Pro Asp Arg Leu Ser Gly Ser Gly Ser Ala Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ala Val Pro Leu Thr Phe Gly Gln Gly Thr Arg Leu Glu Ile
               100                 105                 110
   Lys Arg
<210> 77
   <211> 342
   <212> DNA
   <213> Homo sapiens
<220>
   <221> CDS
   <222> (1).. (342)
   <223>
<400> 77
   gat att gtg atg acc cag acg cca gac tcc ctg gct gtg tct ctg ggc 48
   Asp Ile Val Met Thr Gln Thr Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ctg ctc att tac tgg gca tct acc cgg gaa tcc ggg gtc 192
   Pro Pro Lys Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc acc ctg cag gct gaa gat gtg gca gtt tat tac tgt cag caa 288
   Ile Ser Thr Leu Gln Ala Glu Asp Val Ala Val Tyr Tyr Cys Gln Gln
   85 90 95
   tat tat agt act cct ccg acg ttc ggc caa ggg acc aag gtg gaa atc 336
   Tyr Tyr Ser Thr Pro Pro Thr Phe Gly Gln Gly Thr Lys Val Glu Ile
   100 105 110
   aaa cgt 342
   Lys Arg

<210> 78
   <211> 114
   <212> PRT
   <213> Homo sapiens
<400> 78
   Asp Ile Val Met Thr Gln Thr Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Thr Leu Gln Ala Glu Asp Val Ala Val Tyr Tyr Cys Gln Gln
   85 90 95
   Tyr Tyr Ser Thr Pro Pro Thr Phe Gly Gln Gly Thr Lys Val Glu Ile
   100 105 110
   Lys Arg
<210> 79
   <211> 342
   <212> DNA
   <213> Homo sapiens
<220>
   <221> CDS
   <222> (1).. (342)
   <223>
<400> 79
   gat gtt gtg atg act cag tct cca gac tcc ctg gct gtg cct ctg ggc 48
   Asp Val Val Met Thr Gln Ser Pro Asp Ser Leu Ala Val Pro Leu Gly
   1 5 10 15
   gag agg gcc acc atc aac tgc aag tcc agc cag agt gtt tta cac aag 96
   Glu Arg Ala Thr Ile Asn Cys Lys Ser Ser Gln Ser Val Leu His Lys
   20 25 30
   tcc aac aat aag aac cat tta gct tgg tac cag cag aaa cca gga cag 144
   Ser Asn Asn Lys Asn His Leu Ala Trp Tyr Gln Gln Lys Pro Gly Gln
   35 40 45
   cct cct aaa ttg ctc att cac tgg gct tct acc cgg gaa ttc ggg gtc 192
   Pro Pro Lys Leu Leu Ile His Trp Ala Ser Thr Arg Glu Phe Gly Val
   50 55 60
   cct gac cga ctc agt ggc agc ggg tct gcg aca gat ttc act ctc acc 240
   Pro Asp Arg Leu Ser Gly Ser Gly Ser Ala Thr Asp Phe Thr Leu Thr
   65 70 75 80
   atc aac agc ctg cag gct gaa gac gcg gca gtc tat tac tgt cag caa 288
   Ile Asn Ser Leu Gln Ala Glu Asp Ala Ala Val Tyr Tyr Cys Gln Gln
   85 90 95
   tat tat gct gtt cct ctc acc ttc ggc caa ggg aca cga ctg gag att 336
   Tyr Tyr Ala Val Pro Leu Thr Phe Gly Gln Gly Thr Arg Leu Glu Ile
   100 105 110
   aaa cgt 342
   Lys Arg
<210> 80
   <211> 114
   <212> PRT
   <213> Homo sapiens
<400> 80
   Asp Val Val Met Thr Gln Ser Pro Asp Ser Leu Ala Val Pro Leu Gly
   1 5 10 15
   Glu Arg Ala Thr Ile Asn Cys Lys Ser Ser Gln Ser Val Leu His Lys
   20 25 30
   Ser Asn Asn Lys Asn His Leu Ala Trp Tyr Gln Gln Lys Pro Gly Gln
   35 40 45
   Pro Pro Lys Leu Leu Ile His Trp Ala Ser Thr Arg Glu Phe Gly Val
   50 55 60
   Pro Asp Arg Leu Ser Gly Ser Gly Ser Ala Thr Asp Phe Thr Leu Thr
   65 70 75 80
   Ile Asn Ser Leu Gln Ala Glu Asp Ala Ala Val Tyr Tyr Cys Gln Gln
   85 90 95
   Tyr Tyr Ala Val Pro Leu Thr Phe Gly Gln Gly Thr Arg Leu Glu Ile
   100 105 110
   Lys Arg
<210> 81
   <211> 342
   <212> DNA
   <213> Homo sapiens
<220>
   <221> CDS
   <222> (1).. (342)
   <223>
<400> 81
   gat att gtg atg act cag tct cca gac tcc ctg gct gtg tct ctg ggc 48
   Asp Ile Val Met Thr Gln Ser Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ctg ctc att tac tgg gca tct acc cgg gaa tcc ggg gtc 192
   Pro Pro Lys Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc acc ctg cag gct gaa gat gtg gca gtt tat tac tgt cag caa 288
   Ile Ser Thr Leu Gln Ala Glu Asp Val Ala Val Tyr Tyr Cys Gln Gln
   85 90 95
   tat tat agt act cct ccg acg ttc ggc caa ggg acc aaa gtg gat atc 336
   Tyr Tyr Ser Thr Pro Pro Thr Phe Gly Gln Gly Thr Lys Val Asp Ile
   100 105 110
   aaa cgt 342
   Lys Arg
<210> 82
   <211> 114
   <212> PRT
   <213> Homo sapiens
<400> 82
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Thr Leu Gln Ala Glu Asp Val Ala Val Tyr Tyr Cys Gln Gln
   85 90 95
   Tyr Tyr Ser Thr Pro Pro Thr Phe Gly Gln Gly Thr Lys Val Asp Ile
   100 105 110
   Lys Arg
<210> 83
   <211> 342
   <212> DNA
   <213> Homo sapiens
<220>
   <221> CDS
   <222> (1).. (342)
   <223>
<400> 83
   gat gtt gtg atg act cag tct cca gac tcc ctg gct gtg tct ctg ggc 48
   Asp Val Val Met Thr Gln Ser Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aaa ttg ctc att cac tgg gct tct acc cgg gaa ttc ggg gtc 192
   Pro Pro Lys Leu Leu Ile His Trp Ala Ser Thr Arg Glu Phe Gly Val
   50 55 60
   cct gac cga ctc agt ggc agc ggg tct gcg aca gat ttc act ctc acc 240
   Pro Asp Arg Leu Ser Gly Ser Gly Ser Ala Thr Asp Phe Thr Leu Thr
   65 70 75 80
   atc agc agc ctg cag gct gaa gac gtg gca gtc tat tac tgt cag caa 288
   Ile Ser Ser Leu Gln Ala Glu Asp Val Ala Val Tyr Tyr Cys Gln Gln
   85 90 95
   tat tat gct gtt cct ctc acc ttc ggc caa ggg aca cga ctg gag att 336
   Tyr Tyr Ala Val Pro Leu Thr Phe Gly Gln Gly Thr Arg Leu Glu Ile
   100 105 110
   aaa cgt 342
   Lys Arg
<210> 84
   <211> 114
   <212> PRT
   <213> Homo sapiens
<400> 84
   Asp Val Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile His Trp Ala Ser Thr Arg Glu Phe Gly Val
   50 55 60
   Pro Asp Arg Leu Ser Gly Ser Gly Ser Ala Thr Asp Phe Thr Leu Thr
   65 70 75 80
   Ile Ser Ser Leu Gln Ala Glu Asp Val Ala Val Tyr Tyr Cys Gln Gln
   85 90 95
   Tyr Tyr Ala Val Pro Leu Thr Phe Gly Gln Gly Thr Arg Leu Glu Ile
   100 105 110
   Lys Arg
<210> 85
   <211> 342
   <212> DNA
   <213> Homo sapiens
<220>
   <221> CDS
   <222> (1).. (342)
   <223>
<400> 85
   gac atc gtg atg acc cag tct cca gac tcc ctg gct gtg tct ctg ggc     48
   Asp Ile Val Met Thr Gln Ser Pro Asp Ser Leu Ala Val Ser Leu Gly
   1               5                   10                  15
   gag agg gcc acc atc aac tgc aag tcc agc cag agt gtt tta tac agc     96
   Glu Arg Ala Thr Ile Asn Cys Lys Ser Ser Gln Ser Val Leu Tyr Ser
               20                  25                  30
   tcc aac aat aag aac tac tta gct tgg tac cag cag aaa cca gga cag     144
   Ser Asn Asn Lys Asn Tyr Leu Ala Trp Tyr Gln Gln Lys Pro Gly Gln
           35                  40                  45
   cct cct aaa ctg ctc att tac tgg gca tct acc cgg gaa tcc ggg gtc     192
   Pro Pro Lys Leu Leu Ile Tyr Trp Ala Ser Thr Arg Glu Ser Gly Val
       50                  55                  60
   cct gac cga ttc agt ggc agc ggg tct ggg aca gat ttc act ctc acc     240
   Pro Asp Arg Phe Ser Gly Ser Gly Ser Gly Thr Asp Phe Thr Leu Thr
   65                  70                  75                  80
   atc agc acc ctg cag gct gaa gat gtg gca gtt tat tac tgt cag caa     288
   Ile Ser Thr Leu Gln Ala Glu Asp Val Ala Val Tyr Tyr Cys Gln Gln
                   85                  90                  95
   tat tat agt act cct ccg acg ttc ggc caa ggg acc aag gtg gaa atc     336
   Tyr Tyr Ser Thr Pro Pro Thr Phe Gly Gln Gly Thr Lys Val Glu Ile
               100                 105                 110
   aaa cgt                                                             342
   Lys Arg
<210> 86
   <211> 114
   <212> PRT
   <213> Homo sapiens
<400> 86
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Ser
   20 25 30
   Ser Asn Asn Lys Asn Tyr Leu Ala Trp Tyr Gln Gln Lys Pro Gly Gln
   35 40 45
   Pro Pro Lys Leu Leu Ile Tyr Trp Ala Ser Thr Arg Glu Ser Gly Val
   50 55 60
   Pro Asp Arg Phe Ser Gly Ser Gly Ser Gly Thr Asp Phe Thr Leu Thr
   65 70 75 80
   Ile Ser Thr Leu Gln Ala Glu Asp Val Ala Val Tyr Tyr Cys Gln Gln
   85 90 95
   Tyr Tyr Ser Thr Pro Pro Thr Phe Gly Gln Gly Thr Lys Val Glu Ile
   100 105 110
   Lys Arg

<210> 87
   <211> 327
   <212> DNA
   <213> Homo sapiens
<220>
   <221> CDS
   <222> (1).. (327)
   <223>
<400> 87
   gaa att gtg ctg act cag tct cca ggc acc ctg tct ttg tct cca ggg 48
   Glu Ile Val Leu Thr Gln Ser Pro Gly Thr Leu Ser Leu Ser Pro Gly
   1 5 10 15
   gaa aga gcc acc ctc tcc tgc aag gcc agt cag agt ttt agc agc aac 96
   Glu Arg Ala Thr Leu Ser Cys Lys Ala Ser Gln Ser Phe Ser Ser Asn
   20 25 30
   tac tta gcc tgg tac cag cag aaa cct ggc cag gct ccc agg ctg ctc 144
   Tyr Leu Ala Trp Tyr Gln Gln Lys Pro Gly Gln Ala Pro Arg Leu Leu
   35 40 45
   atc tat ggt gca tcc agc agg gcc act ggc atc cca gac agg ttc agt 192
   Ile Tyr Gly Ala Ser Ser Arg Ala Thr Gly Ile Pro Asp Arg Phe Ser
   50 55 60
   ggc agt aaa tct ggg aca gac ttc act ctc acc atc agc aga ctg gag 240
   Gly Ser Lys Ser Gly Thr Asp Phe Thr Leu Thr Ile Ser Arg Leu Glu
   65 70 75 80
   cct gaa gat ttt gca gtg tat tac tgt cag cag tat gtt acc tca ccg 288
   Pro Glu Asp Phe Ala Val Tyr Tyr Cys Gln Gln Tyr Val Thr Ser Pro
   85 90 95
   tac act ttt ggc ctg ggg acc aag gtg gag atc aaa cgt 327
   Tyr Thr Phe Gly Leu Gly Thr Lys Val Glu Ile Lys Arg
   100 105
<210> 88
   <211> 109
   <212> PRT
   <213> Homo sapiens
<400> 88
   Glu Ile Val Leu Thr Gln Ser Pro Gly Thr Leu Ser Leu Ser Pro Gly
   1               5                   10                  15
   Glu Arg Ala Thr Leu Ser Cys Lys Ala Ser Gln Ser Phe Ser Ser Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Lys Ser Gly Thr Asp Phe Thr Leu Thr Ile Ser Arg Leu Glu
   65                  70                  75                  80
   Pro Glu Asp Phe Ala Val Tyr Tyr Cys Gln Gln Tyr Val Thr Ser Pro
                   85                  90                  95
   Tyr Thr Phe Gly Leu Gly Thr Lys Val Glu Ile Lys Arg
               100                 105
<210> 89
   <211> 325
   <212> DNA
   <213> Homo sapiens
<220>
   <221> CDS
   <222> (1).. (324)
   <223>
<400> 89
   gat gtt ggg atg aca cag tct tca gcc acc cta tct ttg tct cca ggg 48
   Asp Val Gly Met Thr Gln Ser Ser Ala Thr Leu Ser Leu Ser Pro Gly
   1 5 10 15
   gaa aga gcc acc ctc tcc tgc agg gcc agt cag agg att agc agt tat 96
   Glu Arg Ala Thr Leu Ser Cys Arg Ala Ser Gln Arg Ile Ser Ser Tyr
   20 25 30
   tta gcc tgg tac caa cag aaa cct ggc cag gct ccc aga ctc ctc atc 144
   Leu Ala Trp Tyr Gln Gln Lys Pro Gly Gln Ala Pro Arg Leu Leu Ile
   35 40 45
   tat gag gca gtc aaa agg gcc act ggc atc cca gcc agg ttc agt ggc 192
   Tyr Glu Ala Val Lys Arg Ala Thr Gly Ile Pro Ala Arg Phe Ser Gly
   50 55 60
   agt ggg tct ggg aca gag ttc acc ctc acc atc aac agc cta gag cct 240
   Ser Gly Ser Gly Thr Glu Phe Thr Leu Thr Ile Asn Ser Leu Glu Pro
   65 70 75 80
   gaa gat ttt gca gtt tat ttc tgt cag cag cgt ggc agc tgt cct ggg 288
   Glu Asp Phe Ala Val Tyr Phe Cys Gln Gln Arg Gly Ser Cys Pro Gly
   85 90 95
   acg ttc ggc cag ggg acc aag ctg gag atc aaa cgt t 325
   Thr Phe Gly Gln Gly Thr Lys Leu Glu Ile Lys Arg
   100 105
<210> 90
   <211> 108
   <212> PRT
   <213> Homo sapiens
<400> 90
   Asp Val Gly Met Thr Gln Ser Ser Ala Thr Leu Ser Leu Ser Pro Gly
   1 5 10 15
   Glu Arg Ala Thr Leu Ser Cys Arg Ala Ser Gln Arg Ile Ser Ser Tyr
   20 25 30
   Leu Ala Trp Tyr Gln Gln Lys Pro Gly Gln Ala Pro Arg Leu Leu Ile
   35 40 45
   Tyr Glu Ala Val Lys Arg Ala Thr Gly Ile Pro Ala Arg Phe Ser Gly
   50 55 60
   Ser Gly Ser Gly Thr Glu Phe Thr Leu Thr Ile Asn Ser Leu Glu Pro
   65 70 75 80
   Glu Asp Phe Ala Val Tyr Phe Cys Gln Gln Arg Gly Ser Cys Pro Gly
   85 90 95
   Thr Phe Gly Gln Gly Thr Lys Leu Glu Ile Lys Arg
   100 105
<210> 91
   <211> 366
   <212> DNA
   <213> Homo sapiens
<220>
   <221> CDS
   <222> (1).. (366)
   <223>
<400> 91
   cag gtc cag ctg gtg caa tct ggg gct gag gtg aag aag cct ggg gcc 48
   Gln Val Gln Leu Val Gln Ser Gly Ala Glu Val Lys Lys Pro Gly Ala
   1 5 10 15
   tca gtg aag gtc tcc tgc aag gct tct gga tac acc ttc acc ggc tac 96
   Ser Val Lys Val Ser Cys Lys Ala Ser Gly Tyr Thr Phe Thr Gly Tyr
   20 25 30
   tat atg cac tgg gtg cga cag gcc cct gga caa ggg ctt gag tgg atg 144
   Tyr Met His Trp Val Arg Gln Ala Pro Gly Gln Gly Leu Glu Trp Met
   35 40 45
   gga tgg atc aac cct aac agt ggt ggc aca aag tat gca cag aag ttt 192
   Gly Trp Ile Asn Pro Asn Ser Gly Gly Thr Lys Tyr Ala Gln Lys Phe
   50 55 60
   cag ggc agg gtc acc atg acc agg gac acg tcc atc agc aca gcc tac 240
   Gln Gly Arg Val Thr Met Thr Arg Asp Thr Ser Ile Ser Thr Ala Tyr
   65 70 75 80
   atg gag ctg agc agg ctg aga tct gac gac acg gcc gtg tat tac tgt 288
   Met Glu Leu Ser Arg Leu Arg Ser Asp Asp Thr Ala Val Tyr Tyr Cys
   85 90 95
   gcg aga gga tac gat att ttg act ggt tat ggc tgg ttc gac ccc tgg 336
   Ala Arg Gly Tyr Asp Ile Leu Thr Gly Tyr Gly Trp Phe Asp Pro Trp
   100 105 110
   ggc cag gga acc ctg gtc acc gtc tcc tca 366
   Gly Gln Gly Thr Leu Val Thr Val Ser Ser
   115 120
<210> 92
   <211> 122
   <212> PRT
   <213> Homo sapiens
<400> 92
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Gly Tyr
   20 25 30
   Tyr Met His Trp Val Arg Gln Ala Pro Gly Gln Gly Leu Glu Trp Met
   35 40 45
   Gly Trp Ile Asn Pro Asn Ser Gly Gly Thr Lys Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Arg Ser Asp Asp Thr Ala Val Tyr Tyr Cys
   85 90 95
   Ala Arg Gly Tyr Asp Ile Leu Thr Gly Tyr Gly Trp Phe Asp Pro Trp
   100 105 110
   Gly Gln Gly Thr Leu Val Thr Val Ser Ser
   115 120
<210> 93
   <211> 360
   <212> DNA
   <213> Homo sapiens
<220>
   <221> CDS
   <222> (1).. (360)
   <223>
<400> 93
   cag gtc cag ctg gtg caa tct ggg gga ggc ttg gtc cag cct ggg ggg 48
   Gln Val Gln Leu Val Gln Ser Gly Gly Gly Leu Val Gln Pro Gly Gly
   1 5 10 15
   tcc ctg aga ctc tcc tgt gca gcc tct gga ttc acc ttt agt agc tat 96
   Ser Leu Arg Leu Ser Cys Ala Ala Ser Gly Phe Thr Phe Ser Ser Tyr
   20 25 30
   tgg atg agt tgg gtc cgc cag gct cca ggg aag ggg ctg gag tgg gtg 144
   Trp Met Ser Trp Val Arg Gln Ala Pro Gly Lys Gly Leu Glu Trp Val
   35 40 45
   gcc aac ata aag caa gat gga agt gag aaa tac tat gtg gac tct gtg 192
   Ala Asn Ile Lys Gln Asp Gly Ser Glu Lys Tyr Tyr Val Asp Ser Val
   50 55 60
   aag ggc cga ttc acc atc tcc aga gac aac gcc aag aac tca ctg tat 240
   Lys Gly Arg Phe Thr Ile Ser Arg Asp Asn Ala Lys Asn Ser Leu Tyr
   65 70 75 80
   ctg caa atg aac acc ctg aga gcc gag gac acg gct gtg tat tac tgt 288
   Leu Gln Met Asn Thr Leu Arg Ala Glu Asp Thr Ala Val Tyr Tyr Cys
   85 90 95
   gcg aga gat cgt ttg tgg acc cag ggg ttt ttt gac tac tgg ggc cag 336
   Ala Arg Asp Arg Leu Trp Thr Gln Gly Phe Phe Asp Tyr Trp Gly Gln
   100 105 110
   gga acc ctg gtc acc gtc tcc tca 360
   Gly Thr Leu Val Thr Val Ser Ser
   115 120
<210> 94
   <211> 120
   <212> PRT
   <213> Homo sapiens
<400> 94
   Gln Val Gln Leu Val Gln Ser Gly Gly Gly Leu Val Gln Pro Gly Gly
   1 5 10 15
   Ser Leu Arg Leu Ser Cys Ala Ala Ser Gly Phe Thr Phe Ser Ser Tyr
   20 25 30
   Trp Met Ser Trp Val Arg Gln Ala Pro Gly Lys Gly Leu Glu Trp Val
   35 40 45
   Ala Asn Ile Lys Gln Asp Gly Ser Glu Lys Tyr Tyr Val Asp Ser Val
   50 55 60
   Lys Gly Arg Phe Thr Ile Ser Arg Asp Asn Ala Lys Asn Ser Leu Tyr
   65 70 75 80
   Leu Gln Met Asn Thr Leu Arg Ala Glu Asp Thr Ala Val Tyr Tyr Cys
   85 90 95
   Ala Arg Asp Arg Leu Trp Thr Gln Gly Phe Phe Asp Tyr Trp Gly Gln
   100 105 110
   Gly Thr Leu Val Thr Val Ser Ser
   115 120
<210> 95
   <211> 339
   <212> DNA
   <213> Homo sapiens
<220>
   <221> CDS
   <222> (1).. (339)
   <223>
<400> 95
   gac atc gtg atg acc cag tct cca gac tcc ctg gct gtg tct ctg ggc 48
   Asp Ile Val Met Thr Gln Ser Pro Asp Ser Leu Ala Val Ser Leu Gly
   1 5 10 15
   gag agg gcc acc atc aac tgc aag tcc agc cag agt gtt tta tac agc 96
   Glu Arg Ala Thr Ile Asn Cys Lys Ser Ser Gln Ser Val Leu Tyr Ser
   20 25 30
   tcc aac aat aag aac tac tta gct tgg tac cag cag aaa cca gga cag 144
   Ser Asn Asn Lys Asn Tyr Leu Ala Trp Tyr Gln Gln Lys Pro Gly Gln
   35 40 45
   cct cct aac ctg ctc att tac tgg gca tct acc cgg gaa tcc ggg gtc 192
   Pro Pro Asn Leu Leu Ile Tyr Trp Ala Ser Thr Arg Glu Ser Gly Val
   50 55 60
   cct gac cga ttc agt ggc agc ggg tct ggg aca gat ttc act ctc acc 240
   Pro Asp Arg Phe Ser Gly Ser Gly Ser Gly Thr Asp Phe Thr Leu Thr
   65 70 75 80
   atc agc agc ctg cag gct gaa gat gtg gca gtt tat tac tgt cag caa 288
   Ile Ser Ser Leu Gln Ala Glu Asp Val Ala Val Tyr Tyr Cys Gln Gln
   85 90 95
   tat tat act act ccg tgg acg ttc ggc caa ggg acc aag gtg gaa atc 336
   Tyr Tyr Thr Thr Pro Trp Thr Phe Gly Gln Gly Thr Lys Val Glu Ile
   100 105 110
   aaa 339
   Lys
<210> 96
   <211> 113
   <212> PRT
   <213> Homo sapiens
<400> 96
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Asn Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Thr Thr Pro Trp Thr Phe Gly Gln Gly Thr Lys Val Glu Ile
               100                 105                 110
   Lys
<210> 97
   <211> 23
   <212> DNA
   <213> Artificial
<220>
   <223> Artificially Synthesized Primer Sequence
<400> 97
   caggtkcagc tggtgcagtc tgg 23
<210> 98
   <211> 23
   <212> DNA
   <213> Artificial
<220>
   <223> Artificially Synthesized Primer Sequence
<400> 98
   caggtccagc ttgtgcagtc tgg 23
<210> 99
   <211> 23
   <212> DNA
   <213> Artificial
<220>
   <223> Artificially Synthesized Primer Sequence
<400> 99
   saggtccagc tggtacagtc tgg 23
<210> 100
   <211> 23
   <212> DNA
   <213> Artificial
<220>
   <223> Artificially Synthesized Primer Sequence
<400> 100
   caratgcagc tggtgcagtc tgg 23
<210> 101
   <211> 24
   <212> DNA
   <213> Artificial
<220>
   <223> Artificially Synthesized Primer Sequence
<400> 101
   cagatcacct tgaaggagtc tggt 24
<210> 102
   <211> 24
   <212> DNA
   <213> Artificial
<220>
   <223> Artificially Synthesized Primer Sequence
<400> 102
   caggtcacct tgarggagtc tggt 24
<210> 103
   <211> 23
   <212> DNA
   <213> Artificial
<220>
   <223> Artificially Synthesized Primer Sequence
<400> 103
   gargtgcagc tggtggagtc tgg 23
<210> 104
   <211> 23
   <212> DNA
   <213> Artificial
<220>
   <223> Artificially Synthesized Primer Sequence
<400> 104
   caggtgcagc tggtggagtc tgg 23
<210> 105
   <211> 23
   <212> DNA
   <213> Artificial
<220>
   <223> Artificially Synthesized Primer Sequence
<400> 105
   gaggtgcagc tgttggagtc tgg 23
<210> 106
   <211> 24
   <212> DNA
   <213> Artificial
<220>
   <223> Artificially Synthesized Primer Sequence
<400> 106
   cagstgcagc tgcaggagtc gggc 24
<210> 107
   <211> 24
   <212> DNA
   <213> Artificial
<220>
   <223> Artificially Synthesized Primer Sequence
<400> 107
   caggtgcagc tacagcagtg gggc 24
<210> 108
   <211> 24
   <212> DNA
   <213> Artificial
<220>
   <223> Artificially Synthesized Primer Sequence
<400> 108
   gargtgcagc tggtgcagtc tgga 24
<210> 109
   <211> 24
   <212> DNA
   <213> Artificial
<220>
   <223> Artificially Synthesized Primer Sequence
<400> 109
   caggtacagc tgcagcagtc aggt 24
<210> 110
   <211> 23
   <212> DNA
   <213> Artificial
<220>
   <223> Artificially Synthesized Primer Sequence
<400> 110
   caggtscagc tggtgcaatc tgg 23
<210> 111
   <211> 24
   <212> DNA
   <213> Artificial
<220>
   <223> Artificially Synthesized Primer Sequence
<400> 111
   tgaggagacg gtgaccaggg tkcc 24
<210> 112
   <211> 24
   <212> DNA
   <213> Artificial
<220>
   <223> Artificially Synthesized Primer Sequence
<400> 112
   tgaagagacg gtgaccattg tccc 24
<210> 113
   <211> 24
   <212> DNA
   <213> Artificial
<220>
   <223> Artificially Synthesized Primer Sequence
<400> 113
   tgaggagacg gtgaccgtgg tccc 24
<210> 114
   <211> 24
   <212> DNA
   <213> Artificial
<220>
   <223> Artificially Synthesized Primer Sequence
<400> 114
   racatccaga tgacccagtc tcca 24
<210> 115
   <211> 24
   <212> DNA
   <213> Artificial
<220>
   <223> Artificially Synthesized Primer Sequence
<400> 115
   gmcatccagt tgacccagtc tcca 24
<210> 116
   <211> 24
   <212> DNA
   <213> Artificial
<220>
   <223> Artificially Synthesized Primer Sequence
<400> 116
   gccatccrga tgacccagtc tcca 24
<210> 117
   <211> 24
   <212> DNA
   <213> Artificial
<220>
   <223> Artificially Synthesized Primer Sequence
<400> 117
   gtcatctgga tgacccagtc tcca 24
<210> 118
   <211> 24
   <212> DNA
   <213> Artificial
<220>
   <223> Artificially Synthesized Primer Sequence
<400> 118
   gatattgtga tgacccagac tcca 24
<210> 119
   <211> 24
   <212> DNA
   <213> Artificial
<220>
   <223> Artificially Synthesized Primer Sequence
<400> 119
   gatrttgtga tgactcagtc tcca 24
<210> 120
   <211> 24
   <212> DNA
   <213> Artificial
<220>
   <223> Artificially Synthesized Primer Sequence
<400> 120
   gaaattgtgt tgacrcagtc tcca 24
<210> 121
   <211> 24
   <212> DNA
   <213> Artificial
<220>
   <223> Artificially Synthesized Primer Sequence
<400> 121
   gaaatagtga tgacgcagtc tcca 24
<210> 122
   <211> 24
   <212> DNA
   <213> Artificial
<220>
   <223> Artificially Synthesized Primer Sequence
<400> 122
   gaaattgtaa tgacacagtc tcca 24
<210> 123
   <211> 24
   <212> DNA
   <213> Artificial
<220>
   <223> Artificially Synthesized Primer Sequence
<400> 123
   gacatcgtga tgacccagtc tcca 24
<210> 124
   <211> 24
   <212> DNA
   <213> Artificial
<220>
   <223> Artificially Synthesized Primer Sequence
<400> 124
   gaaacgacac tcacgcagtc tcca 24
<210> 125
   <211> 24
   <212> DNA
   <213> Artificial
<220>
   <223> Artificially Synthesized Primer Sequence
<400> 125
   gaaattgtgc tgactcagtc tcca 24
<210> 126
   <211> 24
   <212> DNA
   <213> Artificial
<220>
   <223> Artificially Synthesized Primer Sequence
<400> 126
   gatgttgtga tgacacagtc tcca 24

<210> 127
   <211> 24
   <212> DNA
   <213> Artificial
<220>
   <223> Artificially Synthesized Primer Sequence
<400> 127
   acgtttgatt tccaccttgg tccc 24
<210> 128
   <211> 24
   <212> DNA
   <213> Artificial
<220>
   <223> Artificially Synthesized Primer Sequence
<400> 128
   acgtttgatc tccascttgg tccc 24
<210> 129
   <211> 24
   <212> DNA
   <213> Artificial
<220>
   <223> Artificially Synthesized Primer Sequence
<400> 129
   acgtttgata tccactttgg tccc 24
<210> 130
   <211> 24
   <212> DNA
   <213> Artificial
<220>
   <223> Artificially Synthesized Primer Sequence
<400> 130
   acgtttaatc tccagtcgtg tccc 24
<210> 131
   <211> 24
   <212> DNA
   <213> Artificial
<220>
   <223> Artificially Synthesized Primer Sequence
<400> 131
   cagtctgtgc tgactcagcc accc 24
<210> 132
   <211> 24
   <212> DNA
   <213> Artificial
<220>
   <223> Artificially Synthesized Primer Sequence
<400> 132
   cagtctgtgy tgacgcagcc gccc 24
<210> 133
   <211> 22
   <212> DNA
   <213> Artificial
<220>
   <223> Artificially Synthesized Primer Sequence
<400> 133
   cagtctgccc tgactcagcc ts 22
<210> 134
   <211> 24
   <212> DNA
   <213> Artificial
<220>
   <223> Artificially Synthesized Primer Sequence
<400> 134
   tcctatgwgc tgactcagcc accc 24
<210> 135
   <211> 24
   <212> DNA
   <213> Artificial
<220>
   <223> Artificially Synthesized Primer Sequence
<400> 135
   tcctatgagc tgacacagcy accc 24
<210> 136
   <211> 24
   <212> DNA
   <213> Artificial
<220>
   <223> Artificially Synthesized Primer Sequence
<400> 136
   tcttctgagc tgactcagga ccct 24
<210> 137
   <211> 24
   <212> DNA
   <213> Artificial
<220>
   <223> Artificially Synthesized Primer Sequence
<400> 137
   tcctatgagc tgatgcagcc accc 24
<210> 138
   <211> 24
   <212> DNA
   <213> Artificial
<220>
   <223> Artificially Synthesized Primer Sequence
<400> 138
   cagcctgtgc tgactcaatc atcc 24
<210> 139
   <211> 24
   <212> DNA
   <213> Artificial
<220>
   <223> Artificially Synthesized Primer Sequence
<400> 139
   cagcttgtgc tgactcaatc gccc 24
<210> 140
   <211> 24
   <212> DNA
   <213> Artificial
<220>
   <223> Artificially Synthesized Primer Sequence
<400> 140
   ctgcctgtgc tgactcagcc cccg 24
<210> 141
   <211> 24
   <212> DNA
   <213> Artificial
<220>
   <223> Artificially Synthesized Primer Sequence
<400> 141
   cagcctgtgc tgactcagcc ayct 24
<210> 142
   <211> 24
   <212> DNA
   <213> Artificial
<220>
   <223> Artificially Synthesized Primer Sequence
<400> 142
   caggctgtgc tgactcagcc ggct 24
<210> 143
   <211> 24
   <212> DNA
   <213> Artificial
<220>
   <223> Artificially Synthesized Primer Sequence
<400> 143
   aattttatgc tgactcagcc ccac 24
<210> 144
   <211> 24
   <212> DNA
   <213> Artificial
<220>
   <223> Artificially Synthesized Primer Sequence
<400> 144
   cagrctgtgg tgactcagga gccc 24
<210> 145
   <211> 24
   <212> DNA
   <213> Artificial
<220>
   <223> Artificially Synthesized Primer Sequence
<400> 145
   cagactgtgg tgacccagga gcca 24
<210> 146
   <211> 24
   <212> DNA
   <213> Artificial
<220>
   <223> Artificially Synthesized Primer Sequence
<400> 146
   cwgcctgtgc tgactcagcc acct 24
<210> 147
   <211> 24
   <212> DNA
   <213> Artificial
<220>
   <223> Artificially Synthesized Primer Sequence
<400> 147
   caggcagggc tgactcagcc accc 24
<210> 148
   <211> 24
   <212> DNA
   <213> Artificial
<220>
   <223> Artificially Synthesized Primer Sequence
<400> 148
   acctaggacg gtgaccttgg tccc 24
<210> 149
   <211> 24
   <212> DNA
   <213> Artificial
<220>
   <223> Artificially Synthesized Primer Sequence
<400> 149
   acctaggacg gtcagcttgg tccc 24
<210> 150
   <211> 24
   <212> DNA
   <213> Artificial
<220>
   <223> Artificially Synthesized Primer Sequence
<400> 150
   accgaggacg gtcagctggg tgcc 24
<210> 151
   <211> 91
   <212> DNA
   <213> Artificial
<220>
   <223> Template Linker Sequence
<400> 151
   ggacaatggt caccgtctct tcaggtggtg gtggttcggg tggtggtggt tcgggtggtg 60
   gcggatcgga catccagatg acccagtctc c 91
<210> 152
   <211> 28
   <212> DNA
   <213> Artificial
<220>
   <223> Artificially Synthesized Primer Sequence
<400> 152
   gcaccctggt caccgtctcc tcaggtgg 28
<210> 153
   <211> 28
   <212> DNA
   <213> Artificial
<220>
   <223> Artificially Synthesized Primer Sequence
<400> 153
   ggacaatggt caccgtctct tcaggtgg 28
<210> 154
   <211> 28
   <212> DNA
<213> Artificial
<220>
   <223> Artificially Synthesized Primer Sequence
<400> 154
   gaaccctggt caccgtctcc tcaggtgg 28
<210> 155
   <211> 28
   <212> DNA
   <213> Artificial
<220>
   <223> Artificially Synthesized Primer Sequence
<400> 155
   ggaccacggt caccgtctcc tcaggtgg 28
<210> 156
   <211> 32
   <212> DNA
   <213> Artificial
<220>
   <223> Artificially Synthesized Primer Sequence
<400> 156
   ggagactggg tcatctggat gtccgatccg cc 32
<210> 157
   <211> 32
   <212> DNA
<213> Artificial
<220>
   <223> Artificially Synthesized Primer Sequence
<400> 157
   ggagactgag tcatcacaac atccgatccg cc 32
<210> 158
   <211> 32
   <212> DNA
   <213> Artificial
<220>
   <223> Artificially Synthesized Primer Sequence
<400> 158
   ggagactgcg tcaacacaat ttccgatccg cc 32
<210> 159
   <211> 32
   <212> DNA
   <213> Artificial
<220>
   <223> Artificially Synthesized Primer Sequence
<400> 159
   ggagactggg tcatcacgat gtccgatccg cc 32
<210> 160
   <211> 32
   <212> DNA
   <213> Artificial
<220>
   <223> Artificially Synthesized Primer Sequence
<400> 160
   ggagactgcg tgagtgtcgt ttccgatccg cc 32
<210> 161
   <211> 32
   <212> DNA
   <213> Artificial
<220>
   <223> Artificially Synthesized Primer Sequence
<400> 161
   ggagactgag tcagcacaat ttccgatccg cc 32
<210> 162
   <211> 42
   <212> DNA
   <213> Artificial
<220>
   <223> Artificially Synthesized Primer Sequence
<400> 162
   ggcggctgcg tcaacacaga ctgcgatccg ccaccgccag ag 42
<210> 163
   <211> 42
   <212> DNA
   <213> Artificial
<220>
   <223> Artificially Synthesized Primer Sequence
<400> 163
   gcaggctgag tcagagcaga ctgcgatccg ccaccgccag ag 42
<210> 164
   <211> 42
   <212> DNA
   <213> Artificial
<220>
   <223> Artificially Synthesized Primer Sequence
<400> 164
   ggtggctgag tcagcacata ggacgatccg ccaccgccag ag 42
<210> 165
   <211> 42
   <212> DNA
   <213> Artificial
<220>
   <223> Artificially Synthesized Primer Sequence
<400> 165
   gggtcctgag tcagctcaga agacgatccg ccaccgccag ag 42
<210> 166
   <211> 42
   <212> DNA
   <213> Artificial
<220>
   <223> Artificially Synthesized Primer Sequence
<400> 166
   ggcggttgag tcagtataac gtgcgatccg ccaccgccag ag 42
<210> 167
   <211> 42
   <212> DNA
   <213> Artificial
<220>
   <223> Artificially Synthesized Primer Sequence
<400> 167
   gacggctgag tcagcacaga ctgcgatccg ccaccgccag ag 42
<210> 168
   <211> 42
   <212> DNA
   <213> Artificial
<220>
   <223> Artificially Synthesized Primer Sequence
<400> 168
   tggggctgag tcagcataaa attcgatccg ccaccgccag ag 42
<210> 169
   <211> 39
   <212> DNA
   <213> Artificial
<220>
   <223> Artificially Synthesized Primer Sequence
<400> 169
   agtattgacc atggcccagg tgcagctggt gcagtctgg 39
<210> 170
   <211> 39
   <212> DNA
   <213> Artificial
<220>
   <223> Artificially Synthesized Primer Sequence
<400> 170
   agtattgacc atggcccagg tcaacttaag ggagtctgg 39
<210> 171
   <211> 39
   <212> DNA
   <213> Artificial
<220>
   <223> Artificially Synthesized Primer Sequence
<400> 171
   agtattgacc atggccgagg tgcagctggt ggagtctgg 39
<210> 172
   <211> 39
   <212> DNA
   <213> Artificial
<220>
   <223> Artificially Synthesized Primer Sequence
<400> 172
   agtattgacc atggcccagg tgcagctgca ggagtcggg 39
<210> 173
   <211> 39
   <212> DNA
   <213> Artificial
<220>
   <223> Artificially Synthesized Primer Sequence
<400> 173
   agtattgacc atggcccagg tgcagctgtt gcagtctgc 39
<210> 174
   <211> 39
   <212> DNA
   <213> Artificial
<220>
   <223> Artificially Synthesized Primer Sequence
<400> 174
   agtattgacc atggcccagg tacagctgca gcagtcagg 39
<210> 175
   <211> 34
   <212> DNA
   <213> Artificial
<220>
   <223> Artificially Synthesized Primer Sequence
<400> 175
   taatgaattc acgtttgatt tccaccttgg tccc 34
<210> 176
   <211> 34
   <212> DNA
   <213> Artificial
<220>
   <223> Artificially Synthesized Primer Sequence
<400> 176
   taatgaattc acgtttgatc tccagcttgg tccc 34
<210> 177
   <211> 34
   <212> DNA
   <213> Artificial
<220>
   <223> Artificially Synthesized Primer Sequence
<400> 177
   taatgaattc acgtttgata tccactttgg tccc 34
<210> 178
   <211> 34
   <212> DNA
   <213> Artificial
<220>
   <223> Artificially Synthesized Primer Sequence
<400> 178
   taatgaattc acgtttgatc tccaccttgg tccc 34
<210> 179
   <211> 34
   <212> DNA
   <213> Artificial
<220>
   <223> Artificially Synthesized Primer Sequence
<400> 179
   taatgaattc acgtttaatc tccagtcgtg tccc 34
<210> 180
   <211> 34
   <212> DNA
   <213> Artificial
<220>
   <223> Artificially Synthesized Primer Sequence
<400> 180
   taatgaattc acctaggacg gtgaccttgg tccc 34
<210> 181
   <211> 34
   <212> DNA
   <213> Artificial
<220>
   <223> Artificially Synthesized Primer Sequence
<400> 181
   taatgaattc acctaggacg gtcagcttgg tccc 34
<210> 182
   <211> 34
   <212> DNA
   <213> Artificial
<220>
   <223> Artificially Synthesized Primer Sequence
<400> 182
   taatgaattc acctaaaacg gtgagctggg tccc 34
<210> 183
   <211> 861
   <212> DNA
   <213> Homo sapiens
<220>
   <221> CDS
   <222> (1)..(861)
   <223>
<400> 183
   atg aaa tac ctg ctg ccg acc gct gct gct ggt ctg ctg ctc ctc gct      48
   Met Lys Tyr Leu Leu Pro Thr Ala Ala Ala Gly Leu Leu Leu Leu Ala
   1               5                   10                  15
   gcc cag ccg gcg atg gcc atg gcc cag gtg cag ctg gtg cag tct ggg 96
   Ala Gln Pro Ala Met Ala Met Ala Gln Val Gln Leu Val Gln Ser Gly
   20 25 30
   gct gag gtg aag aag cct ggg gcc tca gtg aag gtc tcc tgc aag gct 144
   Ala Glu Val Lys Lys Pro Gly Ala Ser Val Lys Val Ser Cys Lys Ala
   35 40 45
   tct gga tac acc ttc acc ggc tac tat atg cac tgg gtg cga cag gcc 192
   Ser Gly Tyr Thr Phe Thr Gly Tyr Tyr Met His Trp Val Arg Gln Ala
   50 55 60
   cct gga caa ggg ctt gag tgg atg gga tgg atc aac cct aac agt ggt 240
   Pro Gly Gln Gly Leu Glu Trp Met Gly Trp Ile Asn Pro Asn Ser Gly
   65 70 75 80
   ggc aca aag tat gca cag aag ttt cag ggc agg gtc acc atg acc agg 288
   Gly Thr Lys Tyr Ala Gln Lys Phe Gln Gly Arg Val Thr Met Thr Arg
   85 90 95
   gac acg tcc atc agc aca gcc tac atg gag ctg agc agg ctg aga tct 336
   Asp Thr Ser Ile Ser Thr Ala Tyr Met Glu Leu Ser Arg Leu Arg Ser
   100 105 110
   gac gac acg gcc gtg tat tac tgt gcg aga gga tac gat att ttg act 384
   Asp Asp Thr Ala Val Tyr Tyr Cys Ala Arg Gly Tyr Asp Ile Leu Thr
   115 120 125
   ggt tat ggc tgg ttc gac ccc tgg ggc cag gga acc ctg gtc acc gtc 432
   Gly Tyr Gly Trp Phe Asp Pro Trp Gly Gln Gly Thr Leu Val Thr Val
   130 135 140
   tcc tca ggt ggt ggt ggt tcg ggt ggt ggt ggt tcg ggt ggt ggc gga 480
   Ser Ser Gly Gly Gly Gly Ser Gly Gly Gly Gly Ser Gly Gly Gly Gly
   145 150 155 160
   tcg gac atc gtg atg acc cag tct cca gac tcc ctg gct gtg tct ctg      528
   Ser Asp Ile Val Met Thr Gln Ser Pro Asp Ser Leu Ala Val Ser Leu
                   165                 170                 175
   ggc gag agg gcc acc atc aac tgc aag tcc agc cag agt gtt tta tac      576
   Gly Glu Arg Ala Thr Ile Asn Cys Lys Ser Ser Gln Ser Val Leu Tyr
               180                 185                 190
   agc tcc aac aat aag aac tac tta gct tgg tac cag cag aaa cca gga      624
   Ser Ser Asn Asn Lys Asn Tyr Leu Ala Trp Tyr Gln Gln Lys Pro Gly
           195                 200                 205
   cag cct cct aaa ctg ctc att tac tgg gca tct acc cgg gaa tcc ggg      672
   Gln Pro Pro Lys Leu Leu Ile Tyr Trp Ala Ser Thr Arg Glu Ser Gly
       210                 215                 220
   gtc cct gac cga ttc agt ggc agc ggg tct ggg aca gat ttc act ctc      720
   Val Pro Asp Arg Phe Ser Gly Ser Gly Ser Gly Thr Asp Phe Thr Leu
   225                 230                 235                 240
   acc atc agc acc ctg cag gct gaa gat gtg gca gtt tat tac tgt cag      768
   Thr Ile Ser Thr Leu Gln Ala Glu Asp Val Ala Val Tyr Tyr Cys Gln
                   245                 250                 255
   caa tat tat agt act cct ccg acg ttc ggc caa ggg acc aag gtg gaa      816
   Gln Tyr Tyr Ser Thr Pro Pro Thr Phe Gly Gln Gly Thr Lys Val Glu
               260                 265                 270
   atc aaa cgt cgt gaa ttc gac tac aag gat gac gac gat aag tga          861
   Ile Lys Arg Arg Glu Phe Asp Tyr Lys Asp Asp Asp Asp Lys
           275                 280                 285

<210> 184
   <211> 286
   <212> PRT
   <213> Homo sapiens
<400> 184
   Met Lys Tyr Leu Leu Pro Thr Ala Ala Ala Gly Leu Leu Leu Leu Ala
   1 5 10 15
   Ala Gln Pro Ala Met Ala Met Ala Gln Val Gln Leu Val Gln Ser Gly
   20 25 30
   Ala Glu Val Lys Lys Pro Gly Ala Ser Val Lys Val Ser Cys Lys Ala
   35 40 45
   Ser Gly Tyr Thr Phe Thr Gly Tyr Tyr Met His Trp Val Arg Gln Ala
   50 55 60
   Pro Gly Gln Gly Leu Glu Trp Met Gly Trp Ile Asn Pro Asn Ser Gly
   65 70 75 80
   Gly Thr Lys Tyr Ala Gln Lys Phe Gln Gly Arg Val Thr Met Thr Arg
   85 90 95
   Asp Thr Ser Ile Ser Thr Ala Tyr Met Glu Leu Ser Arg Leu Arg Ser
   100 105 110
   Asp Asp Thr Ala Val Tyr Tyr Cys Ala Arg Gly Tyr Asp Ile Leu Thr
   115 120 125
   Gly Tyr Gly Trp Phe Asp Pro Trp Gly Gln Gly Thr Leu Val Thr Val
   130 135 140
   Ser Ser Gly Gly Gly Gly Ser Gly Gly Gly Gly Ser Gly Gly Gly Gly
   145 150 155 160
   Ser Asp Ile Val Met Thr Gln Ser Pro Asp Ser Leu Ala Val Ser Leu
   165 170 175
   Gly Glu Arg Ala Thr Ile Asn Cys Lys Ser Ser Gln Ser Val Leu Tyr
   180 185 190
   Ser Ser Asn Asn Lys Asn Tyr Leu Ala Trp Tyr Gln Gln Lys Pro Gly
   195 200 205
   Gln Pro Pro Lys Leu Leu Ile Tyr Trp Ala Ser Thr Arg Glu Ser Gly
   210 215 220
   Val Pro Asp Arg Phe Ser Gly Ser Gly Ser Gly Thr Asp Phe Thr Leu
   225 230 235 240
   Thr Ile Ser Thr Leu Gln Ala Glu Asp Val Ala Val Tyr Tyr Cys Gln
   245 250 255
   Gln Tyr Tyr Ser Thr Pro Pro Thr Phe Gly Gln Gly Thr Lys Val Glu
   260 265 270
   Ile Lys Arg Arg Glu Phe Asp Tyr Lys Asp Asp Asp Asp Lys
   275 280 285
<210> 185
   <211> 846
   <212> DNA
   <213> Homo sapiens
<220>
   <221> CDS
   <222> (1)..(846)
   <223>
<400> 185
   atg aaa tac ctg ctg ccg acc gct gct gct ggt ctg ctg ctc ctc gct 48
   Met Lys Tyr Leu Leu Pro Thr Ala Ala Ala Gly Leu Leu Leu Leu Ala
   1 5 10 15
   gcc cag ccg gcg atg gcc atg gcc cag gtg cag ctg gtg cag tct ggg 96
   Ala Gln Pro Ala Met Ala Met Ala Gln Val Gln Leu Val Gln Ser Gly
   20 25 30
   gct gag gtg aag aag cct ggg gcc tca gtg aag gtc tcc tgc aag gct 144
   Ala Glu Val Lys Lys Pro Gly Ala Ser Val Lys Val Ser Cys Lys Ala
   35 40 45
   tct gga tac acc ttc acc ggc tac tat atg cac tgg gtg cga cag gcc 192
   Ser Gly Tyr Thr Phe Thr Gly Tyr Tyr Met His Trp Val Arg Gln Ala
   50 55 60
   cct gga caa ggg ctt gag tgg atg gga tgg atc aac cct aac agt ggt 240
   Pro Gly Gln Gly Leu Glu Trp Met Gly Trp Ile Asn Pro Asn Ser Gly
   65 70 75 80
   ggc aca aag tat gca cag aag ttt cag ggc agg gtc acc atg acc agg 288
   Gly Thr Lys Tyr Ala Gln Lys Phe Gln Gly Arg Val Thr Met Thr Arg
   85 90 95
   gac acg tcc atc agc aca gcc tac atg gag ctg agc agg ctg aga tct 336
   Asp Thr Ser Ile Ser Thr Ala Tyr Met Glu Leu Ser Arg Leu Arg Ser
   100 105 110
   gac gac acg gcc gtg tat tac tgt gcg aga gga tac gat att ttg act 384
   Asp Asp Thr Ala Val Tyr Tyr Cys Ala Arg Gly Tyr Asp Ile Leu Thr
   115 120 125
   ggt tat ggc tgg ttc gac ccc tgg ggc cag gga acc ctg gtc acc gtc 432
   Gly Tyr Gly Trp Phe Asp Pro Trp Gly Gln Gly Thr Leu Val Thr Val
   130 135 140
   tcc tca ggt ggt ggt ggt tcg ggt ggt ggt ggt tcg ggt ggt ggc gga 480
   Ser Ser Gly Gly Gly Gly Ser Gly Gly Gly Gly Ser Gly Gly Gly Gly
   145 150 155 160
   tcg gaa att gtg ctg act cag tct cca ggc acc ctg tct ttg tct cca 528
   Ser Glu Ile Val Leu Thr Gln Ser Pro Gly Thr Leu Ser Leu Ser Pro
   165 170 175
   ggg gaa aga gcc acc ctc tcc tgc aag gcc agt cag agt ttt agc agc 576
   Gly Glu Arg Ala Thr Leu Ser Cys Lys Ala Ser Gln Ser Phe Ser Ser
   180 185 190
   aac tac tta gcc tgg tac cag cag aaa cct ggc cag gct ccc agg ctg 624
   Asn Tyr Leu Ala Trp Tyr Gln Gln Lys Pro Gly Gln Ala Pro Arg Leu
   195 200 205
   ctc atc tat ggt gca tcc agc agg gcc act ggc atc cca gac agg ttc 672
   Leu Ile Tyr Gly Ala Ser Ser Arg Ala Thr Gly Ile Pro Asp Arg Phe
   210 215 220
   agt ggc agt aaa tct ggg aca gac ttc act ctc acc atc agc aga ctg 720
   Ser Gly Ser Lys Ser Gly Thr Asp Phe Thr Leu Thr Ile Ser Arg Leu
   225 230 235 240
   gag cct gaa gat ttt gca gtg tat tac tgt cag cag tat gtt acc tca 768
   Glu Pro Glu Asp Phe Ala Val Tyr Tyr Cys Gln Gln Tyr Val Thr Ser
   245 250 255
   ccg tac act ttt ggc cag ggg acc aag gtg gag atc aaa cgt cgt gaa 816
   Pro Tyr Thr Phe Gly Gln Gly Thr Lys Val Glu Ile Lys Arg Arg Glu
   260 265 270
   ttc gac tac aag gat gac gac gat aag tga 846
   Phe Asp Tyr Lys Asp Asp Asp Asp Lys
   275 280
<210> 186
   <211> 281
   <212> PRT
   <213> Homo sapiens
<400> 186
   Met Lys Tyr Leu Leu Pro Thr Ala Ala Ala Gly Leu Leu Leu Leu Ala
   1 5 10 15
   Ala Gln Pro Ala Met Ala Met Ala Gln Val Gln Leu Val Gln Ser Gly
   20 25 30
   Ala Glu Val Lys Lys Pro Gly Ala Ser Val Lys Val Ser Cys Lys Ala
   35 40 45
   Ser Gly Tyr Thr Phe Thr Gly Tyr Tyr Met His Trp Val Arg Gln Ala
   50 55 60
   Pro Gly Gln Gly Leu Glu Trp Met Gly Trp Ile Asn Pro Asn Ser Gly
   65 70 75 80
   Gly Thr Lys Tyr Ala Gln Lys Phe Gln Gly Arg Val Thr Met Thr Arg
   85 90 95
   Asp Thr Ser Ile Ser Thr Ala Tyr Met Glu Leu Ser Arg Leu Arg Ser
   100 105 110
   Asp Asp Thr Ala Val Tyr Tyr Cys Ala Arg Gly Tyr Asp Ile Leu Thr
   115 120 125
   Gly Tyr Gly Trp Phe Asp Pro Trp Gly Gln Gly Thr Leu Val Thr Val
   130 135 140
   Ser Ser Gly Gly Gly Gly Ser Gly Gly Gly Gly Ser Gly Gly Gly Gly
   145 150 155 160
   Ser Glu Ile Val Leu Thr Gln Ser Pro Gly Thr Leu Ser Leu Ser Pro
   165 170 175
   Gly Glu Arg Ala Thr Leu Ser Cys Lys Ala Ser Gln Ser Phe Ser Ser
   180 185 190
   Asn Tyr Leu Ala Trp Tyr Gln Gln Lys Pro Gly Gln Ala Pro Arg Leu
   195 200 205
   Leu Ile Tyr Gly Ala Ser Ser Arg Ala Thr Gly Ile Pro Asp Arg Phe
   210 215 220
   Ser Gly Ser Lys Ser Gly Thr Asp Phe Thr Leu Thr Ile Ser Arg Leu
   225 230 235 240
   Glu Pro Glu Asp Phe Ala Val Tyr Tyr Cys Gln Gln Tyr Val Thr Ser
   245 250 255
   Pro Tyr Thr Phe Gly Gln Gly Thr Lys Val Glu Ile Lys Arg Arg Glu
   260 265 270
   Phe Asp Tyr Lys Asp Asp Asp Asp Lys
   275 280
<210> 187
   <211> 852
   <212> DNA
   <213> Homo sapiens
<220>
   <221> CDS
   <222> (1)..(852)
   <223>
<400> 187
   atg aaa tac ctg ctg ccg acc gct gct gct ggt ctg ctg ctc ctc gct 48
   Met Lys Tyr Leu Leu Pro Thr Ala Ala Ala Gly Leu Leu Leu Leu Ala
   1              5                   10                  15
   gcc cag ccg gcg atg gcc atg gcc cag gtc cag ctg gtg caa tct ggg         96
   Ala Gln Pro Ala Met Ala Met Ala Gln Val Gln Leu Val Gln Ser Gly
               20                  25                  30
   gga ggc ttg gtc cag cct ggg ggg tcc ctg aga ctc tcc tgt gca gcc        144
   Gly Gly Leu Val Gln Pro Gly Gly Ser Leu Arg Leu Ser Cys Ala Ala
           35                  40                  45
   tct gga ttc acc ttt agt agc tat tgg atg agt tgg gtc cgc cag gct        192
   Ser Gly Phe Thr Phe Ser Ser Tyr Trp Met Ser Trp Val Arg Gln Ala
       50                  55                  60
   cca ggg aag ggg ctg gag tgg gtg gcc aac ata aag caa gat gga agt        240
   Pro Gly Lys Gly Leu Glu Trp Val Ala Asn Ile Lys Gln Asp Gly Ser
   65                  70                  75                  80
   gag aaa tac tat gtg gac tct gtg aag ggc cga ttc acc atc tcc aga        288
   Glu Lys Tyr Tyr Val Asp Ser Val Lys Gly Arg Phe Thr Ile Ser Arg
                   85                  90                  95
   gac aac gcc aag aac tca ctg tat ctg caa atg aac acc ctg aga gcc        336
   Asp Asn Ala Lys Asn Ser Leu Tyr Leu Gln Met Asn Thr Leu Arg Ala
               100                 105                 110
   gag gac acg gct gtg tat tac tgt gcg aga gat cgt ttg tgg acc cag        384
   Glu Asp Thr Ala Val Tyr Tyr Cys Ala Arg Asp Arg Leu Trp Thr Gln
           115                 120                 125
   ggg ttt ttt gac tac tgg ggc cag gga acc ctg gtc acc gtc tcc tca        432
   Gly Phe Phe Asp Tyr Trp Gly Gln Gly Thr Leu Val Thr Val Ser Ser
       130                 135                 140
   ggt ggt ggt ggt tcg ggt ggt ggt ggt tcg ggt ggt ggc gga tcg gac        480
   Gly Gly Gly Gly Ser Gly Gly Gly Gly Ser Gly Gly Gly Gly Ser Asp
   145 150 155 160
   atc gtg atg acc cag tct cca gac tcc ctg gct gtg tct ctg ggc gag 528
   Ile Val Met Thr Gln Ser Pro Asp Ser Leu Ala Val Ser Leu Gly Glu
   165 170 175
   agg gcc acc atc aac tgc aag tcc agc cag agt gtt tta tac agc tcc 576
   Arg Ala Thr Ile Asn Cys Lys Ser Ser Gln Ser Val Leu Tyr Ser Ser
   180 185 190
   aac aat aag aac tac tta gct tgg tac cag cag aaa cca gga cag cct 624
   Asn Asn Lys Asn Tyr Leu Ala Trp Tyr Gln Gln Lys Pro Gly Gln Pro
   195 200 205
   cct aac ctg ctc att tac tgg gca tct acc cgg gaa tcc ggg gtc cct 672
   Pro Asn Leu Leu Ile Tyr Trp Ala Ser Thr Arg Glu Ser Gly Val Pro
   210 215 220
   gac cga ttc agt ggc agc ggg tct ggg aca gat ttc act ctc acc atc 720
   Asp Arg Phe Ser Gly Ser Gly Ser Gly Thr Asp Phe Thr Leu Thr Ile
   225 230 235 240
   agc agc ctg cag gct gaa gat gtg gca gtt tat tac tgt cag caa tat 768
   Ser Ser Leu Gln Ala Glu Asp Val Ala Val Tyr Tyr Cys Gln Gln Tyr
   245 250 255
   tat act act ccg tgg acg ttc ggc caa ggg acc aag gtg gaa atc aaa 816
   Tyr Thr Thr Pro Trp Thr Phe Gly Gln Gly Thr Lys Val Glu Ile Lys
   260 265 270
   cgt gaa ttc gac tac aag gat gac gac gat aag tga 852
   Arg Glu Phe Asp Tyr Lys Asp Asp Asp Asp Lys
   275 280
<210> 188
   <211> 283
   <212> PRT
   <213> Homo sapiens
<400> 188
   Met Lys Tyr Leu Leu Pro Thr Ala Ala Ala Gly Leu Leu Leu Leu Ala
   1 5 10 15
   Ala Gln Pro Ala Met Ala Met Ala Gln Val Gln Leu Val Gln Ser Gly
   20 25 30
   Gly Gly Leu Val Gln Pro Gly Gly Ser Leu Arg Leu Ser Cys Ala Ala
   35 40 45
   Ser Gly Phe Thr Phe Ser Ser Tyr Trp Met Ser Trp Val Arg Gln Ala
   50 55 60
   Pro Gly Lys Gly Leu Glu Trp Val Ala Asn Ile Lys Gln Asp Gly Ser
   65 70 75 80
   Glu Lys Tyr Tyr Val Asp Ser Val Lys Gly Arg Phe Thr Ile Ser Arg
   85 90 95
   Asp Asn Ala Lys Asn Ser Leu Tyr Leu Gln Met Asn Thr Leu Arg Ala
   100 105 110
   Glu Asp Thr Ala Val Tyr Tyr Cys Ala Arg Asp Arg Leu Trp Thr Gln
   115 120 125
   Gly Phe Phe Asp Tyr Trp Gly Gln Gly Thr Leu Val Thr Val Ser Ser
   130 135 140
   Gly Gly Gly Gly Ser Gly Gly Gly Gly Ser Gly Gly Gly Gly Ser Asp
   145 150 155 160
   Ile Val Met Thr Gln Ser Pro Asp Ser Leu Ala Val Ser Leu Gly Glu
   165 170 175
   Arg Ala Thr Ile Asn Cys Lys Ser Ser Gln Ser Val Leu Tyr Ser Ser
   180 185 190
   Asn Asn Lys Asn Tyr Leu Ala Trp Tyr Gln Gln Lys Pro Gly Gln Pro
   195 200 205
   Pro Asn Leu Leu Ile Tyr Trp Ala Ser Thr Arg Glu Ser Gly Val Pro
   210 215 220
   Asp Arg Phe Ser Gly Ser Gly Ser Gly Thr Asp Phe Thr Leu Thr Ile
   225 230 235 240
   Ser Ser Leu Gln Ala Glu Asp Val Ala Val Tyr Tyr Cys Gln Gln Tyr
   245 250 255
   Tyr Thr Thr Pro Trp Thr Phe Gly Gln Gly Thr Lys Val Glu Ile Lys
   260 265 270
   Arg Glu Phe Asp Tyr Lys Asp Asp Asp Asp Lys
   275 280

## Description

### Technical Field

The present invention relates to methods for isolating polynucleotides encoding antibodies against lesional tissues and methods for producing such antibodies.

### Background Art

Lymphocytes are widely known to infiltrate cancer tissues ( Hurliamnn et al. (1985) Int J Cancer 35:753; Whiteside et al. (1986) Cancer Immunol Immunother 23:169; Wolf et al. (1986) Otolaryngol Head Neck Surg 95:142; Husby et al. (1976) J Clin Invest 57:1471; Vose et al. (1979) Int J Cancer 24:579). Experimental and clinical data suggest that lymphocyte infiltration of cancer tissues is associated with host immunoreactions against cancer (Rosenberg et al. (1988) New Engl J Med 319:1676; Van Pel et al. (1995) Immunol Reviews 145:229; Kreider et al. (1984) Cancer Metastasis Rev 3:53).

In the host immune defense system against cancer, cytotoxic T cells (CTLs) are the effector cells that directly kill cancer cells (Nobholz and MacDonald (1983) Annu Rev Immunol 1:273). Furthermore, some of the antibodies produced by plasma cells, which are the final form of differentiated B cells, are assumed to have the ability to bind to cancer cells (Roitt et al. (1969) Lancet 2: 367; Borsos (1971) Progress in Immunology: p841. New York, Academic Press; Kodera and Bean (1975) Int J Cancer 16:579).

For example, it has been shown that B cells which infiltrate cancer tissues express antibodies, and that these antibodies bind selectively to antigens on cancer cells ( Punt et al. (1994) Cancer Immunol Immunother 38:225; Zhang et al. (1995) Cancer Res 55:3584). This suggests that cancer antigens can be identified by using antibodies expressed in infiltrating B cells. Antibodies having such reactivity will be useful for cancer treatment and diagnosis if they can be obtained.

Antibodies that bind to cancer cells destroy cancer cells by activating the complement system or by triggering antibody-dependent cytotoxicity. However, in reality, there have been few reports on the specificity and the repertoire of variable regions in antibodies produced by cancer tissue-infiltrating B cells.

One of the reasons why it is difficult to analyze antibodies expressed in cancer tissue-infiltrating B cells is the difficulty of isolating antibodies that are produced by infiltrating B cells. Antibody analysis generally requires the cloning of antibody-producing cells. As a technique for cloning antibody-producing cells, a method that immortalizes human B cells using Epstein-Barr viruses (EBVs) is known. However, the probability of establishing a human antibody-producing cell line through EBV infection is extremely low (Henderson et al. (1977) Virology 76:152; Aman et al. (1984) J Exp Med 159:208).

The hybridoma method devised for establishing murine antibody-producing cell lines is another technique for cloning antibody-producing cells. The hybridoma method comprises the step of fusing an antigen-specific B cell with an immortalized myeloma cell to establish an antibody-expressing cell line. However, no partner cells that efficiently fuse with human B cells have been found so far. Murine myeloma cells are effective in the case of murine hybridomas; however, when used as a fusion partner for human B cells, characteristics of human hybridomas become unstable due to the preferential occurrence of deletions in human chromosomes, thus failing to lead to the establishment of antibody-producing cell lines (Winter and Milstein (1991) Nature 349:293). At present, it is thus technically difficult to establish human-derived antibody-producing cell lines.

Recombinant DNA techniques for producing antibodies with similar antigen-binding activity as the cloned antibody-producing cells, that is, methods for cloning antibody genes and preparing recombinant antibody proteins, have been established (Marks et al. (1991) J Mol Biol 222:581; Larrick et al. (1992) Immunol Reviews 130:69). Antibody genes encoding, for example, Fv, scFv, Fab, IgG, or IgM, can be generated by cloning genes that encode antibody variable regions (Skerra et al. (1988) Science 240:1038-1041; Bird et al. (1988) Science 242:423; Better et al. (1988) Science 240:1041). The smallest recombinant antibody molecule, scFv, has a structure in which the heavy chain variable region arid the light chain variable region are connected through a linker.

Needless to say, cloned B cells express a single antibody gene. Accordingly, antibodies having an activity similar to that of antibodies produced by B cells can be reconstructed by cloning the heavy and light chain variable regions from these cells. However, B cells present in peripheral blood and those infiltrating cancer tissues are (polyclonal) cell populations which produce various antibodies (Kotlan et al. (1999) Immunol Lett 65:143; Hansen et al. (2001) Pro Natl Acad Sci USA 98:12659). Therefore, it would not be easy to clone antibody genes from such cell populations and reconstruct them as human antibodies.

### Disclosure of the Invention

An objective of the present invention is to provide methods for obtaining polynucleotides encoding antibodies having a specific reactivity from antibody-producing cells comprised in a polyclonal cell population, and methods for producing such antibodies.

In general, to clone antibody genes using gene recombination techniques, B cells cloned by some technique are required. This limitation poses difficulties for, for example, obtaining antibodies produced by B cells which have infiltrated cancer tissues. The present inventors searched for methods that would enable one to obtain antibody genes without having to rely on B cell cloning, and conceived that it may be possible to utilize cellular mRNAs isolated from cell populations as a cloning source. In general, B cells in peripheral blood are used to clone human antibody genes. However, since B cell populations in peripheral blood are polyclonal cell populations producing antibodies with various reactivities, these B cells were thought to be inappropriate for selectively isolating antibodies with a specific reactivity.

Microdissection is a technique for excising and isolating specific cells from a specimen composed of heterogeneous cell populations such as a tissue section. For example, a system for isolating cells in which the periphery of target cells is excised with an ultraviolet lases has been put into practical use. This system is referred to as laser microdissection (LMD), and is commercially available. LMD has spread as a technique that can be used to obtain target cells with high precision and little damage to cells. By using LMD, a specific cellular gene can be obtained and amplified by PCR.

The present inventors thought that B cells which have infiltrated cancer tissues are very likely to produce antibodies having reactivities to cancer cells, and therefore, polynucleotides encoding antibodies which bind to cancer cells may be efficiently isolated by isolating infiltrating B cells using the LMD system or such. Then, the present inventors showed that polynucleotides encoding the antibodies can be obtained by utilizing, as a cloning source, B cells which have actually infiltrated a lesional tissue, and thereby completed the present invention. That is, the present invention, as described below, relates to methods for isolating antibody genes and preparing antibodies encoded by these genes. Further described herein are antibodies obtained by these methods. In particular, the present invention provides:
1. A method for isolating a polynucleotide encoding an antibody against a lesional tissue, wherein the method comprises the step of obtaining a polynucleotide encoding an antibody from a B cell obtained by isolating a lesional tissue-infiltrating B cell, wherein the lesional tissue-infiltrating B cell is isolated by excising a region comprising a B cell from an isolated section of said lesional tissue by laser microdissection, wherein the lesional tissue is derived from solid tumor lesions, arteriosclerosis lesions, inflammatory disease lesions, lesions generated by infectious pathogens, or autoimmune disease lesions.
2. The method of item 1, wherein the lesional tissue is a cancer tissue.
3. The method of item 2, wherein the cancer tissue is selected from the group consisting of breast cancer tissues, lung cancer tissues, liver cancer tissues, colon cancer tissues, pancreas cancer tissues and prostate cancer tissues.
4. The method of item 1, wherein the polynucleotide encoding an antibody is obtained by amplifying a gene encoding an antibody variable region.
5. A method for producing an antibody, wherein the method comprises the steps of:
   (i) - obtaining a polynucleotide encoding an antibody from a B cell obtained by isolating a lesional tissue-infiltrating B cell, wherein the lesional tissue-infiltrating B cell is isolated by excising a region comprising a B cell from an isolated section of said lesional tissue by laser microdissection, wherein the lesional tissue is derived from solid tumor lesions, arteriosclerosis lesions, inflammatory disease lesions, lesions generated by infectious pathogens, or autoimmune disease lesions;
   (ii) preparing an expression vector comprising the polynucleotide of step (i),
   (iii) transforming a host cell with the expression vector of step (ii) to obtain a host cell expressing the polynucleotide,
   (iv) culturing the host cell of step (iii); and
   (v) recovering an antibody which is the expression product.
6. The antibody production method of item 5, wherein the method further comprises the steps of
   (vi) contacting the antibody obtained by the method of item 5 with a lesional tissue;
   (vii) detecting the binding between the antibody and the lesional tissue; and
   (viii) selecting an antibody that binds to the lesional tissue.
7. The method of item 1, wherein the number of B cells to be isolated is 20 cells or less.
8. The method of item 7, wherein the number of B cells to be isolated is 5 cells or less.

The present invention is defined by the claims.

The present invention relates to methods for isolating a polynucleotide encoding an antibody against a lesional tissue, wherein the methods comprise the steps of:
(a) isolating a lesional tissue infiltrating B cell; and
(b) obtaining an antibody-encoding polynucleotide from the isolated B cell.

B cells which have infiltrated a lesion are very likely to produce antibodies against that lesion. In other words, it can be said that lesions have accumulations of B cells that produce antibodies recognizing the lesion. Therefore, antibody genes derived from B cells isolated from a cell population that has infiltrated a lesion is useful for generating antibodies against that lesion. In this invention, the term "antibodies against a lesion" refers to antibodies that recognize the antigens which constitute the lesion or antigenic substances produced at the lesion. Such antibodies are useful for diagnosing and treating the lesion. Furthermore, when the lesion is due to an autoimmune disease, such antibodies provide important information for epitope analysis in the autoimmune disease.

For the methods of the present invention, any lesion recognized as non-self by the immune system can be utilized. For example, the following lesions are preferable for obtaining B cells. These lesions can be referred to as naturally occurring lesions, which can also be obtained from human subjects being treated for said lesions.
- solid tumor lesions
- arteriosclerosis lesions

- inflammatory disease lesions
- lesions generated by infectious pathogens
- autoimmune disease lesions
   On the other hand, artificially prepared lesions can also be utilized in this invention. For example, the following lesions are artificially prepared lesions, and can be obtained from, for example, immune animals. By utilizing artificial lesions, polynucleotides encoding antibodies against any antigen can be obtained.
- heterogenous cells and tissues artificially transplanted into immune animals
- cells and tissues that express non-self genes artificially transplanted into immune animals

Cancer tissues can be exemplified as preferable lesions of the present invention. Specifically, the present invention relates to methods for isolating a polynucleotide encoding an antibody against a cancer cell, wherein the method comprises the steps of:
(a) isolating a cancer tissue-infiltrating B cell; and
(b) obtaining an antibody-encoding polynucleotide from the isolated B cell.

Cancer tissues to be used in this invention are not limited. Specifically, breast cancer tissues, lung cancer tissues, liver cancer tissues, colon cancer tissues, pancreas cancer tissues, prostate cancer tissues, and such can be used. Among them, cancers comprising many B cell infiltrations are preferable cancer tissues of this invention. Examples of cancers comprising many B cell infiltrations include breast cancers, lung cancers, and melanomas. Cancer tissues can be removed by surgical excision. For example, cancer tissues removed for biopsy can be used as cancer tissues for the present invention. Furthermore, tissues taken out from patients by surgical removal are also useful as cancer tissues. These tissues can be those removed for obtaining antibody genes; alternatively, tissues removed for histopathological examination or surgical treatment can also be used.

In this invention, the method used for isolating B cells which infiltrate cancer tissues is laser microdissection, a technique for excising specific cells from a tissue section. For example, target cells can be isolated from frozen tissue sections using the Laser Microdissection (LMD) system. A system for excising tissue sections with an ultraviolet laser is commercially available. The use of this system, which involves specifying the region to be excised on a computer image under microscopic observation, makes it possible to excise any region from a tissue section.

In this case, a large number of B cells can be isolated by observing a specimen under a microscope and selecting a portion enriched with B cells. Alternatively, a small number of B cells can be easily obtained by excising a region with low B cell density. As shown in the Examples, it is even possible to obtain a single cell.

In the present invention, B cells can be isolated from any specimen prepared for pathological analysis. For example, thin-section frozen specimens are preferable pathological specimens in this invention. As a pathological specimen, not only fresh tissues but also specimens fixed with paraformaldehyde (PFA) or such can be used. Accordingly, it is also possible to obtain antibody genes from, for example, preserved pathological specimens using the methods of the present invention. As described above, a broad range of cloning sources can be selected for the methods of this invention. That is, the present invention provides methods that can be conveniently used to obtain a variety of antibody genes.

Microdissection is a system also utilized in gene analysis of specialized cells in tissues using PCR or such. However, there has been no report on the utilization of microdissection for obtaining antibody genes. So far, LMD is only considered as a diagnostic method for cancer (Bonner et al. (1997) Science 278:1481). The present inventors focused on the fact that it is possible to use lesion-infiltrating B cell populations as cell populations that are very likely to produce antibodies with a desired reactivity. Furthermore, by using the antibody-producing cells obtained from such a cell population as the cloning source, the present inventors thus made it possible to obtain antibody genes.

More specifically, B cells or plasma cells which infiltrate cancer tissues and produce antibodies can be extracted based on pathological analysis under microscopic observation. B cells and plasma cells can be distinguished by staining with toluidine blue or such. Compared with conventional methods in which B cells or plasma cells are extracted from tissue fractions comprising mixtures of peripheral blood and cancerous/non-cancerous portions, this method makes it possible to isolate genes of antibodies that recognize cancer cells with a much higher probability.

In the present invention, the term "cell isolation" refers to the separation of B cells from a cell population comprising mixtures of heterogeneous cells. The "cell isolation" of the present invention includes isolation of cells harboring antigen genes when they coexist with other cells that do not carry an antibody gene. For example, as shown in the Examples, carrier cells that are clearly incapable of producing antibodies may be added to the antigen-producing cells. Carrier cells are added to facilitate the extraction of target mRNAs. That is, even if only a limited number of antibody-producing cells are coexisting with other cells which do not carry antibody genes, these antibody-producing cells can be said to be in an isolated state.

In this invention, the number of B cells to be isolated is not limited. Specifically, for example, one to 100 cells, usually one to 50 cells, preferably 20 cells or less, more preferably five cells or less, and yet more preferably one cell is isolated.

Isolation of single cells enhances the possibility of obtaining an antibody gene in a state that maintains its heavy and light chain combinations. When reconstructing functional antibody molecules, it is an important condition to obtain an antibody gene in such a state. In cloning antibody genes, the use of monoclonal antibody-producing cells as a cloning source is an essential condition for accurately reconstructing combinations of heavy and light chains. However, if an antibody gene obtained from a single cell is used, repeated cloning of the gene would be required when comparing multiple antibodies with different reactivities. Comparison of multiple antibodies is useful for obtaining a suitable antibody with purpose-oriented characteristics.

On the contrary, when multiple cells are isolated as the cloning source, combinations of the heavy and light chains of the obtained antibody genes cannot be identified. However, it is possible to obtain antibody genes derived from multiple cells, that is, a library of antibody genes. By screening such a library with antibody activity as an index, genes of antibodies with the desired reactivity can be obtained. It is impossible to confirm whether heavy and light chain combinations of an antibody gene selected by screening are derived from the same cell. However, regardless of whether the genes are derived from the same cell or not, the purpose of screening is accomplished by obtaining antibodies having the desired reactivity.

In the methods of the present invention, any lesion-infiltrating antibody-producing cell can be used as a cloning source. Normally, B cells circulating in peripheral blood have various levels of differentiation. At the early stages of differentiation, B-cells have µ-chains on their surfaces as an antigen receptor. B cells mature and differentiate into IgG-secreting cells through differentiation and activation by antigen stimulation. B cells at the final stage of differentiation are referred to as plasma cells (or plasmocytes). Plasma cells produce 2,000 molecules of IgG per second. Therefore, by isolating plasma cells, more mRNAs can be obtained.

In general, many lesion-infiltrating B cells are found to be at an advanced stage of differentiation. Further, it is highly likely that the infiltrating B cells produce antibodies against the lesion. Therefore, by using B cells isolated from a lesion as the cloning source, the specificity of the resulting antibody will be naturally centered on the lesion. Furthermore, antibody genes are expressed at an extremely high level in plasma cells and in B cells at an advanced stage of differentiation. Accordingly, utilization of such cells as the cloning source leads to increased possibility of obtaining antibody genes. By utilizing lesion-infiltrating B cells, antibody genes of interest can be obtained with a high probability using relatively few cells.

Antibody genes can be obtained from isolated B cells by amplifying the antibody genes. Methods for amplifying genes are known in the art. For example, the PCR method is preferred as a method for amplifying antibody genes. A method for isolating antibody genes using the PCR method is described below.

First, mRNAs are extracted from isolated B cells. cDNAs are synthesized by using the extracted mRNAs as a template to obtain a cDNA library. Commercially available kits are conveniently used for extracting mRNAs and for constructing the cDNA library. In this invention, mRNAs derived from a small number of B cells are used. In practice, mRNAs obtained from only few cells are extremely small in amount, and have low yields when directly purified. Therefore, mRNAs are usually purified after the addition of carrier RNAs that clearly contain no antibody genes. Alternatively, with a certain amount of RNAs being extracted, the mRNAs of antibody-producing cells themselves can also be efficiently extracted. The addition of carrier RNAs may not be required for extracting mRNAs from, for example, 10 or more, 30 or more, preferably 50 or more antibody-producing cells.

By using the cDNA library thus obtained as a template, antibody genes can be amplified by the PCR method. Primers for amplifying antibody genes by the PCR method are known in the art. For example, primers for amplifying human antibody genes can be designed based on the disclosures in research papers (J. Mol. Biol. (1991) 222, 581-597) and web sites (http://www.mrc-cpe.cam.ac.uk/vbase-ok.php?menu=901). These primers have different nucleotide sequences depending on the subclass of immunoglobulins. Accordingly, when a cDNA library with unknown subclasses is used as a template, the PCR method is performed considering all possibilities.

For example, to obtain genes encoding human IgG, primers that allow the amplification of genes encoding γ1 to γ5 as the heavy chain, and κ-chain and λ-chain as the light chains, can be used. For amplifying genes of the IgG variable region, a primer capable of annealing to a portion that corresponds to the hinge region is generally used as the 3' primer. Meanwhile, a primer corresponding to each subclass can be used as the 5' primer.

PCR products obtained using primers for amplifying genes of the respective heavy and light chain subclasses are considered as independent libraries. Using thus synthesized libraries, it is possible to reconstitute immunoglobulins comprising combinations of heavy chains and light chains. The lesion-binding activity of the reconstituted immunoglobulin can be used as an index to screen for antibodies of interest.

For example, to obtain antibodies targeting cancer tissues, it is preferred that the antibodies of the present invention bind to cancer cells. It is even more preferable that the antibodies bind specifically to cancer cells. Cancer-binding antibodies can be screened by, for example, the following steps of:
(1) contacting an antibody obtained by a method of the present invention with a cancer cell;
(2) detecting binding between the antibody and the cancer cell; and
(3) selecting an antibody that binds to the cancer cell.

Methods for detecting binding between antibodies and cancer cells are known in the art. Specifically, an immobilized cancer specimen is reacted with a test antibody, and then with a labeled secondary antibody which recognizes the test antibody. The binding of the test antibody to cancer cells can be confirmed by detecting labeled antibody on the immobilized specimen after washing. Enzymatically active proteins such as peroxidase and β-galactosidase, and fluorescent substances such as FTTC can be used as the label. Cancer tissues to be analyzed for antibody-binding activity may be cancer tissues forming the lesion from which B cells are obtained. Alternatively, cancer tissues of the same organ which have been removed from different individuals, and cell lines derived from these cancer tissues, can be also used. Furthermore, cancer tissues of different organs, or cell lines derived therefrom, can also be used to screen for antibodies which commonly react with different types of cancers.

In the present invention, an antibody whose reactivity towards a cancer tissue is significantly high compared to the normal tissue can be said to bind specifically to the cancer. For comparing the reactivities of the antibodies of this invention, the same type of tissue is generally used. That is, the reactivity of an antibody is compared between a cancer tissue and a normal tissue of the organ from which the cancer tissue is derived. Under conditions in which an antibody's reactivity towards a cancer tissue can be confirmed, the antibody is said to have a specific reactivity towards the cancer tissue when no binding activity towards the normal tissue can be detected.

Methods of screening for antibodies using binding activity as an index include the panning method which utilizes phage vectors. When the obtained antibody genes are gene libraries of heavy and light chain subclasses as described above, screening methods that use phage vectors are advantageous. As described in the Examples, genes encoding variable regions of the heavy and light chains can be made into single chain Fv (scFv) through conjugation with a suitable linker sequence. Phages expressing scFv on their surface can be obtained by inserting a scFv-encoding gene into a phage vector. By contacting these phages with an antigen of interest and recovering the phages bound to the antigen, DNAs encoding scFv with the desired binding activity can be recovered. scFv having the desired binding activity can be concentrated by repeating this procedure as necessary.

An antibody-encoding polynucleotide of the present invention may encode a whole antibody or a portion of the antibody. The term "a portion of the antibody" means any portion of an antibody molecule. Hereinbelow, a portion of an antibody may be referred to as "an antibody fragment". Preferred antibody fragments of the present invention comprise the complementarity determination region (CDR) of an antibody. More preferably, antibody fragments of the present invention comprise all three CDRs that constitute the variable region.

For example, polynucleotides encoding the variable region of an antibody are preferred antibody fragments of the present invention. A complete immunoglobulin molecule can be reconstituted by obtaining a polynucleotide encoding a variable region and linking the polynucleotide with a polynucleotide encoding a constant region. Constant regions of antibodies within the same class have approximately the same structure. That is, the structure of the constant region has no effect on the antigen-binding activity. Accordingly, if the structure of the variable region of an antibody can be elucidated, antibodies having similar activity can be reconstituted through conjugation with the constant region that is already obtained.

Antibodies obtained by the methods of the present invention include genetically recombinant antibodies whose structures have been artificially modified. For example, mouse-human chimeric antibodies can be prepared by: conjugating a human constant region gene with an antibody gene obtained not from a human but from a non-human animal such as a mouse. Methods for humanizing a mouse variable region by transferring CDRs which constitute the variable region of a non-human animal such as mouse to a human variable region are also known in the art.

Polynucleotides encoding the antibodies of the present invention can be DNAs, RNAs, or chimeric molecules thereof. Furthermore, artificial structures such as PNAs can also be included, as long as the nucleotide sequence is maintained. Methods for synthesizing polynucleotides having the same nucleotide sequence, based on the nucleotide sequence of a gene that encodes an antibody isolated from B cells, are known in the art.

The polynucleotides of the present invention can comprise a sequence identical or highly homologous to the gene encoding an antibody isolated from B cells. Herein, the term "highly homologous" generally refers to a homology of 70% or more, preferably 80% or more, more preferably 90% or more, and even more preferably 95% or more.

The present invention relates to polynucleotides encoding antibodies obtained by the above-described methods. The polynucleotides of this invention can be incorporated into any expression vector. Appropriate host cells can be transformed with the expression vector to obtain antibody-expressing cells. By culturing the antibody-expressing cells and then recovering expression products therefrom, antibodies encoded by the genes can be obtained. The following describes expressing antibody genes isolated by the above-described methods.

When the antibody genes have been isolated and introduced into an appropriate host, hosts and expression vectors can be used in appropriate combinations to produce the antibodies. As eukaryotic host cells, animal cells, plant cells, and fungal cells may be used. Known animal cells include: (1) mammalian cells such as CHO, COS, myeloma, baby hamster kidney (BHK), HeLa, and Vero cells; (2) amphibian cells such as *Xenopus* oocytes; or (3) insect cells such as sf9, sf21, and Tn5. Known plant cells include cells derived from the *Nicotiana* genus such as *Nicoriana tabacum,* which can be callus cultured. Known fungal cells include yeasts such as the *Saccharomyces* genus, for example *Saccharomyces cerevisiae*, and filamentous fungi such as the *Aspergillus* genus, for example *Aspergillus niger*. Prokaryotic cells can also be used in production systems that utilize bacterial cells. Known bacterial cells include *E. coli* and *Bacillus subtilis.* The antibodies can be obtained by transferring the antibody genes of interest into these cells using transformation, and then culturing the transformed cells *in vitro.*

Furthermore, the antibodies obtained by the methods of the present invention may be antibody fragments or modified antibodies thereof. For example, the antibody fragments may be Fab, F(ab')2, Fv, or single chain Fv (scFv) in which Fv from H or L chains are ligated by an appropriate linker. More specifically, the antibody fragments can be obtained by treating the antibodies with enzymes such as papain and pepsin to produce antibody fragments. Alternatively, genes encoding these antibody fragments can be prepared and introduced into expression vectors for expression in appropriate host cells (see, for example, Co, M. S. et al., J. Immunol. (1994) 152, 2968-2976; Better, M. & Horwitz, A. H. Methods in Enzymology (1989) 178, 476-496, Academic Press, Inc.; Plueckthun, A. & Skerra, A. Methods in Enzymology (1989) 178, 476-496, Academic Press, Inc.; Lamoyi, E., Methods in Enzymology (1989) 121, 663-669; and Bird, R. E, et al., TINRECH (1991) 9, 132-137).

scFv can be obtained by ligating the V regions of the antibody H-chain and L-chain. In the scFv, the V regions of the H chain and L chain are ligated via a linker, and preferably via a peptide linker (Huston, J. S. et al., Proc. Natl. Acad. Sci. U.S.A (1988) 85, 5879-5883). The V regions of the scFv H chain and L chain may be derived from any of the antibodies described herein.

The peptide linker used to ligate the V regions may be any single-chain peptide consisting of 12 to 19 residues. DNA encoding scFv can be amplified by PCR using as a template either the whole DNA, or a partial DNA encoding a desired DNA, selected from a DNA encoding the H chain or the V region of the H chain of the above antibody, and a DNA encoding the L chain or the V region of the L chain of the above antibody; and using a primer pair that defines the two ends. Further amplification can be subsequently conducted using the combination of DNA encoding the peptide linker portion, and the primer pair that defines both ends of the DNA to be ligated to the H chain and the L chain respectively. Once DNAs encoding scFvs are constructed, expression vectors containing the DNAs, and hosts transformed by these expression vectors, can be obtained according to conventional methods. Furthermore, scFvs can be obtained according to conventional methods using the resulting hosts.

These antibody fragments can be produced in hosts by obtaining genes encoding the antibody fragments and expressing them in a similar manner to that outlined above. Antibodies bound to various types of molecules, such as polyethylene glycol (PEG), may be used as modified antibodies. Furthermore, antibodies may bind to radioisotopes, chemotherapeutics, cytotoxic substances such as bacteria-derived toxin, and labeling substances. Such modified antibodies can be obtained by chemically modifying the resulting antibodies. Methods for modifying antibodies are already established in the art. The term "antibody" in the present invention also encompasses the modified anybodies as described above.

Furthermore, the antibodies obtained by the methods of the present invention may be bispecific antibodies. The bispecific antibodies may have antigen-binding sites recognizing different epitopes on the antigen molecules, or may have one antigen-binding site recognizing antigens and the other recognizing a cytotoxic substance such as radioactive substance, chemotherapeutic agent, and cell-derived toxin. In this case, it is possible to inhibit the growth of cancer cells by directly applying the cytotoxic substance to the cells expressing antigens to specifically damage them. Bispecific antibodies can be prepared by linking HL pairs of two kinds of antibodies, or obtained by fusing hybridomas that produce different monoclonal antibodies to prepare fused cells generating bispecific antibodies. Furthermore, bispecific antibodies can be generated by using genetic engineering techniques.

Antibodies expressed and produced as described above can be purified by conventional methods for purifying normal proteins. Antibodies can be separated and purified by, appropriately selecting and/or combining affinity columns such as protein A columns or chromatography columns, filtration, ultrafiltration, salt precipitation, dialysis, and such (Antibodies A Laboratory Manual. Ed Harlow, David Lane, Cold Spring Harbor Laboratory, 1988).

Conventional means can be used to measure the antigen-binding activity of the antibodies (Antibodies A Laboratory Manual. Ed Harlow, David Lane, Cold Spring Harbor Laboratory, 1988). For example, enzyme linked immunosorbent assays (ELISAs), enzyme immunoassays (EIAs), radioimmunoassays (RIAs), or fluoroimmunoassays may be used.

Techniques common to the field of genetic engineering can be used to carry out procedures for constructing an expression system for producing the antibodies of the present invention, and for constructing recombinant vectors appropriate for the hosts (for example, Sambrook et al., Molecular Cloning, Cold Spring Harbor Laboratories (1989)). Host cells may be prokaryotic cells such as bacteria, and eukaryotic cells such as yeast, animal cells, insect cells, and plant cells, as long as the cells are capable of expressing the light chains of the present invention or the antibodies comprising the light chains. Mammalian cells are particularly preferred in view of glycosylation.

Expression vectors need to comprise units that regulate the transcription and translation of genetic information, such as promoters and terminators. For example, when *Escherichia* microorganisms such as *E. coli* are used as hosts, plasmids of the pBR or pUC series can be used as plasmid vectors, and any promoters selected from those such as lac, trp, tac, trc, λ phage PL, and PR can be used. Terminators may originate from trpA, phage, and rmB ribosomal RNA.

When the hosts are *Bacillus* microorganisms such as *B. subtilis*, plasmids such as those of the pUB110 and pC194 series can be used, and genes may be integrated into chromosomes in some cases. Promoters and terminators may be derived from apr, npr, amy, and such.

Other prokaryotic cells include microorganisms such as *Pseudomonas* (e.g. *P. putida, P. cepacia*; pKT240 vectors, and such), *Brevibacteria* (e.g. *B. lactofermentum*; pAJ43), *Corynebacteria* (e.g. *C. glutamicum*; pCS11, pCB101), *Streptococcus* (e.g. pHV1301, pGK1), *Lactobatcillaceae* (e.g. pAMβ1), *Rhodcoccus* (e.g. plasmids isolated from *R. rhodochrous* (J. Gen. Microbiol. 138: 1003 (1992)), *Streptomyces* (e.g. *S. lividans, S.virginiae*; pIJ486, pKC1064, pUWL-KS), *Enterobacter, Erwinia, Klebsiella, Proteus, Salmonella* (e.g. *S. typhimurium*), *Serratia* (e.g. *S. marcescans*), and *Shigella.*

Among expression systems utilizing eukaryotic microorganisms, a system using *Saccharomyces cerevisiae* as a host, and plasmids from YRp, YEp, YCp, and YIp series is known. Therein, promoters and terminators such as ADH, GAPDH, PHO, GAL, PGK, and ENO can be used. Other microorganisms used in the expression vector systems of the present invention include *Kluyveromyces* (e.g. *K. lactis;* plasmids of the 2 µm, pKDI, pGKI1, and KARS series, and such), *Schizosaccharomyces* (e.g. *S. pombe;* pAUR224), *Zygosaccharomyces* (e.g. *Z. rouxii*; pSB3 and PH05 promoters from *S. cerevisiae*), *Hansenula* (e.g. *H. polymorpha*), *Pichia* (e.g. *P. pastoris*), *Candida* (e.g. *C. maltosa, C. tropicalis, C. utilis,* and *C. albicans*), *Aspergillus* (e.g. *A. oryzae, A. niger*), and *Trichoderma* (e.g. *T. reesei*).

In another embodiment, plant cells may be used as hosts. For example, host cells may be those from cotton, corn, potato, tomato, soybean, petunia, and tobacco. A particularly well-known system uses cells from *Nicotina tabacum,* which are cultured as a callus. To transform plant cells, expression vectors such as pMON530 are introduced into bacteria such as *Agrobacterium tumefaciens.* By infecting these bacteria into tobacco (*Nicorina tabacum*), desired polypeptides can be obtained from the tobacco leaves.

Cells from insects such as silkworms (*Bombyx mori*), mosquitoes (e.g. *Aede aegypti, Aedes albopictus*) and fruit flies (*Drosophila melanogaster*) can be used as hosts. For example, when using silkworms as hosts, DNAs encoding antibodies may be inserted into baculovirus vectors, these vectors may be used to infect silkworms, and desired polypeptides can be obtained from the silkworm body fluids (Nature 315: 592-594 (1985)).

Examples of expression vectors when using animal cells as hosts include pME18S (Med. Immunol. 20: 27-32 (1990)), pEF-BOS (Nucleic Acids Res. 18: 5322 (1990)), pCDM8 (Nature 329: 840-842 (1987)), pRSVneo, pSV2-neo, pcDNAI/Amp (Invitrogen), pcDNAI, pAMoERC3Sc, pCDM8 (Nature 329: 840 (1987)), pAGE107 (Cytotechnology 3: 133 (1990)), pREP4 (Invitrogen), pAGE103 (J. Biochem. 101: 1307 (1987)), pAMoA, pAS3-3, pCAGGS (Gene 108: 193-200 (1991)), pBK-CMV, pcDNA3.1 (Invitrogen), and pZeoSV (Stratagene).

Promoters may be cytomegalovirus IE gene promoter and enhancer, SV40 early promoter, a retrovirus LTR such as those from RSV HIV, and MMLV, and gene promoters from animal cells such as metallothionein, β-actin, elongation factor-1, HSP genes, and such. Alternatively, viral vectors such as those mentioned above may also be used. Viral vectors may be derived from DNA viruses and RNA viruses such as retroviruses, adenoviruses, adeno-associated viruses, herpes viruses, vaccinia viruses, poxviruses, Simbu viruses, Sendai viruses. SV40, and HIV.

Host animal cells may be mouse myeloma cells (e.g. SP2/0, NSO), rat myeloma cells (e.g. YB2/0), mouse hybridoma cells, Namalwa cells (including KJM-1 cells), human embryonic kidney cells (e.g. 293 cells), human leukemia cells (e.g. BALL-1), CHO cells, COS cells (e.g. COS-1, COS-7), hamster embryonic kidney cells (e.g. BHK), mouse Sertoli cells (e.g. TM4), African green monkey kidney cells (e.g. VERO-76), HBT637 cells, HeLa cells, rabbit kidney cells (e.g. MDCK), human liver cells (e.g. HepG2), mouse mammary tumor cells (e.g. MMT060562), TRI cells, MRC cells, FS3 cells, etc.

Methods for introducing expression vectors depend on the type of host cell and vector, but any method can be used as long as it facilitates introduction of antibody-encoding DNA into cells. Vectors can be introduced into prokaryotic cells by methods utilizing calcium ions (Proc. Natl. Acad. Sci. USA 69: 2110 (1972)), protoplasts (Japanese Patent Application No. JP-A 6324829), electroporation (Gene 17:107 (1982); Molecular & General Genetics 168: 111 (1979)), and such; introduced into yeast cells by using electroporation (Methods in Enzymology, 194:182 (1990)), spheroplasts (Proc. Natl. Acad. Sci. USA 81:4889 (1984)), lithium acetate (J. Bacteriol. 153: 163 (1983)) ), and such; introduced into plant cells by using *Agrobacterium* (Gene 23: 315 (1983); WO89/05859), sonication (WO91/00358), and such; and into animal cells by using electroporation (Cytotechnology 3: 133 (1990)), calcium phosphate (JP-A 2227075), lipofection (Proc. Natl. Acad. Sci. USA 84: 7413 (1987); Virology 52: 456 (1973)), co-precipitation with calcium phosphate, DEAE-dextran, direct injection of DNA using microcapillaries), and such.

Transformants obtained as described above can be cultured, for example, by the following methods:

culture media for transformants of prokaryotes and eukaryotic microorganisms can be natural or synthetic, as long as the media facilitates efficient culture of the transformants, and comprises utilizable nutrients essential for growth, such as carbon and nitrogen sources, and inorganic salts. Culture may be carried out under aerobic or anaerobic conditions, and other conditions such as temperature, pH of the medium and duration of the culture can be determined appropriately by one skilled in the art, depending on the type of transformant. When using expression vectors equipped with inducible promoters, inducers may be added to the medium as necessary. For example, expression of vectors comprising the lac promoter can be induced by adding IPTG; and expression of vectors comprising the trp promoter can be induced by adding IAA as an inducer.

When using insect cells as hosts, a medium such as the TNM-FH medium (Pharmingen), Sf-900 II SFM (Life Technologies), ExCell400 and ExCell405 (JRH Biosciences), and Grace's Insect Medium (Nature 195: 788 (1962)) can be used. If necessary, antibiotics such as gentamicin may be added to the medium.

For animal cell transformants, common media such as RPMI1640 (The Journal of American Medical Association 199: 519 (1967)), Eagle's MEM (Science 122: 501 (1952)), DMEM (Virology 8: 396 (1959)), and 199 medium (Proceeding of the Society for the Biological Medicine 73: 1 (1950)), or such media added with BSA and the like, can be used. Culture can be carried out under normal conditions such as pH 6 to 8, 30 to 40°C, and 5% CO₂. If necessary, antibiotics such as kanamycin and penicillin may be added to the medium.

The antibodies of the present invention obtained as above can be isolated from within host cells, or from the culture medium if secreted in to the extracellular space using signal sequences. They can then be purified as substantially pure polypeptides. The antibodies of the present invention can be separated or purified by appropriately selecting, or combining as necessary, methods generally used in separation and purification. Such methods can be chromatography, filtration, ultrafiltration, salting out, solvent precipitation, solvent extraction, distillation, immunoprecipitation, SDS-polyacrylamide gel electrophoresis, isoelectric point focusing, dialysis, and recrystallization. Chromatography includes affinity chromatography, ion exchange chromatography, hydrophobic chromatography, gel filtration, reverse phase chromatography, absorption chromatography, and the like (Strategies for Protein Purification and Characterization: A Laboratory Course Manual, Daniel R Marshak et al. eds., Cold Spring Harbor Laboratory Press (1996); Antibodies: A Laboratory Course Manual, Harlow and David Lane eds., Cold Spring Harbor Laboratory Press (1988)). Such chromatographies may be performed using liquid chromatographies such as HPLC, FPLC, and the like. In addition, the antibodies of the present invention may be purified by making use of their affinities towards antigens.

### Brief Description of the Drawings

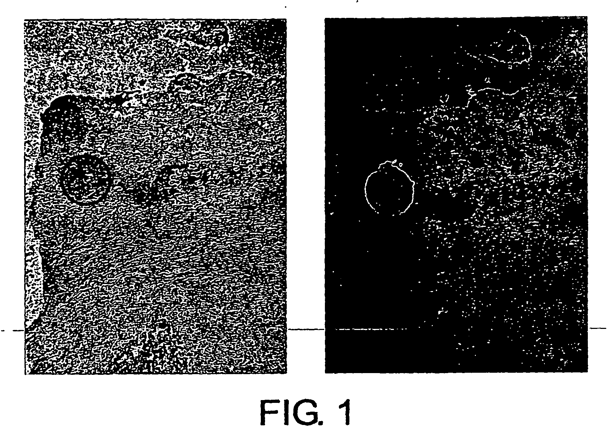

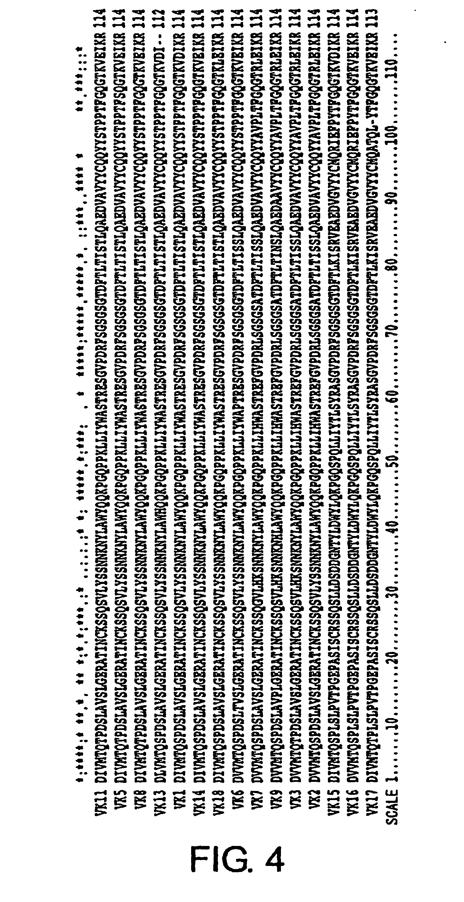

Fig. 1 shows photographs of approximately 200 plasma cells or B cells excised from a thin-section frozen specimen. Left: specimen before excision. Right: specimen after excision. The clear white spot in the right photograph shows the portion that was excised.

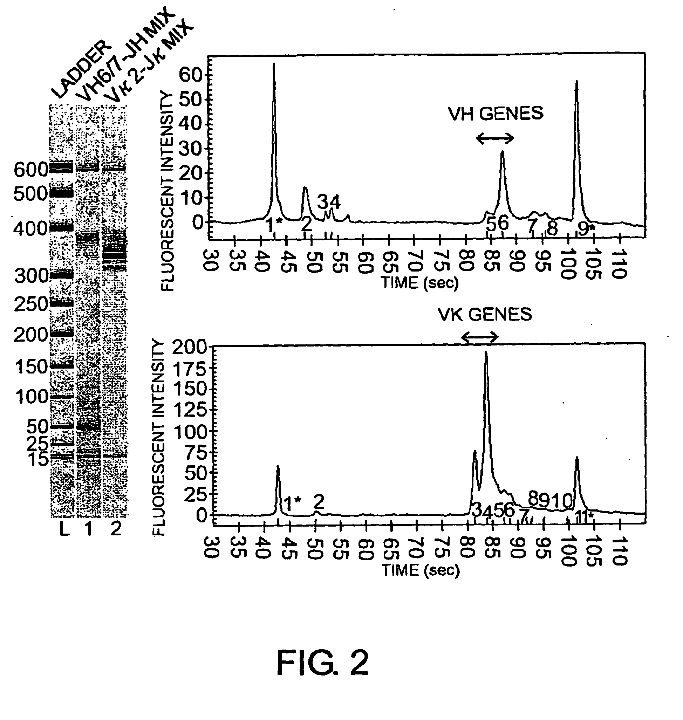

Fig. 2 shows photographs and graphs representing results of amplifying antibody genes expressed in approximately 200 plasma cells or B cells excised from a thin-section frozen specimen by RT-PCR, and analyzing a portion of the amplification products by electrophoresis using Agilent 2100. The photograph on the left represents the electrophoresis results of (from the left): molecular weight marker, amplification products of the heavy chain variable region (VH6/7-JHmix), and amplification products of the light chain variable region (Vκ2-Jκmix). Graphs on the right show electrophoresis time measured using Agilent 2100. The vertical axis shows the fluorescence intensity and horizontal axis shows the electrophoresis time (second).

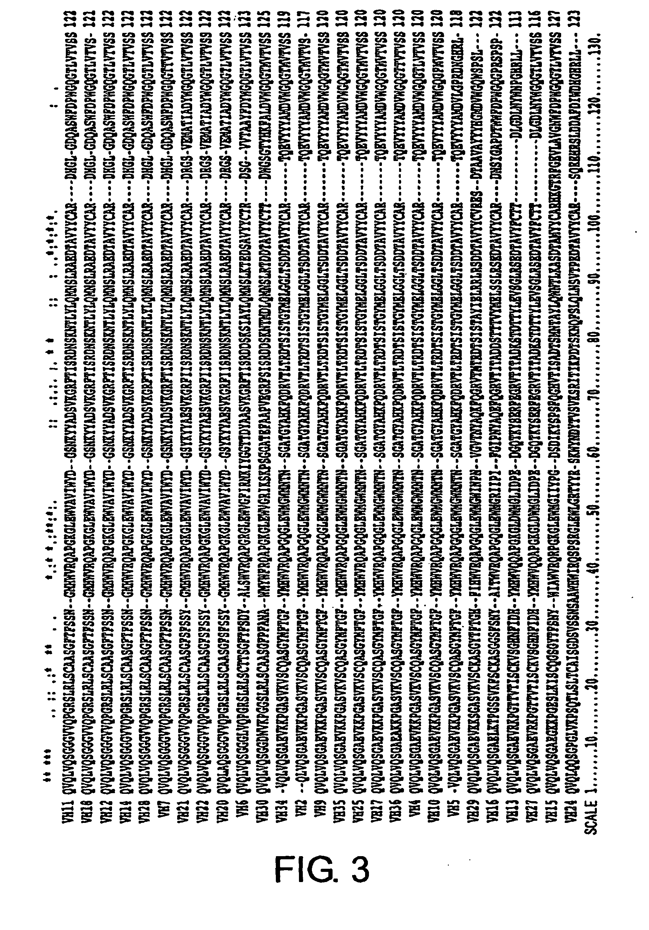

Fig. 3 represents results of determining nucleotide sequences for the heavy chain variable regions of antibodies expressed in the approximately 200 plasma cells or B cells excised from a thin-section frozen specimen, and performing a multiple alignment of the amino acid sequences encoded by these nucleotide sequences using ClustalX. The highly conserved positions are shown in the top row of the alignment. Levels of conservation are shown using three symbols, "*", ":", and ".", as follows:
   "*" Positions where identical amino acid residues are conserved in all sequences
   ":" Positions where an amino acid residue in any one of the following highly conserved groups is present in all sequences
      STA, NEQK, NHQK, NDEQ, QHRK, MILV, MILF, HY, and FYW
   "." Positions where an amino acid residue in any one of the following lowly conserved groups is present in all sequences
      CSA, ATV, SAG, STNK, STPA, SGND, SNDEQK,
      NDEQHK, NEQHRK, FVLIM, and HFY
Fig. 4 represents results of determining nucleotide sequences for the κ-chain variable regions of antibody genes expressed in the approximately 200 plasma cells or B cells excised from a thin-section frozen specimen, and performing a multiple alignment of the encoded amino acid sequences by ClustalX.

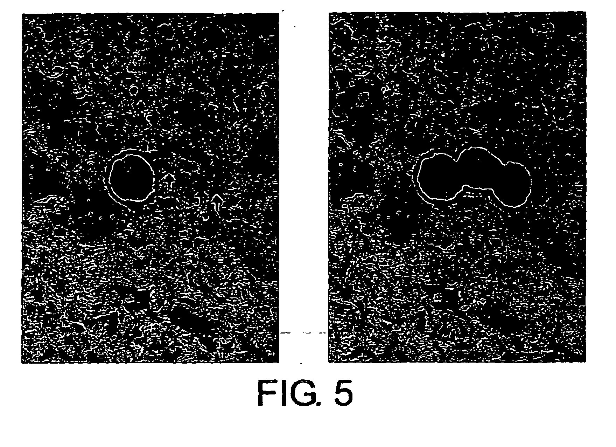

Fig. 5 shows photographs of five plasma cells or B cells excised from a thin-section frozen specimen. Left: specimen before excision. Right: specimen after excision. The white clear portion in the right photograph shows the portion that was excised.

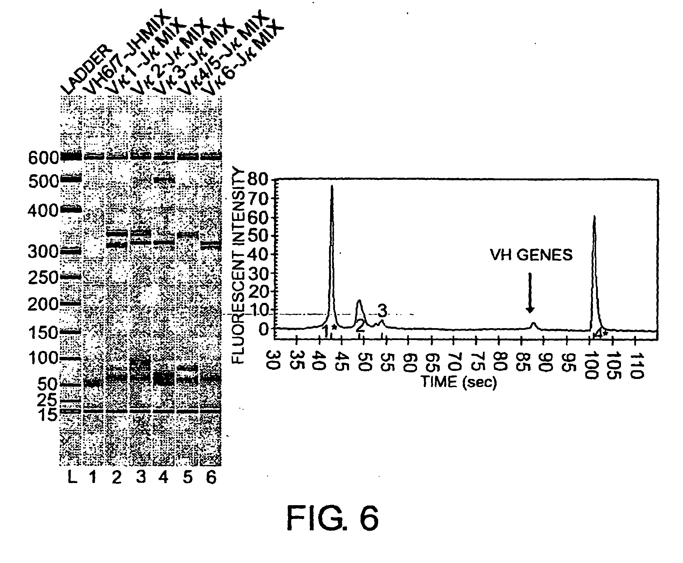

Fig. 6 shows a photograph and a graph representing the results of amplifying antibody genes expressed in the five plasma cells or B cells excised from a thin-section frozen specimen by RT-PCR, and analyzing a portion of the amplification products by electrophoresis using Agilent 2100. Electrophoresis results shown in the left photograph represent results of amplification using the following primer sets (from left to right):
   molecular weight marker;
   heavy chain variable region (VH6/7-JHmix);
   light chain variable region (Vκ1-Jκmix);
   light chain variable region (Vκ2-Jκcmix);
   light chain variable region (Vκ3-Jκmix);
   light chain variable region (Vκ4/5-Jκmix); and
   light chain variable region (Vκ6-Jκmix).

   The graph on the right shows electrophoresis time measured using Agilent 2100. The vertical axis shows fluorescence intensity, and the horizontal axis shows electrophoresis time (seconds).

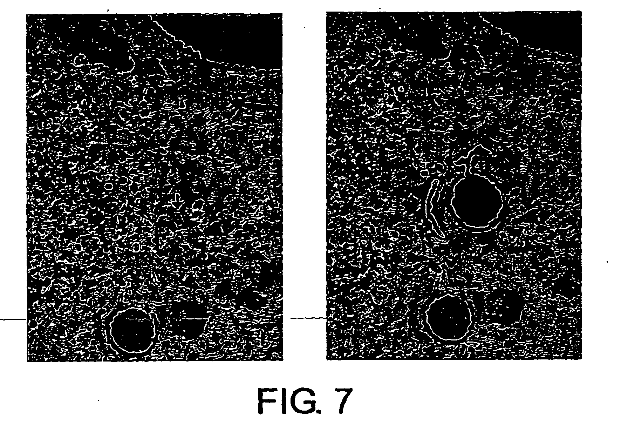

Fig. 7 shows photographs of a single plasma cell excised from a thin-section frozen specimen. Left: specimen before excision. Right: specimen after excision. The white clear portion in the right photograph shows the portion that was excised.

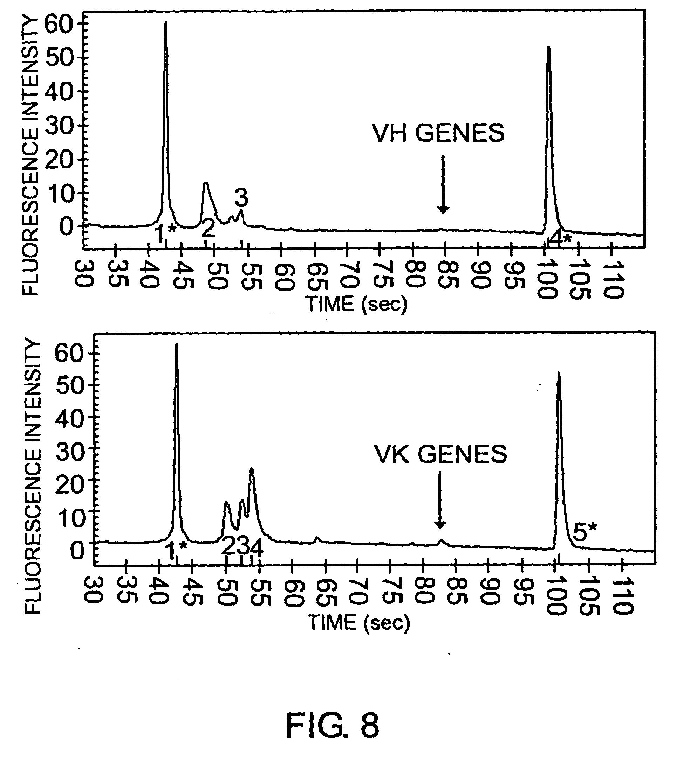

Fig. 8 shows graphs representing results of amplifying antibody genes expressed in a single plasma cell excised from a thin-section frozen specimen by RT-PCP, and analyzing a portion of the amplification products by electrophoresis using Agilent 2100. In the graphs, the vertical axis shows fluorescence intensity, and the horizontal axis shows electrophoresis time (seconds).

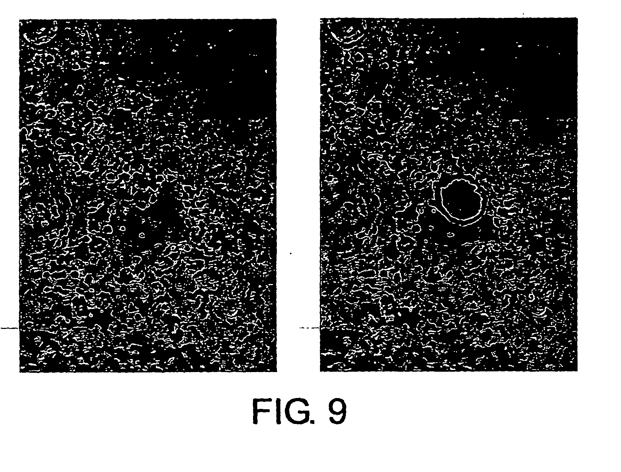

Fig. 9 shows photographs of a single plasma cell excised from a thin section frozen specimen. Left: specimen before excision. Right: specimen after excision. In the left photograph, arrow shows the spot to be excised. The white clear portion in the right photograph shows the portion that was excised.

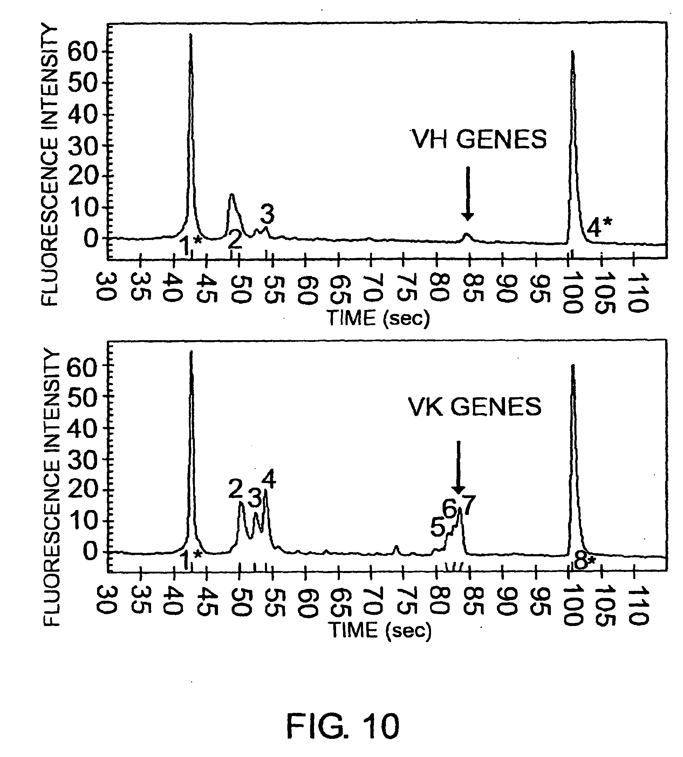

Fig. 10 shows graphs representing results of amplifying antibody genes expressed in a single plasma cell excised from a thin-section frozen specimen by RF-PCR and analyzing a portion of the amplification products by electrophoresis using Agilent 2100. In the graphs, the vertical axis shows fluorescence intensity, and the horizontal axis shows electrophoresis time (seconds).

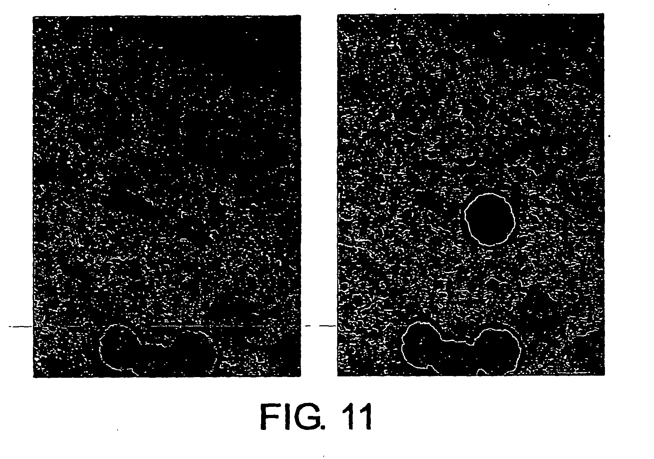

Fig. 11 shows photographs of a single plasma cell excised from a thin-section frozen specimen. Left: specimen before excision. Right: specimen after excision. Arrow in the left photograph shows the spot to be excised. The white clear portion in the right photograph shows the portion that was excised.
Fig: 12 shows a photograph and a graph representing the results of amplifying antibody genes expressed in a single plasma cell excised from a thin-section frozen specimen by RT-PCR, and analyzing a portion of the amplification products by electrophoresis using Agilent 2100. Electrophoresis results in the left photograph represent results of amplification using the following primer sets (from left to right):
   molecular weight marker;
   heavy chain variable region (VH6/7-JHmix);
   light chain variable region (Vκ1-Jκmix);
   light chain variable region (Vκ2-Jκmix);
   light chain variable region (Vκ3-Jκmix);
   light chain variable region (Vκ4/5-Jκmix); and light chain variable region (Vκ6-Jκmix).

   The graph on the right shows electrophoresis time measured using Agilent 2100. The vertical axis shows fluorescence intensity, and the horizontal axis shows electrophoresis time (seconds).

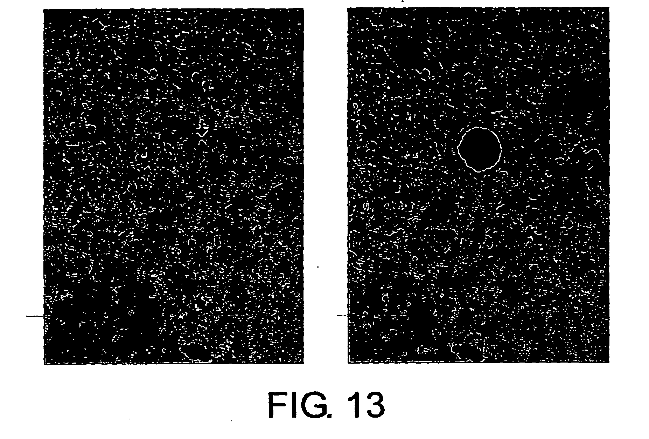

Fig. 13 shows photographs of a single plasma cell excised from a thin-section frozen specimen. Left: specimen before excision. Right: specimen after excision. Arrow in the left photograph shows the spot to be excised. The white clear portion in the right photograph represents the portion that was excised.

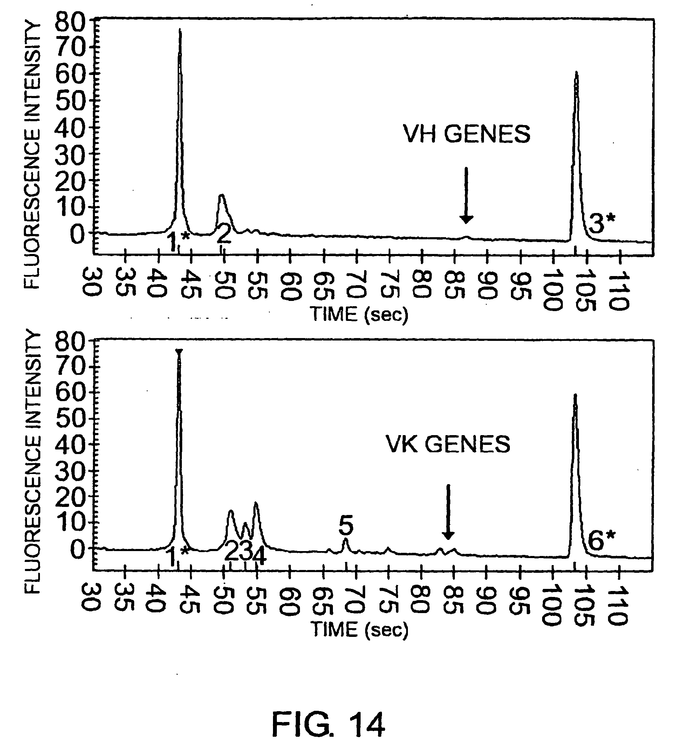

Fig. 14 shows graphs representing the results of amplifying antibody genes expressed in the single plasma cell excised from a thin-section frozen specimen by RT-PCP, and analyzing a portion of the amplification products by electrophoresis using Agilent 2100. In the graphs, the vertical axis shows fluorescence intensity, and the horizontal axis shows electrophoresis time (seconds).

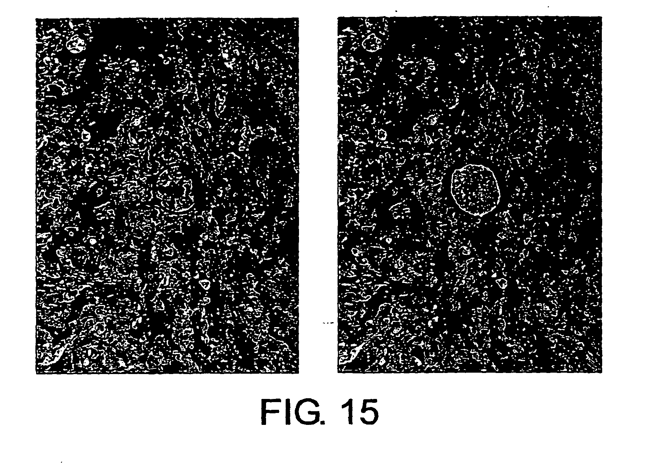

Fig. 15 shows photographs of a single plasma cell excised from a thin section of PFA-fixed frozen specimen. Left: specimen before excision. Right: specimen after excision. Cells to be excised are circled in the left photograph. The clear white portion in the right photograph represents the portion that was excised.

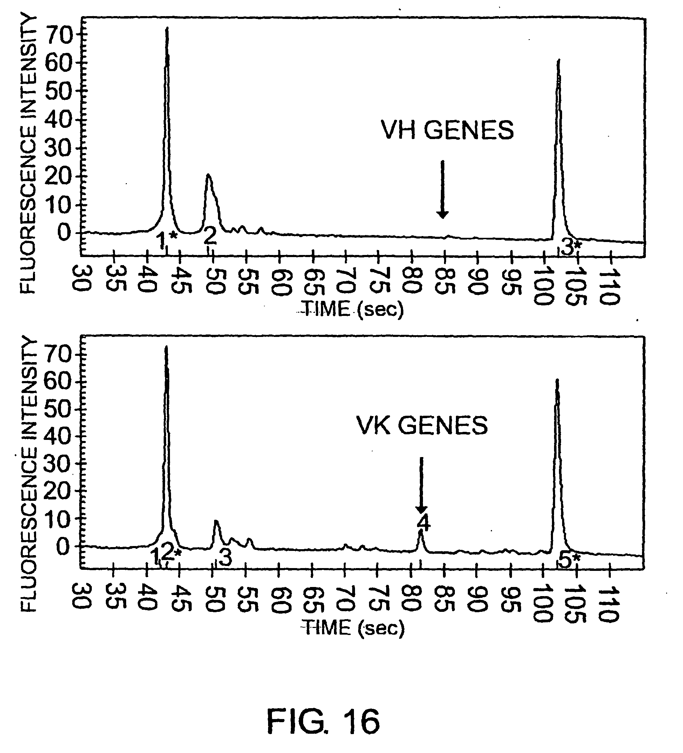

Fig. 16 shows graphs representing the results of amplifying antibody genes expressed in a single plasma cell excised from a thin-section PFA-fixed frozen specimen by RT-PCR, and analyzing a portion of the amplification products by electrophoresis using Agilent 2100. In the graphs, the vertical axis shows fluorescence intensity, and the horizontal axis shows electrophoresis time (seconds).

### Best Mode for Carrying Out the Invention

Herein below, the present invention will be specifically described using Examples, but it is not to be construed as being limited thereto.

### [Example 1] LMD isolation of a single cancer tissue-infiltrating B cell

A fresh human tissue (breast cancer tissue) was sliced into a suitable size to prepare frozen blocks using an OCT compound (Tissue-tek), and fixed prior to freezing with fixatives such as paraformaldehyde-lysine-periodate, as necessary. Next, thin specimen sections were prepared from each frozen block and attached onto LMD slides (Matsunami Glass Co.). Resulting thin-section frozen specimens were dried in air, fixed with fixatives such as acetone, and stained with toluidine blue (Muto Pure Chemicals Co., Ltd) or such. After staining, plasma cells were excised with a laser microdissection system (Leica AS-LMD), and recovered using a recovery buffer (RLT solution attached to QIAGEN RNeasy Mini Kit). Plasma cells before and after the excision are shown in Figs. 1, 5, 7, 9, 11, 13, and 15. In all figures, the left photograph shows the state of the cells before excision, and the right photograph shows the state of the cells after excision. Furthermore, when the cell(s) to be excised can be specified, they are indicated with an arrow or such in the "before excision" photographs (left).

### [Example 2] RNA preparation and cDNA synthesis

A suspension of approximately one to five B cells excised from a thin-section LMD specimen was mixed with a suspension of about 300 carrier cells which do not express any antibody genes. Total RNAs were prepared from the resultant mixture, using the RNeasy Mini Kit (QIAGEN) according to the manufacturer's instructions. When 50 cells or more were excised from the thin-section specimen, total RNAs were prepared without the addition of carrier cells. cDNA was synthesized using all 35 µl of the RNA eluate fraction as a template, and Sensiscript Reverse Transcriptase (QIAGEN) according to the manufacturer's instructions. cDNA synthesis reaction was performed on an 80-µl scale using 40 ng of Oligo dT primers (Promega) and 0.8 µg of random hexamers (Invitrogen) as the reverse transcription primers at 37°C for 1 h. When cDNAs thus synthesized were not immediately subjected to PCR, they were stored at -80°C.

### [Example 3] Cloning of human antibody variable regions

PCR primers to be used for cloning human antibody variable regions were designed based on the research paper "J. Mol. Biol. (1991) 222, 581-597", and the web site of Medical Research Council (MRC), "V BASE" (http://wwwmrc-cpe.cam.ac.uk/vbase-ok.php?menu=901). These nucleotide sequences are shown below (SEQ ID NOs: 97 to 150). Primers for cloning heavy chain variable regions are designated with VH and JH as the initial letters. Primers for amplifying the light chain κ-chain or the light chain λ-chain are designated with VK and JK, or VL and JL as the initial letters.
- VH1a: 5'-CAGGT(GT)CAGCTGGTGCAGTCTGG-3'
- VH1b: 5'-CAGGTCCAGCTTGTGCAGTCTGG-3'
- VH1c: 5'-(GC)AGGTCCAGCTGGTACAGTCTGG-3'
- VH1d: 5'-CA(AG)ATGCAGCTGGTGCAGTCTGG-3'
- VH2a: 5'-CAGATCACCTTGAAGGAGTCTGGT-3'
- VH2b: 5'-CAGGTCACCTTGA(AG)GGAGTCTGGT-3'
- VH3a: 5'-GA(AG)GTGCAGCTGGTGGAGTCTGG-3'
- VH3b: 5'-CAGGTGCAGCTGGTGGAGTCTGG-3'
- VH3c: 5'-GAGGTGCAGCTGTTGGAGTGTGG-3'
- VH4a: 5'-CAG(CG)TGCAGCTGCAGGAGTCGGGC-3'
- VH4b: 5'-CAGGTGCAGCTACAGCAGTGGGGC-3'
- VH5a: 5'-GA(AG)GTGCAGCTGGTGCAGTCTGGA-3'
- VH6a: 5'-CAGGTACAGCTGCAGCAGTCAGGT-3'
- VH7a: 5'-CAGGT(CG)CAGCTGGTGCAATCTGG-3'
- JH1245: 5'-TGAGGAGACGGTGACCAGGGT(GT)CC-3'
- JH3: 5'-TGAAGAGACGGTGACCATTGTCCC-3'
- JH6: 5'-TGAGGAGACGGTGACCGTGGTCCC-3'
- VK1a: 5'-(AG)ACATCCAGATGACCCAGTCTCCA-3'
- VK1b: 5'-G(AC)CATCCAGTTGACCCAGTCTCCA-3'
- VK1c: 5'-GCCATCC(AG)GATGACCCAGTCTCCA-3'
- VK1d: 5'-GTCATCTGGATGACCCAGTCTCCA-3'
- VK2a: 5'-GATATTGTGATGACCCAGACTCCA-3'
- VK2b: 5'-GAT(AG)TTGTGATGACTCAGTCTCCA-3'
- VK3a: 5'-GAAATTGTGTTGAC(AG)CAGTCTCCA-3'
- VK3b: 5'-GAAATAGTGATGACGCAGTCTCCA-3'
- VK3c: 5'-GAAATTGTAATGACACAGTCTCCA-3'
- VK4a: 5'-GACATCGTGATGACCCAGTCTCCA-3'
- VK5a: 5'-GAAACGACACTCACGCAGTCTCCA-3'
- VK6a: 5'-GAAATTGTGCTGACTCAGTCTCCA-3'
- VK6b: 5'-GATGTTGTGATGACACAGTCTCCA-3'
- JK1: 5'-ACGTTTGATTTCCACCTTGGTCCC-3'
- JK24: 5'-ACGTTTGATCTCCA(CG)CTTGGTCCC-3'
- JK3: 5'-ACGTTTGATATCCACTTTGGTCCC-3'
- JK5: 5'-ACGTTTAATCTCCAGTCGTGTCCC-3'
- VL1a: 5'-CAGTCTGTGCTGACTCAGCCACCC-3'
- VL1b: 5'-CAGTCTGTG(CT)TGACGCAGCCGCCC-3'
- VL2: 5'-CAGTCTGCCCTGACTCAGCCT(CG)-3'
- VL3a: 5'-TCCTATG(AT)GCTGACTCAGCCACCC-3'
- VL3b: 5'-TCCTATGAGCTGACACAGC(CT)ACCC-3'
- VL3c: 5'-TCTTCTGAGCTGACTCAGGACCCT-3'
- VL3d: 5'-TCCTATGAGCTGATGCAGCCACCC-3'
- VL4a: 5'-CAGCCTGTGCTGACTCAATCATCC-3'
- VL4b: 5'-CAGCTTGTGCTGACTCAATCGCCC-3'
- VL4c: 5'-CTGCCTGTGCTGACTCAGCCCCCG-3'
- VL5a: 5-CAGCCTGTGCTGACTCAGCCA(CT)CT-3'
- VL5c: 5'-CAGGCTGTGCTGACTCAGCCGGCT-3'
- VL6: 5'-AATTTTATGCTGACTCAGCCCCAC-3'
- VL7: 5'-CAG(AG)CTGTGGTGACTCAGGAGCCC-3'
- VL8: 5'-CAGACTGTGGTGACCCAGGAGCCA-3'
- VL4_9: 5-C(AT)GCCTGTGCTGACTCAGCCACCT-3'
- VL10: 5'-CAGGCAGGGCTGACTCAGCCACCC-3'
- JL1: 5'-ACCTAGGACGGTGACCTTGGTCCC-3'
- JL23: 5'-ACCTAGGACGGTCAGCTTGGTCCC-3'
- JL7: 5'-ACCGAGGACGGTCAGCTGGGTGCC-3'

For cloning the heavy chain variable regions, κ-chain variable regions, and λ-chain variable regions, PCR amplifications were carried out using combinations of the Taq DNA polymerase Core Kit (QIAGEN) and primer mixtures for each gene subset. For amplifying the heavy chain and κ-chain, five different prime mixtures were prepared for each. The kinds of reaction solutions were prepared using these primer mixtures. Combinations of primers in the mixtures are shown in Table 1. Prepared reaction mixtures (20 µl) had a final concentration of 1x reaction buffer containing 4 µl of the template cDNA, 1x Q solution (QIAGEN), 0.4 mM dNTP, 0.4 µM each of the forward and reverse primers, and 2 U Taq DNA polymerase. The reaction mixture was subjected to 40 cycles of amplification reaction on Applied Biosystems PE9700. The amplification cycle was carried out under conditions of denaturation at 94°C for 10 see, annealing at 50°C for 30 sec, and elongation at 72°C for 30 sec.

**Table 1**

| PRIMER SET | FORWARD | | | | REVERSE | | | |
|---|---|---|---|---|---|---|---|---|
| VH1-JH MIX | VH1a | VH1b | VH1c | VH1d | JH1245 | JH3 | JH6 | |
| VH2-JH MIX | VH2a | VH2b | | | JH1245 | JH3 | JH6 | |
| VH3/5-JH MIX | VH3a | VH3b | VH3c | VH3d | JH1245 | JH3 | JH6 | |
| VH4-JH MIX | VH4a | VH4b | | | JH1245 | JH3 | JH6 | |
| VHB/7-JH MIX | VH6a | VH7a | | | JH1245 | JH3 | JH6 | |
| VK1-JK MIX | VK1a | VK1b | VK1c | VK1d | JK1 | JK24 | JK3 | JK5 |
| VK2-JK MIX | VK2a | VK2b | | | JK1 | JK24 | JK3 | JK5 |
| VK3-JK MIX | VK3a | VK3b | VK3c | | JK1 | JK24 | JK3 | JK5 |
| VK4/5-JK MIX | VK4a | VK5a | | | JK1 | JK24 | JK3 | JK5 |
| VK6-JK M1X | VK6a | VK6b | | | JK1 | JK24 | JK3 | JK5 |

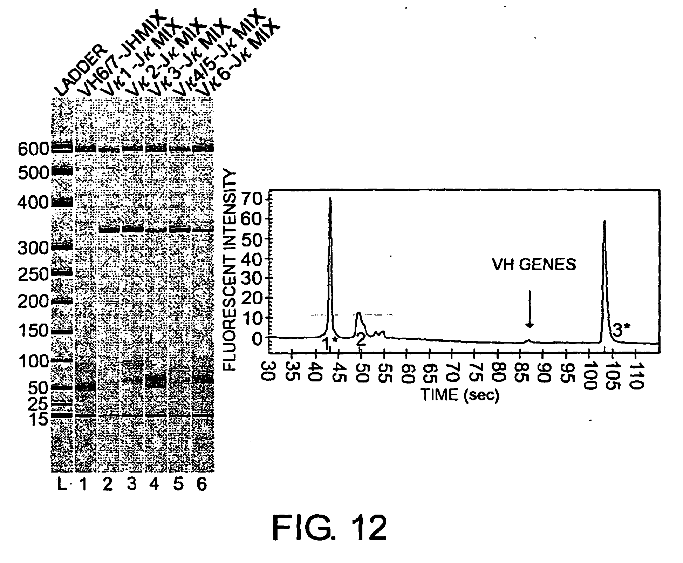

Following the reaction, amplification products were analyzed using DNA 7500 Labchip and Agilent 2100. Results of the amplification were shown in Figs. 2, 6, 8, 10, 12, 14, and 16. Amplification products were purified using the QIAGEN PCR Purification Kit. When the amount of PCR-amplified products was small, the products were electraphoresed on an agarose gel, and the region corresponding to the molecular weight of the antibody variable region gene was excised and then amplified again. DNA fragments thus obtained were cloned into pGEM-T Easy (Promega), and then introduced into *E. coli* DH5α. Nucleotide sequences of the insert sequences in the recombinant plasmids were determined and it was confirmed that antibody genes were amplified. The nucleotide sequences thus determined are shown in SEQ ID NOs: 1 to 54 (heavy chains) and SEQ ID NOs: 55 to 84 (light chains). Furthermore, the determined amino acid sequences were aligned as shown in Figs. 3 and 4. The alignment results confirmed that multiple clones of the antibody genes were obtained by the methods of this invention. However, since the isolated variable regions do not have diverse types of amino acid sequences, B cells present in the specimens may very likely have been cell populations that had multiplied on specific antigen stimulations.

### [Example 4] Preparation of single-stranded antibody molecules

The linker sequences to be used for preparing single-stranded antibody genes were produced according to the method of Marks et al. (J. Mol. Biol. (1991) 222, 581-597). The template DNA sequences and the nucleotide sequences of primers used in the preparation are shown below. Linker fragments synthesized using PCR amplification were confirmed by agarose gel electrophoresis, and bands containing the respective fragments were excised and purified.
Template DNA sequence (Template linker)/ SEQ ID NO: 151 Nucleotide sequences of primers:

| Reverse JH for linker/ SEQ ID NO: 152 to 155 | |
|---|---|
| 1 LJH1_2 | 5'-GCACCCTGGTCACCGTCTCCTCAGGTGG-3' |
| 2 LJH3 | 5'-GGACAATGGTCACCGTCTCTTCAGGTGG-3' |
| 3 LJH4_5 | 5'-GAACCCTGGTCACCGTCTCCTCAGGTGG-3' |
| 4 LJH6 | 5'-GGACCACGGTCACCGTCTCCTCAGGTGG-3' |

| Reverse VK for linker/ SEQ ID NO: 156 to 161 | |
|---|---|
| 5 LVK1 | 5'-GGAGACTGGGTCATCTGGATGTCCGATCCGCC-3' |
| 6 LVK2 | 5'-GGAGACTGAGTCATCACAACATCCGATCCGCC-3' |
| 7 LVK3 | 5'-GGAGACTGCGTCAACACAATTT-CCGATCCGCC-3' |
| 8 LVK4 | 5'-GGAGACTGGGTCATCACGATGTCCGATCCGCC-3' |
| 9 LVK5 | 5'-GGAGACTGCGTGAGTGTCGTTTCCGATCCGCC-3' |
| 10 LVK6 | 5'-GGAGACTGAGTCAGCACAATTTCCGATCCGCC-3' |

| Reverse VL for linker/ SEQ ID NO: 162 to 168 | |
|---|---|
| 11 LVL1 | 5'-GGCGGCTGCGTCAACACAGACTGCGATCCGCCACCGCCAGAG-3' |
| 12 LVL2 | 5'-GCAGGCTGAGTCAGAGCAGACTGCGATCCGCCACCGCCAGAG-3' |
| 13 LVL3a | 5'-GGTGGCTGAGTCAGCACATAGGACGATCCGCCACCGCCAGAG-3' |
| 14 LVL3b | 5'-GGGTCCTGAGTCAGCTCAGAAGACGATCCGCCACCGCCAGAG-3' |
| 15 LVL4 | 5'-GGCGGTTGAGTCAGTATAACGTGCGATCCGCCACCGCCAGAG-3' |
| 16 LVL5 | 5'-GACGGCTGAGTCAGCACAGACTGCGATCCGCCACCGCCAGAG-3' |
| 17 LVL6 | 5'-TGGGGCTGAGTCAGCATAAAATTCGATCCGCCACCGCCAGAG-3' |

PCR using the primer sets (SEQ ID NOs: 169 to 182) as shown below was performed by mixing the heavy chain variable region, κ-chain variable region or λ-chain variable region, and linker sequence, which were PCR-amplified using the cDNA synthesized from mRNA extracts of a single B cell as a template. For κ-chain light chains, reaction solutions were prepared using a combination of VH and JK primers. For λ-chain light chains, the reaction solutions were prepared with a combination of VH and JL primers. These reaction mixtures were prepared using KOD plus DNA polymerase (TOYOBO) according to manufacturer's instructions. Prior to the addition of primers, seven cycles of denaturation (at 94°C for 15 sec) and elongation (at 68°C for 1 min) were performed, and after that, 20 cycles of denaturation (at 94°C for 15 sec) and elongation (at 68°C for 1 min) followed.
- VH1NACKNco: 5'-AGTATTGACCATGGCCCAGGTGCAGCTGGTGCAGTCTGG-3'
- VH2BACKNco: 5'-AGTATTGACCATGGCCCAGGTCAACTTAAGGGAGTCTGG-3'
- VH3BACKNco: 5'-AGTATTGACCATGGCCGAGGTGCAGGTGGTGGAGTCTGG-3'
- VH4BACKNco: 5'-AGTATTGACCATGGCCCAGGTGGAGGTGCAGGAGTCGGG-3'
- VH5BACKNco: 5'-AGTATTGACCATGGCCCAGGTGCAGCTGTTGCAGTCTGC-3'
- VH6BACKNco: 5'-AGTATTGACCATGGCCCAGGTACAGCTGCAGCAGTCAGG-3'
- JK1FOREco: 5'-TAATGAATTCACGTTTGATTTCCACCTTGGTCCC-3'
- JK2FOREco: 5'-TAATGAATTCACGTTTGATCTCCAGCTTGGTCCC-3`
- JK3FOREco: 5'-TAATGAATTCACGTTTGATATCCACTTTGGTCCC-3'
- JK4FOREco: 5'-TAATGAATTCACGTTTGATCTCCACCTTGGTCCC-3'
- JK5FOREco: 5'-TAATGAATTCACGTTTAATCTCCAGTCGTGTCCC-3'
- JL1FOREoo: 5'-TAATGAATTCACCTAGGACGGTGACCTTGGTCCC-3'
- JL_3FOREco: 5'-TAATGAATTCACCTAGGACGGTCAGCTTGGTCCC-3'.
- JL4_5FOREco: 5'-TAATGAATTCACCTAAAACGGTGAGCTGGGTCCC-3'

After the presence of the amplification products were confirmed by agarose gel electrophoresis, bands containing the corresponding gene fragments were excised and purified. Gene fragments thus excised were cleaved with restriction enzymes and inserted into expression vectors. The expression vectors were designed such that the inserts can be expressed under the control of a T7 promoter and a FLAG tag can be added to the C terminus of the recombinants. The expression vectors thus obtained were introduced into *E. coli* DH5α. The expression plasmids were confirmed to have the inserts by DNA sequencing, and then introduced into the *E. coli* BL21 (DE3) strain.

The nucleotide sequences of the single-stranded antibodies constructed in this Example, and their translated amino acid sequences, are shown in SEQ ID NOs: 183 to 188. 70-6scFV (SEQ ID NOs: 187-188) is a single-stranded antibody prepared from a heavy chain (SEQ ID NOs: 93-94) and a light chain (SEQ ID NOs: 95-96) isolated from a single B cell. On the other hand, 70-5AscFv (SEQ ID NOs: 183-184) and 70-5BscFv (SEQ ID NOs: 185-186) are single-stranded antibodies formed by combining heavy chains and light chains obtained from five B cells. The nucleotide sequences and amino acid sequences that constitute the heavy chains comprised in 70-5AscFv and 70-5BscFv are shown in SEQ ID NOs: 91-92, and the nucleotide sequences and amino acid sequences which constitute the light chains are shown in SEQ ID NOs: 85-88.

Recombinant single-stranded antibodies were prepared from *E. coli* culture supernatants. During the logarithmic growth phase of transformed *E. coli* cells, expression of the recombinant antibodies was induced by the addition of 0.5 mM isopropyl-β-thiogalactopyranoside at 30°C. After overnight culture, the culture solution was centrifuged to separate the culture supernatant from cells. Following filtration, the resulting supernatant was applied to Anti-FLAG M2 affinity column (Sigma) and the recombinant protein was absorbed thereto. After washing the column, the recombinant protein was eluted with 0.1 M glycine (pH3.5). The eluate was applied to PD10 column (Amersham Pharmacia Biotech), and the buffer was immediately replaced with PBS containing 0.01% Tween-20. Presence of the protein was confirmed by Coomassie staining or Western blotting with anti-FLAG antibodies following SDS-PAGE.

### [Example 3] Immunostaining

Flash-frozen sections of a cancer tissue were fixed with PBS solution containing 1% paraformaldehyde for 10 min. The endogenous peroxidase activity was blocked with 0.3% hydrogen peroxide. In order to avoid non-specific bindings of recombinant antibodies, tissue sections were blocked with 10% fetal bovine serum prior to incubation with a solution containing recombinant antibodies. The solution containing recombinant antibodies was dilute in PBS containing 1% BSA and 0.1% Tween-20, and tissue sections were incubated in this solution. The bound recombinant antibodies were detected by the conversion of 3,3-diamino-benzidine-tetrahydrochloride with the peroxidase-conjugated anti-FLAG antibody (FLAG) into a brown precipitate in the presence of hydrogen peroxide. Before and after the addition of antibodies, wishing was carried out three times with PBS containing 0.1 % Tween-20 at room temperature for 5 min. Prior to mounting, tissue sections were counterstained with hematoxylin, and dehydrated using ethanol and xylene. As a negative control for antibody staining, similar procedures were performed without adding the recombinant antibodies.

### Industrial Applicability

The present invention enables the isolation of polynucleotides encoding antibodies against a lesional tissue without depending on B cell cloning. Since the methods of this invention do not rely on B cell cloning, they enable easy acquisition of genes derived from human antibody-producing cells which are difficult to clone.

Based on the present invention, genes encoding cancer tissue-recognizing antibodies can be isolated from B cells that infiltrate the cancer tissue. Antibodies recognizing cancer cells are useful for the diagnosis and treatment of cancer. By using this invention, antibody genes can be easily obtained from human antibody-producing cells as well. Acquisition of genes for cancer tissue-recognizing human antibodies is of great significance in cancer diagnosis and treatment.

For diagnosis and treatment of cancer using antibodies, antibodies are administered to humans. For example, to diagnose cancer using antibodies, antibody molecules with a traceable label are administered so as to indicate the presence of cancer at the region where the antibodies are localized. For cancer treatment, antibodies are utilized in target therapy. That is, antibodies conjugated with an anticancer agent are administered to patients. Human antibodies can be expected to be highly safe when administered to humans. In addition, since human antibodies are unlikely to be recognized as foreign proteins, the concentration can be stably maintained in blood over a long period of time.

## Claims

1. A method for isolating a polynucleotide encoding an antibody against a lesional tissue, wherein the method comprises the step of obtaining a polynucleotide encoding an antibody from a B cell obtained by isolating a lesional tissue-infiltrating B cell, wherein the lesional tissue-infiltrating B cell is isolated by excising a region comprising a B cell from an isolated section of said lesional tissue by laser microdissection, wherein the lesional tissue is derived from solid tumor lesions, arteriosclerosis lesions, inflammatory disease lesions, lesions generated by infectious pathogens, or autoimmune disease lesions.

2. The method of claim 1, wherein the lesional tissue is a cancer tissue.

3. The method of claim 2, wherein the cancer tissue is selected from the group consisting of breast cancer tissues, lung cancer tissues, liver cancer tissues, colon cancer tissues, pancreas cancer tissues and prostate cancer tissues.

4. The method of claim 1, wherein the polynucleotide encoding an antibody is obtained by amplifying a gene encoding an antibody variable region.

5. A method for producing an antibody, wherein the method comprises the steps of:
(i) obtaining a polynucleotide encoding an antibody from a B cell obtained by isolating a lesional tissue-infiltrating B cell, wherein the lesional tissue-infiltrating B cell is isolated by excising a region comprising a B cell from an isolated section of said lesional tissue by laser microdissection, wherein the lesional tissue is derived from solid tumor lesions, arteriosclerosis lesions, inflammatory disease lesions, lesions generated by infectious pathogens, or autoimmune disease lesions;
(ii) preparing an expression vector comprising the polynucleotide of step (i),
(iii) transforming a host cell with the expression vector of step (ii) to obtain a host cell expressing the polynucleotide,
(iv) culturing the host cell of step (iii); and
(v) recovering an antibody which is the expression product.

6. The antibody production method of claim 5, wherein the method further comprises the steps of
(vi) contacting the antibody obtained by the method of claim 5 with a lesional tissue;
(vii) detecting the binding between the antibody and the lesional tissue; and
(viii) selecting an antibody that binds to the lesional tissue.

7. The method of claim 1, wherein the number of B cells to be isolated is 20 cells or less.

8. The method of claim 7, wherein the number of B cells to be isolated is 5 cells or less.

## Patentansprüche

1. Verfahren zur Isolierung eines Polynucleotids, das einen Antikörper gegen ein geschädigtes Gewebe codiert, wobei das Verfahren den Schritt des Erhaltens eines Polynucleotids umfasst, das einen Antikörper von einer B-Zelle codiert, die erhalten wird durch Isolieren einer ein geschädigtes Gewebe infiltrierenden B-Zelle, wobei die ein geschädigtes Gewebe infiltrierende B-Zelle isoliert wird durch Herausschneiden einer eine B-Zelle umfassenden Region von einem isolierten Teil des geschädigten Gewebes durch Lasermikrodissektion, wobei das geschädigte Gewebe von Schädigungen durch solide Tumoren, Arterioskleroseschädigungen, Schädigungen von Entzündungserkrankungen, von infektiösen Pathogenen erzeugten Schädigungen oder Schädigungen von Autoimmunerkrankungen stammen.

2. Verfahren nach Anspruch 1, wobei das geschädigte Gewebe Krebsgewebe ist.

3. Verfahren nach Anspruch 2, wobei das Krebsgewebe ausgewählt ist aus der Gruppe bestehend aus Brustkrebsgewebe, Lungenkrebsgewebe, Leberkrebsgewebe, Dickdarmkrebsgewebe, Bauchspeicheldrüsenkrebsgewebe und Prostatakrebsgewebe.

4. Verfahren nach Anspruch 1, wobei das Polynucleotid, das einen Antikörper codiert, erhalten wird durch Amplifikation eines Gens, das eine variable Region eines Antikörpers codiert.

5. Verfahren zur Herstellung eines Antikörpers, wobei das Verfahren die Schritte umfasst:
(i) das Erhalten eines Polynucleotids, das einen Antikörper von einer B-Zelle codiert, die erhalten wird durch Isolieren einer ein geschädigtes Gewebe infiltrierenden B-Zelle, wobei die ein geschädigtes Gewebe infiltrierende B-Zelle isoliert wird durch Herausschneiden einer eine B-Zelle umfassenden Region von einem isolierten Teil des geschädigten Gewebes durch Lasermikrodissektion, wobei das geschädigte Gewebe von Schädigungen durch solide Tumoren, Arterioskleroseschädigungen, Schädigungen von Entzündungserkrankungen, von infektiösen Pathogenen erzeugten Schädigungen oder Schädigungen von Autoimmunerkrankungen stammen;
(ii) Herstellen eines Expressionsvektors, der das Polynucleotid nach Schritt (i) umfasst;
(iii) Transformieren einer Wirtszelle mit dem Expressionsvektor nach Schritt (ii), um eine Wirtszelle zu erhalten, die das Polynucleotid exprimiert;
(iv) Züchten der Wirtszelle nach Schritt (iii); und
(v) Gewinnen eines Antikörpers, der das Expressionsprodukt ist.

6. Antikörperherstellungsverfahren nach Anspruch 5, wobei das Verfahren ferner die Schritte umfasst:
(vi) Inkontaktbringen des Antikörpers, erhalten durch das Verfahren nach Anspruch 5, mit einem geschädigten Gewebe;
(vii) Nachweis der Bindung zwischen dem Antikörper und dem geschädigten Gewebe; und
(viii) Auswahl eines Antikörpers, der an das geschädigte Gewebe bindet.

7. Verfahren nach Anspruch 1, wobei die Anzahl der zu isolierenden B-Zellen 20 Zellen oder weniger ist.

8. Verfahren nach Anspruch 7, wobei die Anzahl der zu isolierenden B-Zellen 5 Zellen oder weniger ist.

## Revendications

1. Méthode pour isoler un polynucléotide codant un anticorps contre un tissu lésionnel, où la méthode comprend l'étape d'obtention d'un polynucléotide codant un anticorps à partir de la cellule B obtenue par isolation d'une cellule B infiltrant le tissu lésionnel, où la cellule B infiltrant le tissu lésionnel est isolée par excision d'une région comprenant une cellule B à partir d'une section isolée dudit tissu lésionnel par microdissection laser, où le tissu lésionnel est dérivé des lésions de tumeur solide, lésions d'artériosclérose, lésions de maladie inflammatoire, lésions générées par des pathogènes infectieux, ou lésions de maladie auto-immune.

2. Méthode de la revendication 1, dans laquelle le tissu lésionnel est un tissu cancéreux.

3. Méthode de la revendication 2, dans laquelle le tissu cancéreux est choisi dans le group constitué par les tissus de cancer du sein, les tissus de cancer du poumon, les tissus de cancer du foie, les tissus de cancer du côlon, les tissus de cancer du pancréas et les tissus de cancer de la prostate.

4. Méthode de la revendication 1, dans laquelle le polynucléotide codant un anticorps est obtenu par amplification d'un gène codant une région variable d'anticorps.

5. Méthode pour produire un anticorps, ladite méthode comprenant les étapes suivantes :
(i) l'obtention d'un polynucleotide codant un anticorps à partir de la cellule B obtenue par isolation d'une cellule B infiltrant le tissu lésionnel, où la cellule B infiltrant le tissu lésionnel est isolée par excision d'une région comprenant une cellule B à partir d'une section isolée dudit tissu lésionnel par microdissection laser, où le tissu lésionnel est dérivé des lésions de tumeur solide, lésions d'artériosclérose, lésions de maladie inflammatoire, lésions générées par des pathogènes infectieux, ou lésions de maladie auto-immune ;
(ii) la préparation d'un vecteur d'expression comprenant le polynucléotide de l'étape (i) ;
(iii) la transformation d'une cellule hôte avec le vecteur d'expression de l'étape (ii) afin d'obtenir une cellule hôte exprimant le polynucléotide ;
(iv) la culture de la cellule hôte de l'étape (iii) ; et
(v) la récupération d'un anticorps qui est le produit d'expression.

6. Méthode de production d'anticorps de la revendication 5, ladite méthode comprenant en outre les étapes de :
(vi) la mise en contact de l'anticorps obtenu par la méthode de la revendication 5 avec un tissu lésionnel ;
(vii) la détection de la liaison entre l'anticorps et le tissu lésionnel; et
(viii) la sélection d'un anticorps qui se lie au tissu lésionnel.

7. Méthode de la revendication 1, dans laquelle le nombre de cellules B à isoler est 20 cellules ou moins.

8. Méthode de la revendication 7, dans laquelle le nombre de cellules B à isoler est 5 cellules ou moins.
